# EUROPEAN PATENT APPLICATION

(11) **EP 2 390 352 A1**
(43) Date of publication of application: **30.11.2011**
(21) Application number: 11159459.4
(22) Date of filing: 18.03.2004
(51) Int. Cl.: C12Q 1/68

(54) **Systems and methods for improving protein and milk production of dairy herds**

(30) Priority: 18.03.2003 CA 2422437; 21.03.2003 US 456489 P; 29.04.2003 US 466523 P; 08.10.2003 US 509755 P; 02.02.2004 US 770307
(62) Divisional of application: 04721437.4
(71) Applicant: Quantum Genetics Ireland Limited, Dublin (IE)
(72) Inventor: The designation of the inventor has not yet been filed
(74) Representative: Coret, Sophie V.G.A.

(57) **Abstract**

The present invention provides for a direct correlation between milk production in livestock animals and the presence of alleles of a gene encoding an adipocyte-specific polypeptide, termed leptin, which gene is hereinafter referred to as *ob*. The invention also provides novel compositions consisting essentially of specific oligonucleotides that are useful as primers to amplify particular regions of the genome during enzymatic nucleic acid amplification, thus providing a rapid, sensitive and specific method for the detection of the *ob*-gene polymorphism which may be present in a specimen. The invention further provides for methods of screening bovine to determine those having predictably more milk productivity and advantageously selecting those livestock for future breeding and management purposes based on the *ob* polymorphisms.

## Description

### INCORPORATION BY REFERENCE

This application is a continuation-in-part of copending application USSN 10/770,307, filed February 2, 2004, which claims priority to U.S. Provisional Application Serial No. 60/466,523 entitled "METHOD FOR IMPROVING EFFICIENCIES IN LIVESTOCK PRODUCTION", filed April 29, 2003, and U.S. Provisional Application Serial No. 60/509,775 entitled "METHOD FOR IMPROVING FEED CONVERSION EFFICIENCY IN LIVESTOCK PRODUCTION", filed October 8, 2003. This application also claims priority to Canadian Patent Application No. 2,422,437 entitled : "IMPROVING PROTEIN AND MILK PRODUCTION OF DAIRY HERDS", filed March 18, 2003 and to U.S. Provisional Application Serial No. 60/456,489 entitled: "PROTEIN AND MILK PRODUCTION OF DAIRY HERDS", filed March 21, 2003. The foregoing applications, and all documents cited therein or during their prosecution ("appln cited documents") and all documents cited or referenced in the appln cited documents, and all documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention.

### FIELD OF THE INVENTION

The present invention relates to a method of managing livestock animals by selecting the animals according to a specific genotype and, in particular, to a method for selecting animals for inclusion in a group of animals according to variations in the *ob* gene so as to select animals with a greater propensity for milk production. The present invention relates to a method of identifying animals of a first genotype that produce more milk and milk protein as compared to the animals of a second different genotype. By selecting animals of the first genotype for inclusion in a group of animals, thereby increasing the number of such animals in the group compared to a conventionally selected group, the amount of milk and milk protein can be increased.

Also provided by the present invention are methods of using genetic markers relating to the regulation of energy intake and metabolism in growing, finishing, lactating or nonlactating, and gestating livestock, methods for identifying such markers, and methods of screening livestock to determine those having predictably more uniform fat deposition and altered milk production and milk components, as well as advantageously selecting those livestock for future breeding and management purposes based on polymorphisms. The markers are based upon the presence or absence of certain polymorphisms in the *ob* gene. Also disclosed herein are oligonucleotides that can be used as primers to amplify specific nucleic acid sequences of the *ob* gene. The present invention also provides oligonucleotides that can be used as probes in the detection of amplified specific nucleic acid sequences of the *ob* gene.

### BACKGROUND OF THE INVENTION

Leptin, a 16-kDa adipocyte-specific polypeptide is expressed predominantly in fat tissues of those animals in which it has been detected, which animals include livestock species such as cattle, pigs, and sheep. Leptin is encoded by the *ob* (obese) gene and appears to be involved in the regulation of appetite, basal metabolism and fat deposition. Increased plasma concentrations of leptin in mice, cattle, pigs and sheep have been associated with decreased body fat deposition and appetite, and increased basal metabolism levels (Blache et al., J Endocrinol. 2000 Jun;165(3):625-37; Delavaud et al., J Endocrinol. 2000 May;165(2):519-26 and Ehrhardt et al., J Endocrinol. 2000 Sep;166(3):519-28). Similar phenotypic characteristics have also been found to be associated with leptin mRNA levels in adipose tissue (Ramsay et al., J Anim Sci. 1998 Feb;76(2):484-90 and Robert et al., Can. J. Anim. Sci. 1998;78:473-82). Consistent with those observations, it has been shown that administration of exogenous leptin dramatically reduces feed intake and body mass of mice, chickens, pigs and sheep (Barb et al., Domest Anim Endocrinol. 1998 Jan;15(1):77-86; Halaas et al., Science. 1995 Jul 28;269(5223):543-6; Henry et al., Endocrinology. 1999 Mar;140(3):1175-82 and Raver et al., Protein Expr Purif. 1998 Dec;14(3):403-8).

The *ob* gene that has been mapped to chromosome 6 in mice (Friedman & Leibel, Cell. 1992 Apr 17;69(2):217-20), chromosome 7q31.3 in humans (Isse et al., J Biol Chem. 1995 Nov 17;270(46):27728-33) chromosome 4 in cattle (Stone et al. Mamm. Genome 1996;7: 399-400), and chromosome 18 in swine (Neuenschwander et al., Anim Genet. 1996 Aug;27(4):275-8 and Saskai et al., Mamm. Genome 1996;7:471). Sequences have been determined for the said gene from mice (Zhang et al., Nature. 1994 Dec 1;372(6505):425-32), cattle (U.S. Patent No. 6,297,027), pigs (U.S. Patent No. 6,277,592 and Neuenschwander et al., Anim Genet. 1996 Aug;27(4):275-8), and humans (U.S. Patent No, 6,309,857) and there is significant conservation among the sequences of *ob* DNAs and leptin polypeptides from those species (Bidwell et al., Anim. Biotech. 1997;8:191-206 and Ramsay et al., J Anim Sci. 1998 Feb;76(2):484-90).

It has been demonstrated that plasma leptin concentrations are significantly diminished in animals homozygous for mutant alleles of the *ob* gene *(ob⁻*/*ob⁻* animals), which alleles do not encode functional leptin, compared to wild-type *(ob⁺*/*ob⁺)* controls. Mutations in the coding sequences of the *ob* gene causing alterations in the amino acid sequence of the leptin polypeptide, have been associated with hyperphagia, hypometabolic activity, and excessive fat deposition; *i.e*., a phenotype characterized by larger body size; a fat phenotype (Zhang et al., Nature. 1994 Dec 1;372(6505):425-32).

Fitzsimmons et al. (Mamm Genome. 1998 Jun;9(6):432-4) reported evidence of three alleles of a microsatellite marker located proximal to the *ob* gene in cattle that occurred with significant frequency in bulls of several breeds (Angus, Charolais, Hereford and Simmental) and comprising 138, 147 and 149 base pairs (bp). The 138-bp and 147-bp alleles, respectively, occurred most frequently. Further, it was determined that occurrence of the 138-bp allele was positively associated with certain carcass characteristics; increased average fat deposition, increased mean fat deposition, increased percent rib fat, and decreased percent rib lean. Thus, bulls homozygous for the 138-bp allele exhibited greater average fat deposition than heterozygous animals and such heterozygotes exhibited greater average fat deposition that bulls homozygous for the 147-bp allele.

Subsequently, Buchanan et al. (Genet Sel Evol. 2002 Jan-Feb;34(1):105-16) identified a cytosine (C) to thymine (T) transition within an exon (exon 2) of the *ob* gene, corresponding to an arginine (ARG) to cysteine (CYS) substitution in the leptin polypeptide. The presence of the T-containing allele in bulls was associated with fatter carcasses than those from bulls with the C-containing allele.

Single nucleotide polymorphisms have also been detected in the porcine *ob* gene and certain of those polymorphisms have been found to be associated with feed intake and carcass traits (Kennes et al., Anim Genet. 2001 Aug;32(4):215-8 and Kulig et al., Arch. Tierz. Dummorscorf 2001;44:291-296). Means of selective amplification of bovine gene are in U.S. Patent No. 6,297,027.

It is possible to distinguish *ob* genotypes by cloning and sequencing DNA fragments from individual animals, or by other methods known in the art. For example, it is possible to distinguish *ob* genotypes by employing synthetic oligonucleotide primed amplification of *ob* gene fragments followed by restriction endonuclease digestion of the amplified product using a restriction enzyme that cuts such product from different *ob* alleles into discrete product fragments of differing length. Such discrete product fragments could then be distinguished using electrophoresis in agarose or acrylamide, for example. The *ob* alleles identified by Buchanan et al. (Genet Sel Evol. 2002 Jan-Feb;34(1):105-16) were distinguished by such means using a mismatch PCR-RFLP strategy wherein, the C-containing allele (as above) yields DNA fragments of 75 and 19 bp following digestion of the amplimer with Kpn 2I, and the T-containing allele (as above) is not cut.

In managing livestock animals using present methods, visible characteristics or phenotypic traits are used to predict how an animal will grow, and thus how the animal should be fed to most profitably achieve market condition. The object of a livestock industry is to convert feed into meat, and much is known about growth patterns of livestock.

Body condition is a determinant of market readiness in commercial livestock feeding and finishing operations. The term body condition is used in livestock industry in reference to the state of development of a livestock animal that is a function of frame type or size, and the amount of intramuscular fat and back fat exhibited by an animal. It is typically determined subjectively and through experienced visual appraisal of live animals. The fat deposition, or the amount of intramuscular fat and back fat on an animal carcass, is important to industry participants because carcasses exhibiting desired amounts and proportions of such fats can often be sold for higher prices than carcasses that exhibit divergences from such desired amounts and proportions.

Furthermore, the desired carcass fat deposition often varies among different markets and buyers, and also often varies with time in single markets and among particular buyers in response to public demand trends with respect to desired of fat and marbling in meat.

Weight gain by a livestock animal during its growth and development typically follows a tri-phasic pattern that is carefully managed by commercial producers, and finishers. The efficiency of dietary caloric (feed) conversion to weight gain during an increment of time varies during three growth phases; a first phase of growth comprises that portion of a livestock animals life from birth to weaning, and is not paid much heed by commercial feeding and finishing operators.

A second growth phase comprises that portion of a livestock animal's life from weaning to attainment of musculo-skeletal maturity. Feed conversation efficiency is relatively high

during this phase; livestock producers usually restrict caloric intake, which has the effect of causing this phase to be prolonged but also typically results in animals with larger frames, which is the aim of dietary management during this phase. During the second growth phase weight gain is associated with skeletal mass and muscle mass accumulation primarily.

During a third growth phase, after an animal has attained musculo-skeletal maturity, the efficiency of feed conversion is reduced, such that it requires more feed to increase an animal's weight. For example with cattle, during the second phase of growth, a typical steer could convert 5 to 6 pounds of feed into one pound of weight gain. Upon entering the third phase, feed conversion efficiency typically decreases, such that 7 up to 10 or more pounds of feed are required to produce one pound of gain.

During the third phase livestock feeders significantly increase the caloric content of animals' rations. During the third growth phase weight gain is associated with fat accumulation primarily. Again using cattle as an example, with a steer weighing 900 pounds at the end of the second phase, of that 900 pounds, typically 350 pounds will be red meat. At the end of the third phase, the steer would typically weigh 1400 pounds and typically 430 pounds will be red meat.

Keeping the cattle industry as an example, initially a cow/calf operator will breed bulls to cows, birth calves from the cows, and allow the calves to feed on their mother's milk until they are weaned some months after birth. This is the first phase of growth of the calf.

After weaning, the calf enters the second stage of growth where it is fed to grow to its full skeletal size. This commonly called the "backgrounding" phase during which musculo-skeletal maturity is achieved. When the animal has reached its full size, it enters the third phase of growth where the fully grown animal puts on weight.

Typically it is at the start of the third stage of growth that the animal enters a finishing feed lot. In the feed lot the object is to feed the animal the proper ration so that it will most quickly obtain the proper market characteristics that are desired at that given time. At present, for instance it is desirable to have beef that is well marbled, i.e., it has considerable intramuscular fat in the meat. At other times it may be desirable to have lean meat with very little intramuscular fat. The price the feed lot owner attains for his cattle, when he sells to the packer can vary significantly depending on marbling of the meat.

Presently, cattle entering a feed lot are divided into groups according to estimated age, frame size, breed, weight and so forth. By doing this the feed lot owner is attempting to group the cattle so that the group can be penned together and fed the same ration and will be ready for market at the same time. Weight and visual clues are the only means possible to sort cattle for feed lot grouping.

The phenotype of an animal is defined as the visible characteristics of the animal resulting from the interaction between the animal's genetic makeup and its environment. Thus, present management techniques group cattle according to uniform phenotypic traits and then keep the environment constant for each animal in the group in hopes that the group will together achieve a different phenotype at some later date. Although the genetic makeup of any individual steer is a significant factor in the ability of that individual steer to grow in the same manner as another steer of the same phenotype, this consideration is presently not taken into account by conventional livestock management practices. Instead, cattle are segregated into groups based on phenotypic traits alone even though results of present livestock feeding and grouping methods show the substantial effects that genetic makeup has on the growth of cattle. For example, considerable variation in phenotypes is present at the end of the third phase among cattle that entered the third phase with a substantially uniform phenotype, despite having been subjected to the same environmental factors as with conventional management methods.

It is not uncommon for a pen of cattle, each having a weight within a range of 100 pounds going into a feeding pen, to have weights varying in a range of 300 pounds or more coming out of the pen for slaughter. It is also known that the feed conversion rate of cattle varies to some degree. Since feed represents a major cost to the feed-lot operator, it is more profitable to feed those cattle with a higher feed conversion rate since an animal that converts a ton of feed into 200 pounds of saleable body weight is more profitable than another animal that converts the same ton of feed into only 180 pounds of saleable meat. Presently, however, it is not known how to identify cattle having a higher feed conversion rate, except by measuring feed eaten against weight gained. It is not economically feasible to perform such measurements on each animal entering a commercial feedlot - the numbers of animals are too great, and individual attention required by the operator to gather the measurements is not possible. In contrast, timing of slaughter is based on the mean visible condition of the group of cattle in each pen, resulting in a wide variation in carcass weight and ensuring that grading premiums for carcasses of a desired condition of weight and fat are not met for a significant number of cattle. In a typical pen, a number of the cattle in the pen would have been at the desired carcass condition earlier, but by the time they are slaughter they are over fat. Similarly, many cattle could readily achieve the desired carcass condition if fed longer. However, conventional management techniques require that all the cattle in the pen are slaughtered at the same time.

Cattle operators breed bulls to cows, choosing the mating based on signals received through the chain of supply from consumers for those traits that are in demand, for example fat beef or lean beef European breeds provide carcasses that are typically leaner than British breeds, therefore the cow/calf operator will typically lean to one or the other as demand changes. They also select breeding animals based on visual traits, such as frame size, and anecdotal traits, such as easy calving history. Again, the object is to provide cattle that will command the highest price from the eventual purchaser, such as a backgrounder or feed lot operator.

A dairy cattle operator is faced with similar issues as packers, feeders and cow/calf operators. Dairy cattle are also segregated into groups based upon phenotypic traits even though genotype can affect milk production. In particular, the time period from calving through to peak lactations is the most stressful period in the life of the dairy cow. During this time, the animal usually falls into negative energy balance because the daily feed intake, although increased, is unable to keep pace with the increased energy demand of lactation. Since certain genotypes affect energy balance, management of animals by genotype will be important for efficient dairy production. Furthermore, the animals' genetic predisposition to lay down fat also impacts milk production.

Citation or identification of any document in this application is not an admission that such document is available as prior art to the present invention.

### SUMMARY OF THE INVENTION

It is an aspect of the present invention to provide a method for improving efficiencies in milk production.

The invention is based in part on Applicants' finding that the leptin single nucleotide polymorphism (SNP) associated with increased fat deposition in beef cattle is also associated with lactation performance. The milk and milk protein yield advantage observed in cows homozygous for the T allele, represent an economic advantage to dairy farmers.

The present invention also provides a method comprising identifying livestock animals having greater milk productivity by identifying a genetic indicator in the animals that allows management of livestock by genetic selection in addition to phenotype.

The present invention discloses nucleic acid sequences (oligonucleotides) useful as primers and/or probes in the detection of a polymorphism in livestock specimens.

The present invention provides oligonucleotide sequences and methods of using them, which permit the prediction of milk production, feed conversion efficiency, and the prediction and modulation of fat deposition in mammals, especially in the bovine species, by looking for mutations in the leptin (*ob*) gene that produces the leptin protein.

Also included in the present invention is a method for detecting the presence of a polymorphism in the nucleic acid molecules for the leptin gene as described herein, or a complementary sequence, in a nucleic acid-containing sample, the method comprising: (a) contacting the sample with an oligonucleotide probe complementary to the sequence of interest under hybridizing conditions; and (b) measuring the hybridization of the probe to the nucleic acid molecule, thereby detecting the presence of the nucleic acid molecule. The above method may additionally comprise before step (a): (c) selectively amplifying the number of copies of the nucleic acid sequence.

It is an object of the invention to provide methods of screening livestock to determine those more likely to have increased milk production. The invention also provides for methods of screening livestock to determine those more likely to have predictably uniform fat deposition. Another object of the invention is to provide a method for identifying genetic markers for feed conversion efficiency, a measure of the ability of the animal to convert feed eaten into weight gain, generally measured as the amount of weight gained per pound of feed eaten, or the amount of feed required to put on a pound of weight gain. Yet another object of the invention is to provide a kit for evaluating a sample of livestock DNA for specific genetic markers for increased milk production, and optionally, genetic markers for fat deposition and feed conversion efficiency.

Another object of the present invention is to provide oligonucleotides that can be used as primers to amplify specific nucleic acid sequences of the *ob* gene.

It is also an object of the present invention is to provide oligonucleotides that can be used as probes in the detection of amplified specific nucleic acid sequences of the *ob* gene.

Another object of the present invention is to provide oligonucleotides that can be used as primers to amplify DNA sequences from a polymorphism of the *ob* gene. In an advantageous embodiment, the *ob* gene polymorphism is a C to T transition that results in an Arg25 Cys in the leptin protein.

It is the object of the present invention to provide a method for improving efficiencies in livestock production. It is a further objective to provide such a method that comprises grouping livestock animals, such as cattle and pigs, during the period of their retention in a feeding facility according to the genetic predisposition of individual livestock animals to deposit fat, and then feeding the animals in each group substantially uniformly. Optionally, it is yet another object of the invention to decrease the amount of feed needed to produce any given increase in weight of livestock animals in a feedlot by selecting the cattle being fed to increase the occurrence of the T-containing allele of the *ob* gene in the cattle being fed.

It is an embodiment of the present invention to provide such a method comprising determining the genetic predisposition of individual livestock animals to meet particular milk production expectations. In one embodiment, homozygosity or heterozygosity of each animal is determined with respect to alleles, and such animals are segregated into groups based on genotype, *e.g., ob* genotype, and optionally, phenotype. In one embodiment, animals are segregated by phenotype, *e.g*., frame type and genotype, *e.g*., homozygosity in respect of a first *ob* allele or homozygosity in respect of a second *ob* allele (e.g., TT or CC animals), or heterozygosity in respect of the first and second *ob* alleles (e.g., CT animals), then feeding and otherwise maintaining animals in a group together and apart from other groups of animals, and ceasing to feed the animals in the group at a time is sustained until the median body fat condition of the animals of that group is of a desired body fat condition.

It is a further embodiment of the present invention to provide such a method of determining homozygosity or heterozygosity of cattle with respect to alleles of the *ob* gene, and sorting the cattle accordingly into three groups, one group homozygous in respect of a first *ob* allele and therefore having the most propensity to lay down fat, a second group homozygous in respect of a second *ob* allele and therefore having the least propensity to lay down fat, and a third group heterozygous in respect of the first and second *ob* alleles and therefore having an intermediate propensity to lay down fat. It is a further object of the present invention to provide such a method wherein the three groups are further divided according to weight or frame size.

To achieve the objects and in accordance with the purpose of the invention, as embodied and broadly described herein, the present invention provides a method for screening cattle to identify those with a higher propensity towards increased milk production, and also to allow grouping of the cattle to yield a consistent quality grade. A sample of genomic DNA is obtained from the cattle, and the sample is analyzed to determine the presence or absence of a polymorphism in the *ob* gene that is correlated with increased milk production. In an advantageous embodiment, the *ob* gene polymorphism is a C to T transition that results in an Arg25Cys in the leptin protein that is correlated with increased weight gain. In one embodiment, the polymorphism is detected using FRET.

In another embodiment the presence or absence of a specific fragment is assayed for by use of primers and DNA polymerase to amplify a specific region of the gene which contains the polymorphism. In an advantageous embodiment, the *ob* gene polymorphism is a C to T transition that results in an Arg25 Cys in the leptin protein.

In one embodiment, the target nucleic acid is first amplified, such as by PCR, SDA, NASBA, TMA, rolling circle, T7, T3, or SP6, each of which methods are well understood in the art, using at least one amplification primer oligomer. The oligomer may be labeled with a moiety useful for attaching the amplification product to a substrate surface. Following amplification, the amplified dsDNA product may be denatured.

In one aspect, during the hybridization of the nucleic acid target with the anchor probe and/or the sensor probe, stringent conditions may be utilized, advantageously along with other stringency affecting conditions, to aid in the hybridization. In yet another aspect, stringency conditions may be varied during the hybridization complex stability determination so as to more accurately or quickly determine whether a SNP is present in the target sequence. Hybridization stability may be influenced by numerous factors, including thermoregulation, chemical regulation, as well as stringency control, either alone or in combination with the other listed factors.

In one mode, the hybridization complex is labeled and the step of determining amount of hybridization includes detecting the amounts of labeled hybridization complex under stringent and destabilizing conditions. The detection device and method may include, but is not limited to, optical imaging, electronic imaging, imaging with a CCD camera, integrated optical imaging, and mass spectrometry. Further, the detection, either labeled or unlabeled, is quantified, which may include statistical analysis. The labeled portion of the complex may be the target, the anchor, the sensor or the hybridization complex in toto. Labeling may be by fluorescent labeling selected from the group of, but not limited to, Cy3, Cy5, Bodipy Texas Red, Bodipy Far Red, Lucifer Yellow, Bodipy 630/650-X, Bodipy R6G-X and 5-CR 6G. Labeling may further be accomplished by colormetric labeling, bioluminescent labeling and/or chemiluminescent labeling. Labeling further may include energy transfer between molecules in the hybridization complex by perturbation analysis, quenching, electron transport between donor and acceptor molecules, the latter of which may be facilitated by double stranded match hybridization complexes. Optionally, if the hybridization complex is unlabeled, detection may be accomplished by measurement of conductance differential between double stranded and non-double stranded DNA. Further, direct detection may be achieved by porous silicon-based optical interferometry or by mass spectrometry. The label may be amplified, and may include for example branched or dendritic DNA. The target DNA may unamplified or amplified. Further, if the target is amplified and the amplification is an exponential method, it may be, for example, PCR amplified DNA or strand displacement amplification (SDA) amplified DNA. Linear methods of DNA amplification such as rolling circle or transcriptional runoff may also be used.

The present invention provides oligonucleotides that can be used as primers to amplify specific nucleic acid sequences of the *ob* gene. The present invention also provides oligonucleotides that can be used as probes in the detection of amplified specific nucleic acid sequences of the *ob* gene, SEQ ID NO:1 or SEQ ID NO:2. The oligonucleotides can be immobilized on a solid support. Alternatively, a plurality of oligonucleotide probes wherein one or more oligonucleotide probes can be immobilized on an oligonucleotide array.

Among the nucleic acids provided herein are the nucleic acids whose sequence is provided in SEQ ID NO:4, SEQ ID NO:5, SEQ ID NO:6, SEQ ID NO:7, or a fragment thereof. Additionally, the invention includes mutant or variant nucleic acids of SEQ ID NO:4, SEQ ID NO:5, SEQ ID NO:6, SEQ ID NO:7, or a fragment thereof, any of whose bases may be changed from the corresponding bases shown in SEQ ID NO:4, SEQ ID NO:5, SEQ ID NO:6 or SEQ ID NO:7, while still hybridizing to the *ob* gene DNA sequence. The invention further includes the complement of the nucleic acid sequence of SEQ ID NO:4, SEQ ID NO:5, SEQ ID NO:6 or SEQ ID NO:7, including fragments, derivatives, analogs and homologs thereof The invention additionally includes nucleic acids or nucleic acid fragments, or complements thereto, whose structures include chemical modifications.

The invention also includes an oligonucleotide that includes a portion of the disclosed nucleic acids. Advantageously, the oligonucleotide can be at least 10 nucleotides in length and include at least nine contiguous nucleotides of SEQ ID NO:4, SEQ ID NO:5, SEQ ID NO:6 or SEQ ID NO:7.

As to detection of the hybridization complex formed between probe and target, it is advantageous that the complex is labeled. Typically, in the step of determining hybridization of probe to target, there is a detection of the amount of labeled hybridization complex at the test site or a portion thereof. Any mode or modality of detection consistent with the purpose and functionality of the invention may be utilized, such as optical imaging, electronic imaging, use of charge-coupled devices or other methods of quantification. Labeling may be of the target, capture, or reporter. Various labeling may be by fluorescent labeling, colormetric labeling or chemiluminescent labeling. In yet another implementation, detection may be via energy transfer between molecules in the hybridization complex. In yet another aspect, the detection may be via fluorescence perturbation analysis. In another aspect the detection may be via conductivity differences between concordant and discordant sites.

In yet another aspect, detection can be carried out using mass spectrometry. In such method, no fluorescent label is necessary. Rather detection is obtained by extremely high levels of mass resolution achieved by direct measurement, for example, by time of flight or by electron spray ionization (ESI).

It is a further object of the present invention to provide such a method that provides to packers increased predictability of carcass grade of livestock purchased. It is a further object of the present invention to provide such a method that allows cow/calf operators to be able to respond to market signals from the feed lot more accurately by producing animals with a greater or lesser genetic predisposition to lay down fat.

Individual animals among assemblies of animals received at feeding facilities are segregated into groups based conventionally on weight and frame type, and additionally based on *ob* genotype. The animals are tested to determine homozygosity or heterozygosity with respect to alleles of the *ob* gene. Animals of such groups will, when maintained together on a uniform diet, exhibit greater body fat condition uniformity at any particular time after such segregation than is exhibited among animals grouped together using current practices.

Individual animals within such a group will attain a desired body condition closer to the time that other individual animals of the same group attain the desired body condition. Such, temporal uniformity exceeds that exhibited in groups of otherwise similarly situated animals maintained and fed together using current grouping practices.

It will be advantageous to optimize milk production to milk dairy cattle when they are genetically predisposed toward increased milk production (hereinafter TT cattle, i.e., cattle homozygous for the T SNP). Conversely, milking dairy cattle that are less genetically predisposed toward increased milk production (hereinafter CC cattle; i.e., homozygous for the c SNP or CT Cattle, i.e. heterozygous for the SNP) may result in less than optimal milk production.

In another embodiment, it will be advantageous to feed cattle to achieve a high fat grade when they are most genetically predisposed to lay down fat (TT cattle). As to those cattle least genetically predisposed to lay down fat (CC cattle), it will be advantageous to feed these cattle so as to achieve a lower fat grade, or a lean grade, rather than feed them longer to achieve the high fat grade. Those cattle intermediately genetically predisposed to lay down fat (CT cattle), can be fed longer to achieve a high fat grade, or shorter to achieve a lean grade, depending on considerations such as market prices, price trends, feed costs, availability of further feeder cattle to bring into the feed lot, and other like external considerations. On occasion such external considerations may dictate that CC cattle should be fed for a fat grade, however this will most often be so inefficient that such feeding would not be cost effective.

One embodiment of a method of livestock management according to the present invention provides a direct correlation between milk production in livestock animals and the presence of alleles of leptin gene, *i.e., ob.* During the first one hundred days of location, TT animals have the highest milk production. CC animals produce the least amount of milk and CT animals produce an intermediate amount of milk. During the third phase of growth, TT animals also have the highest feed conversion rate, whereas CC animals have the lowest feed conversion rate and CT animals have an intermediate feed conversion rate.

For a ton of feed eaten, TT animals, and particularly TT cattle, will gain the most weight, CC cattle will gain the least weight, and CT cattle will gain an intermediate amount of weight. Thus, for any given number of cattle, the amount of feed eaten per pound of weight gain will decrease as the occurrence of the *ob* T-allele increases. Further, by grouping cattle according to genotype, as in the method of the present invention, and feeding grouped cattle together, more uniform sized carcasses can be realized since cattle with more similar feed conversion rates will grow in a more similar manner when environmental conditions, such as feed content, are constant. A carcass with a certain minimum level of intramuscular fat will be graded AAA in Canada, corresponding to Choice Grade in the United States. At present such AAA carcasses will bring a premium payment for the feedlot operator.

The invention further comprises a kit for evaluating a sample of livestock DNA. At a minimum, the kit is a container with one or more reagents that identify a polymorphism in the livestock *ob* gene. Advantageously, the reagent is a probe or set of primers that hybridize with the livestock *ob* gene or fragments thereof. Advantageously, the probe is selected from SEQ ID NO:4 and SEQ ID NO:5 or a fragment thereof.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

It is noted that in this disclosure and particularly in the claims, terms such as "comprises", "comprised", "comprising" and the like can have the meaning attributed to it in U.S. Patent law; *e.g*., they can mean "includes", "included", "including", and the like; and that terms such as "consisting essentially of" and "consists essentially of" have the meaning ascribed to them in U.S. Patent law, *e.g*., they allow for elements not explicitly recited, but exclude elements that are found in the prior art or that affect a basic or novel characteristic of the invention.

These and other objects, features, and advantages of the invention become further apparent in the following detailed description of the invention when taken in conjunction with the accompanying drawings that illustrate, by way of example, the principles of this invention.

### BRIEF DESCRIPTION OF THE DRAWINGS

The following detailed description, given by way of example, but not intended to limit the invention solely to the specific embodiments described, may best be understood in conjunction with the accompanying drawings, in which:
FIG. 1 depicts non-esterified fatty acid (NEFA)-by leptin and days in milk (DIM),
FIG. 2 depicts beta-hydroxy butyrate (BHBA)-by leptin,

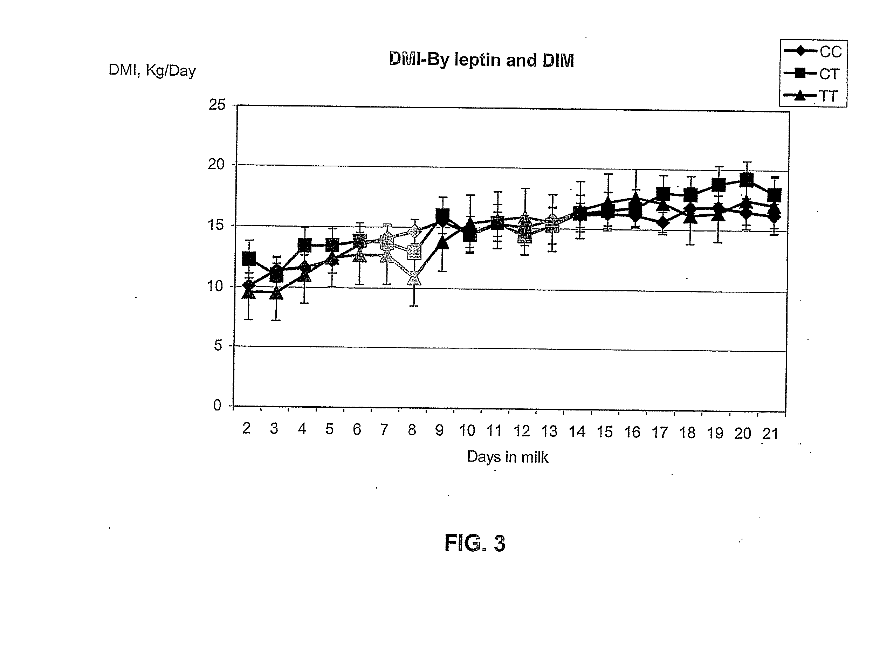

FIG. 3 depicts dry matter intake (DMI)-by leptin and DIM and

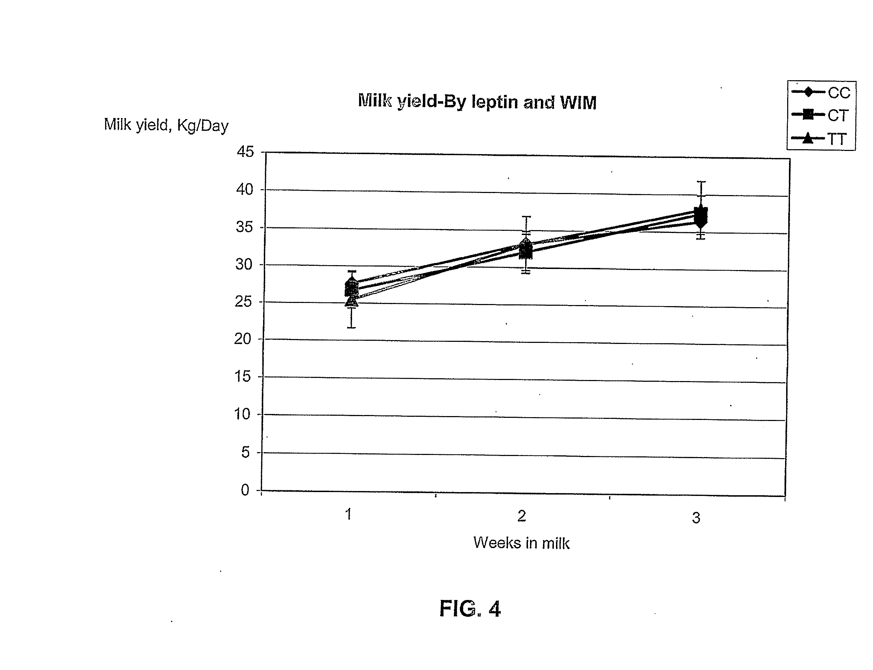

FIG. 4 depicts milk yield-by leptin and weeks in milk (WIM).

### DETAILED DESCRIPTION

In the description that follows, a number of terms are extensively utilized. In order to provide a clear and consistent understanding of the specification and claims, including the scope to be given such terms, the following terminology is provided:

An "amplification primer" is an oligonucleotide that is capable of annealing adjacent to a target sequence and serving as an initiation point for DNA synthesis when placed under conditions in which synthesis of a primer extension product which is complementary to a nucleic acid strand is initiated.

By "amplifying a segment" as used herein, is meant the production of sufficient multiple copies of the segment to permit relatively facile manipulation of the segment. Manipulation refers to both physical and chemical manipulation, that is, the ability to move bulk quantities of the segment around and to conduct chemical reactions with the segment that result in detectable products. A "segment" of a polynucleotide refers to an oligonucleotide that is a partial sequence of entire nucleotide sequence of the polynucleotide. A "modified segment" refers to a segment in which one or more natural nucleotides have been replaced with one or more modified nucleotides. A "modified, labeled segment refers to a modified segment that also contains a nucleotide, which is different from the modified nucleotide or nucleotides therein, and which is detectably labeled.

By "analysis" is meant either detection of variance in the nucleotide sequence among two or more related polynucleotides or, in the alternative, the determination of the full nucleotide sequence of a polynucleotide. By "analyzing" the hybridized fragments for an incorporated detectable label identifying the suspected polymorphism is meant that, at some stage of the sequence of events that leads to hybridized fragments, a label is incorporated. The label may be incorporated at virtually any stage of the sequence of events including the amplification, the cleavage or the hybridization procedures. The label may even be introduced into the sequence of events after cleavage but before hybridization or even after hybridization. The label so incorporated is then observed visually or by instrumental means. The presence of the label identifies the polymorphism due to the fact that the fragments obtained during cleavage are specific to the modified nucleotide(s) used in the amplification and at least one of the modified nucleotide is selected so as to replace a nucleotide involved in the polymorphism.

The term "animal" is used herein to include all vertebrate animals, including humans. It also includes an individual animal in all stages of development, including embryonic and fetal stages. As used herein, the term "production animals" is used interchangeably with "livestock animals" and refers generally to animals raised primarily for food. For example, such animals include, but are not limited to, cattle (bovine), sheep (ovine), pigs (porcine or swine), poultry (avian), and the like. As used herein, the term "cow" or "cattle" is used generally to refer to an animal of bovine origin of any age. Interchangeable terms include "bovine", "calf", "steer", "bull", "heifer" and the like. As used herein, the term "pig" or is used generally to refer to an animal of porcine origin of any age. Interchangeable terms include "piglet", "sow" and the like.

The term "antisense" is intended to refer to polynucleotide molecules complementary to a portion of an RNA marker of the *ob* gene, as defined herein. "Complementary" polynucleotides are those which are capable of base-pairing according to the standard Watson-Crick complementarity rules. That is, the larger purines will base pair with the smaller pyrimidines to form combinations of guanine paired with cytosine (G:C) and adenine paired with either thymine (A:T) in the case of DNA, or adenine paired with uracil (A:U) in the case of RNA. Inclusion of less common bases such as inosine, 5-methylcytosine, 6-methyladenine, hypoxanthine and others in hybridizing sequences does not interfere with pairing.

By the term "complementarity" or "complementary" is meant, for the purposes of the specification or claims, a sufficient number in the oligonucleotide of complementary base pairs in its sequence to interact specifically (hybridize) with the target nucleic acid sequence of the *ob* gene polymorphism to be amplified or detected. As known to those skilled in the art, a very high degree of complementarity is needed for specificity and sensitivity involving hybridization, although it need not be 100%. Thus, for example, an oligonucleotide that is identical in nucleotide sequence to an oligonucleotide disclosed herein, except for one base change or substitution, may function equivalently to the disclosed oligonucleotides. A "complementary DNA" or "cDNA" gene includes recombinant genes synthesized by reverse transcription of messenger RNA ("mRNA").

By the term "composition" is meant, for the purposes of the specification or claims, a combination of elements which may include one or more of the following: the reaction buffer for the respective method of enzymatic amplification, plus one or more oligonucleotides specific for *ob* gene polymorphisms, wherein said oligonucleotide is labeled with a detectable moiety.

By the terms "consisting essentially of a nucleotide sequence" is meant, for the purposes of the specification or claims, the nucleotide sequence disclosed, and also encompasses nucleotide sequences which are identical except for a one base change or substitution therein.

A "cyclic polymerase-mediated reaction" refers to a biochemical reaction in which a template molecule or a population of template molecules is periodically and repeatedly copied to create a complementary template molecule or complementary template molecules, thereby increasing the number of the template molecules over time.

"Denaturation" of a template molecule refers to the unfolding or other alteration of the structure of a template so as to make the template accessible to duplication. In the case of DNA, "denaturation" refers to the separation of the two complementary strands of the double helix, thereby creating two complementary, single stranded template molecules. "Denaturation" can be accomplished in any of a variety of ways, including by heat or by treatment of the DNA with a base or other denaturant.

A "detectable amount of product" refers to an amount of amplified nucleic acid that can be detected using standard laboratory tools. A "detectable marker" refers to a nucleotide analog that allows detection using visual or other means. For example, fluorescently labeled nucleotides can be incorporated into a nucleic acid during one or more steps of a cyclic polymerase-mediated reaction, thereby allowing the detection of the product of the reaction using, *e.g*. fluorescence microscopy or other fluorescence-detection instrumentation.

By the term "detectable moiety" is meant, for the purposes of the specification or claims, a label molecule (isotopic or non-isotopic) which is incorporated indirectly or directly into an oligonucleotide, wherein the label molecule facilitates the detection of the oligonucleotide in which it is incorporated when the oligonucleotide is hybridized to amplified *ob* gene polymorphisms sequences. Thus, "detectable moiety" is used synonymously with "label molecule". Synthesis of oligonucleotides can be accomplished by any one of several methods known to those skilled in the art. Label molecules, known to those skilled in the art as being useful for detection, include chemiluminescent or fluorescent molecules. Various fluorescent molecules are known in the art which are suitable for use to label a nucleic acid substrate for the method of the present invention. The protocol for such incorporation may vary depending upon the fluorescent molecule used. Such protocols are known in the art for the respective fluorescent molecule.

By "detectably labeled" is meant that a fragment or an oligonucleotide contains a nucleotide that is radioactive, that is substituted with a fluorophore or some other molecular species that elicits a physical or chemical response can be observed by the naked eye or by means of instrumentation such as, without limitation, scintillation counters, colorimeters, UV spectrophotometers and the like. As used herein, a "label" or "tag" refers to a molecule that, when appended by, for example, without limitation, covalent bonding or hybridization, to another molecule, for example, also without limitation, a polynucleotide or polynucleotide fragment, provides or enhances a means of detecting the other molecule. A fluorescence or fluorescent label or tag emits detectable light at a particular wavelength when excited at a different wavelength. A radiolabel or radioactive tag emits radioactive particles detectable with an instrument such as, without limitation, a scintillation counter. Other signal generation detection methods include: chemiluminescence, electrochemiluminescence, raman, colorimetric, hybridization protection assay, and mass spectrometry

"DNA amplification" as used herein refers to any process that increases the number of copies of a specific DNA sequence by enzymatically amplifying the nucleic acid sequence. A variety of processes are known. One of the most commonly used is the polymerase chain reaction (PCR) process of Mullis as described in U.S. Pat. Nos. 4,683,195 and 4,683,202. PCR involves the use of a thermostable DNA polymerase, known sequences as primers, and heating cycles, which separate the replicating deoxyribonucleic acid (DNA), strands and exponentially amplify a gene of interest. Any type of PCR, such as quantitative PCR, RT-PCR, hot start PCR, LAPCR, multiplex PCR, touchdown PCR, *etc.*, may be used. Advantageously, real-time PCR is used. In general, the PCR amplification process involves an enzymatic chain reaction for preparing exponential quantities of a specific nucleic acid sequence. It requires a small amount of a sequence to initiate the chain reaction and oligonucleotide primers that will hybridize to the sequence. In PCR the primers are annealed to denatured nucleic acid followed by extension with an inducing agent (enzyme) and nucleotides. This results in newly synthesized extension products. Since these newly synthesized sequences become templates for the primers, repeated cycles of denaturing, primer annealing, and extension results in exponential accumulation of the specific sequence being amplified. The extension product of the chain reaction will be a discrete nucleic acid duplex with a termini corresponding to the ends of the specific primers employed.

"DNA" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia,* in linear DNA molecules (*e.g.,* restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (*i.e.,* the strand having a sequence homologous to the mRNA).

By the terms "enzymatically amplify" or "amplify" is meant, for the purposes of the specification or claims, DNA amplification, *i.e*., a process by which nucleic acid sequences are amplified in number. There are several means for enzymatically amplifying nucleic acid sequences. Currently the most commonly used method is the polymerase chain reaction (PCR). Other amplification methods include LCR (ligase chain reaction) which utilizes DNA ligase, and a probe consisting of two halves of a DNA segment that is complementary to the sequence of the DNA to be amplified, enzyme QB replicase and a ribonucleic acid (RNA) sequence template attached to a probe complementary to the DNA to be copied which is used to make a DNA template for exponential production of complementary RNA; strand displacement amplification (SDA); Qβ replicase amplification (QßRA); self-sustained replication (3SR); and NASBA (nucleic acid sequence-based amplification), which can be performed on RNA or DNA as the nucleic acid sequence to be amplified.

The "extension of the primer molecules" refers to the addition of nucleotides to a primer molecule so as to synthesize a nucleic acid complementary to a template molecule. "Extension of the primer molecules" does not necessarily imply that the primer molecule is extended to synthesize a complete complementary template molecule. Rather, even if only a fraction of the template molecule has been copied, the primer is still considered extended.

A "fragment" of a molecule such as a protein or nucleic acid is meant to refer to any portion of the amino acid or nucleotide genetic sequence.

As used herein, "fluorescence resonance energy transfer pair" or "FRET pair" refers to a pair of fluorophores comprising a donor fluorophore and acceptor fluorophore, wherein the donor fluorophore is capable of transferring resonance energy to the acceptor fluorophore. In other words the emission spectrum of the donor fluorophore overlaps the absorption spectrum of the acceptor fluorophore. In advantageous fluorescence resonance energy transfer pairs, the absorption spectrum of the donor fluorophore does not substantially overlap the absorption spectrum of the acceptor fluorophore. As used herein, "a donor oligonucleotide probe" refers to an oligonucleotide that is labeled with a donor fluorophore of a fluorescent resonance energy transfer pair. As used herein, "an acceptor oligonucleotide probe" refers to an oligonucleotide that is labeled with an acceptor fluorophore of a fluorescent resonance energy transfer pair. As used herein, "FRET oligonucleotide pair" refers to the donor oligonucleotide probe and the acceptor oligonucleotide probe pair that form a fluorescence resonance energy transfer relationship when the donor oligonucleotide probe and the acceptor oligonucleotide probe are both hybridized to their complementary target nucleic acid sequences. Two separate FRET oligonucleotide pairs, each specific for one locus and each comprising a different acceptor dye may be used at the same time. Acceptable fluorophore pairs for use as fluorescent resonance energy transfer pairs are well know to those skilled in the art and include, but are not limited to, fluorescein/rhodamine, phycoerythrin/Cy7, fluorescein/Cy5, fluorescein/Cy5.5, fluorescein/LC Red 640, and fluorescein/LC Red 705.

A "functional derivative" of a sequence, either protein or nucleic acid, is a molecule that possesses a biological activity (either functional or structural) that is substantially similar to a biological activity of the protein or nucleic acid sequence. A functional derivative of a protein may or may not contain post-translational modifications such as covalently linked carbohydrate, depending on the necessity of such modifications for the performance of a specific function. The term "functional derivative" is intended to include the "fragments," "segments," "variants," "analogs," or "chemical derivatives" of a molecule.

As used herein, the term "genome" refers to all the genetic material in the chromosomes of a particular organism. Its size is generally given as its total number of base pairs. Within the genome, the term "gene" refers to an ordered sequence of nucleotides located in a particular position on a particular chromosome that encodes specific functional product (*e.g*., a protein or RNA molecule). For example, it is known that the protein leptin is encoded by the *ob* (obese) gene and appears to be involved in the regulation of appetite, basal metabolism and fat deposition In general, an animal's genetic characteristics, as defined by the nucleotide sequence of its genome, are known as its "genotype," while the animal's physical traits are described as its "phenotype."

By "heterozygous" or "heterozygous polymorphism" is meant that the two alleles of a diploid cell or organism at a given locus are different, that is, that they have a different nucleotide exchanged for the same nucleotide at the same place in their sequences.

By "homozygous" is meant that the two alleles of a diploid cell or organism at a given locus are identical, that is, that they have the same nucleotide for nucleotide exchange at the same place in their sequences.

By "hybridization" or "hybridizing," as used herein, is meant the formation of A-T and C-G base pairs between the nucleotide sequence of a fragment of a segment of a polynucleotide and a complementary nucleotide sequence of an oligonucleotide. By complementary is meant that at the locus of each A, C, G or T (or U in a ribonucleotide) in the fragment sequence, the oligonucleotide sequenced has a T, G, C or A, respectively. The hybridized fragment/oligonucleotide is called a "duplex."

A "hybridization complex", such as in a sandwich assay, means a complex of nucleic acid molecules including at least the target nucleic acid and sensor probe. It may also include an anchor probe.

By "immobilized on a solid support" is meant that a fragment, primer or oligonucleotide is attached to a substance at a particular location in such a manner that the system containing the immobilized fragment, primer or oligonucleotide may be subjected to washing or other physical or chemical manipulation without being dislodged from that location. A number of solid supports and means of immobilizing nucleotide-containing molecules to them are known in the art; any of these supports and means may be used in the methods of this invention.

As used herein, the term "increased weight gain" means a biologically significant increase in weight gain above the mean of a given population.

As used herein, the term "locus" or "loci" refers to the site of a gene on a chromosome. Pairs of genes, known as "alleles" control the hereditary trait produced by a gene locus. Each animal's particular combination of alleles is referred to as its "genotype". Where both alleles are identical the individual is said to be homozygous for the trait controlled by that gene pair; where the alleles are different, the individual is said to be heterozygous for the trait.

A "melting temperature" is meant the temperature at which hybridized duplexes dehybridize and return to their single-stranded state. Likewise, hybridization will not occur in the first place between two oligonucleotides, or, herein, an oligonucleotide and a fragment, at temperatures above the melting temperature of the resulting duplex. It is presently advantageous that the difference in melting point temperatures of oligonucleotide-fragment duplexes of this invention be from about 1°C to about 10°C so as to be readily detectable.

As used herein, the term "nucleic acid molecule" is intended to include DNA molecules (*e.g*., cDNA or genomic DNA), RNA molecules (*e.g*., mRNA), analogs of the DNA or RNA generated using nucleotide analogs, and derivatives, fragments and homologs thereof. The nucleic acid molecule can be single-stranded or double-stranded, but advantageously is double-stranded DNA. An "isolated" nucleic acid molecule is one that is separated from other nucleic acid molecules that are present in the natural source of the nucleic acid. A "nucleoside" refers to a base linked to a sugar. The base may be adenine (A), guanine (G) (or its substitute, inosine (I)), cytosine (C), or thymine (T) (or its substitute, uracil (U)). The sugar may be ribose (the sugar of a natural nucleotide in RNA) or 2-deoxyribose (the sugar of a natural nucleotide in DNA). A "nucleotide" refers to a nucleoside linked to a single phosphate group.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides may be chemically synthesized and may be used as primers or probes. Oligonucleotide means any nucleotide of more than 3 bases in length used to facilitate detection or identification of a target nucleic acid, including probes and primers.

"Polymerase chain reaction" or "PCR" refers to a thermocyclic, polymerase-mediated, DNA amplification reaction. A PCR typically includes template molecules, oligonucleotide primers complementary to each strand of the template molecules, a thermostable DNA polymerase, and deoxyribonucleotides, and involves three distinct processes that are multiply repeated to effect the amplification of the original nucleic acid. The three processes (denaturation, hybridization, and primer extension) are often performed at distinct temperatures, and in distinct temporal steps. In many embodiments, however, the hybridization and primer extension processes can be performed concurrently. The nucleotide sample to be analyzed may be PCR amplification products provided using the rapid cycling techniques described in U.S. Pat. Nos. 6,569,672; 6,569,627; 6,562,298; 6,556,940; 6,569,672; 6,569,627; 6,562,298; 6,556,940; 6,489,112; 6,482,615; 6,472,156; 6,413,766; 6,387,621; 6,300,124; 6,270,723; 6,245,514; 6,232,079; 6,228,634; 6,218,193; 6,210,882; 6,197,520; 6,174,670; 6,132,996; 6,126,899; 6,124,138; 6,074,868; 6,036,923; 5,985,651; 5,958,763; 5,942,432; 5,935,522; 5,897,842; 5,882,918; 5,840,573; 5,795,784; 5,795,547; 5,785,926; 5,783,439; 5,736,106; 5,720,923; 5,720,406; 5,675,700; 5,616,301; 5,576,218 and 5,455,175, the disclosures of which are incorporated by reference in their entireties. Other methods of amplification include, without limitation, NASBR, SDA, 3SR, TSA and rolling circle replication. It is understood that, in any method for producing a polynucleotide containing given modified nucleotides, one or several polymerases or amplification methods may be used. The selection of optimal polymerization conditions depends on the application.

A "polymerase" is an enzyme that catalyzes the sequential addition of monomeric units to a polymeric chain, or links two or more monomeric units to initiate a polymeric chain. In advantageous embodiments of this invention, the "polymerase" will work by adding monomeric units whose identity is determined by and which is complementary to a template molecule of a specific sequence. For example, DNA polymerases such as DNA pol 1 and Taq polymerase add deoxyribonucleotides to the 3' end of a polynucleotide chain in a template-dependent manner, thereby synthesizing a nucleic acid that is complementary to the template molecule. Polymerases may be used either to extend a primer once or repetitively or to amplify a polynucleotide by repetitive priming of two complementary strands using two primers.

A "polynucleotide" refers to a linear chain of nucleotides connected by a phosphodiester linkage between the 3'-hydroxyl group of one nucleoside and the 5'-hydroxyl group of a second nucleoside which in turn is linked through its 3'-hydroxyl group to the 5'-hydroxyl group of a third nucleoside and so on to form a polymer comprised of nucleosides liked by a phosphodiester backbone. A "modified polynucleotide" refers to a polynucleotide in which one or more natural nucleotides have been partially or substantially completely replaced with modified nucleotides.

A "primer" is a short oligonucleotide, the sequence of which is complementary to a segment of the template which is being replicated, and which the polymerase uses as the starting point for the replication process. By "complementary" is meant that the nucleotide sequence of a primer is such that the primer can form a stable hydrogen bond complex with the template; *i.e*., the primer can hybridize to the template by virtue of the formation of base-pairs over a length of at least ten consecutive base pairs.

The primers herein are selected to be "substantially" complementary to different strands of a particular target DNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to hybridize therewith and thereby form the template for the synthesis of the extension product.

"Probes" refer to nucleic acid sequences of variable length, used in the detection of identical, similar, or complementary nucleic acid sequences by hybridization. An oligonucleotide sequence used as a detection probe may be labeled with a detectable moiety. Various labeling moieties are known in the art. Said moiety may, for example, either be a radioactive compound, a detectable enzyme (*e.g*. horse radish peroxidase (HRP)) or any other moiety capable of generating a detectable signal such as a calorimetric, fluorescent, chemiluminescent or electrochemiluminescent signal. The detectable moiety may be detected using known methods. In one embodiment the probe oligomers are generally 8 to 44-mers and advantageously about 10 to 12-mers and advantageously about 11-mers.

As used herein, the term "protein" refers to a large molecule composed of one or more chains of amino acids in a specific order. The order is determined by the base sequence of nucleotides in the gene coding for the protein. Proteins are required for the structure, function, and regulation of the body's cells, tissues, and organs. Each protein has a unique function.

As used herein, the terms "quality traits" or "physical characteristics" refer to advantageous properties of the animal resulting from genetics. Quality traits include, but are not limited to, the animal's genetic ability to metabolize energy, produce milk, put on intramuscular fat, lay eggs, produce offspring, produce particular proteins in meat or milk, or retain protein in milk. Physical characteristics include marbled or lean meats. The terms are used interchangeably.

A "restriction enzyme" refers to an endonuclease (an enzyme that cleaves phosphodiester bonds within a polynucleotide chain) that cleaves DNA in response to a recognition site on the DNA. The recognition site (restriction site) consists of a specific sequence of nucleotides typically about 4-8 nucleotides long.

A "single nucleotide polymorphism" or "SNP" refers to polynucleotide that differs from another polynucleotide by a single nucleotide exchange. For example, without limitation, exchanging one A for one C, G or T in the entire sequence of polynucleotide constitutes a SNP. Of course, it is possible to have more than one SNP in a particular polynucleotide. For example, at one locus in a polynucleotide, a C may be exchanged for a T, at another locus a G may be exchanged for an A and so on. When referring to SNPs, the polynucleotide is most often DNA and the SNP is one that usually results in a deleterious change in the genotype of the organism in which the SNP occurs.

As used herein, a "template" refers to a target polynucleotide strand, for example, without limitation, an unmodified naturally-occurring DNA strand, which a polymerase uses as a means of recognizing which nucleotide it should next incorporate into a growing strand to polymerize the complement of the naturally-occurring strand. Such DNA strand may be single-stranded or it may be part of a double-stranded DNA template. In applications of the present invention requiring repeated cycles of polymerization, *e.g*., the polymerase chain reaction (PCR), the template strand itself may become modified by incorporation of modified nucleotides, yet still serve as a template for a polymerase to synthesize additional polynucleotides.

A "thermocyclic reaction" is a multi-step reaction wherein at least two steps are accomplished by changing the temperature of the reaction.

A "thermostable polymerase" refers to a DNA or RNA polymerase enzyme that can withstand extremely high temperatures, such as those approaching 100°C. Often, thermostable polymerases are derived from organisms that live in extreme temperatures, such as *Thermus aquaticus.* Examples of thennostable polymerases include Taq, Tth, Pfu, Vent, deep vent, UlTma, and variations and derivatives thereof.

A "variance" is a difference in the nucleotide sequence among related polynucleotides. The difference may be the deletion of one or more nucleotides from the sequence of one polynucleotide compared to the sequence of a related polynucleotide, the addition of one or more nucleotides or the substitution of one nucleotide for another. The terms "mutation," "polymorphism" and "variance" are used interchangeably herein. As used herein, the term "variance" in the singular is to be construed to include multiple variances; *i.e*., two or more nucleotide additions, deletions and/or substitutions in the same polynucleotide. A "point mutation" refers to a single substitution of one nucleotide for another.

A typical growth curve is correlated with the weight of production animals. Present production practices vary among the specific industries as to the point on the curve at which the animal is considered ready for slaughter. For poultry and pigs, for example, present practice is to slaughter near the beginning of phase three, where the growth curve begins to flatten. At this portion of the curve, the amount of time and feed required to produce a pound of gain increase, so economics dictates that the animal should be slaughtered at that time and replaced in the feeding facility with an animal in the second phase where weight gain is much more rapid and efficient in terms of feed conversion.

The growth curve can also be correlated with milk production in dairy cattle.

Following the successful live birth of a heifer calf, the dairy farmer begins to manage that animal toward the goal of successfully breeding that animal at 15 months of age. The heifer must be quickly and effectively moved through a diet of milk replacer and on to roughage diets to ensure that she attains a target weight gain. In the case of Holsteins, she must reach a minimum weight of 800 lbs when she is successfully bred at 15 months of age.

During the early phase of life, the heifer is subject to illness and so a fully functional immune system is very important. Leptin has been shown to have a moderating influence on elements of the inflammatory response and can play a role in an immune function (Houseknecht). Leptin genotype, and the resulting changes in structure and function of the protein, can influence the effectiveness of the protein in moderating immune function.

Attainment of appropriate growth during the time period from birth to 15 months of age is required to ensure the heifer will make her target weight. Leptin has been shown to influence growth rate and carcass composition in growing cattle (Geary) and the effect of the leptin genotype on body composition in cattle is well described in previous patent applications.

As the animal grows, it will eventually attain puberty. Leptin has a poweful influence on reproduction (Margetic; Williams) and puberty (Amstalden; Baker). Alterations in the central or peripheral control of reproduction via changes in leptin function resulting from the genotype differences has the potential to significantly influence the attainment of puberty and successful breeding in heifers.

In the time period up to 15 months, and from 15 months through to calving, the heifer must develop a normal udder. Leptin is present in fat tissue in the udder and normal development of the udder - required for proper lactation performance - is affected by leptin and will likely be affected by leptin genotype (Silva; Chilliard).

The time period from calving through to peak lactation (approximately the first 100 days of lactation), is the most stressful period in the life of the dairy cow. That animal must move quickly from essentially a roughage diet to one which is high in energy value (concentrate) in order that she can meet the huge energy demands of lactation. During this time period, her appetite and daily feed intake is increasing but unable to keep pace with the increased energy demand of lactation and so she falls into negative energy balance. This period of negative energy balance makes her susceptible to metabolic disease and the relationship between leptin, glucose, non-esterified fatty acids and beta-hydroxybutyrate in the regulation of energy production in the post calving dairy cow is extremely important (Delavaud). Recent work from studies examining the relationship between leptin genotype and energy balance in post-calving dairy cows suggest that genotype will have a major impact on energy balance in the post-calving period (Leslie, et at - see attached).

A variety of studies have shown the relationship between leptin concentration and milk yield, feed intake, live weight and estrus in dairy cattle (Leifers; Leifers). The ability of the cow to manage the energy demands of lactation, increase her feed consumption and come back into estrus in order that she can be bred in a timely fashion is extremely challenging. Because leptin is a hormone that is central to regulation of feed intake, the leptin polymorphism will likely influence feed intake, energy balance, milk yield and reproduction, the polymorphism will be shown to be significantly related to all of these events, and management of animals by gentoype will be central to efficient dairy production.

Assuming that the cows becomes pregnant, then she must retain the calf during the remainder of her lactation. Approximately 45-60 days prior to her next lactation, the cows is "dried off' and prepared for her next lactation. During late lactation, the cow has returned to positive energy balance and she is beginning to lay down body fat reserves in preparation for her next lactation. It has been shown that the leptin polymorphism will result in alterations in circulating leptin concentration in the period immediately prior to calving which will influence body condition score, feed intake and preparedness for calving and subsequent lactation (Leifers). Managing cows by genotype during this time period, and altering rations as a result will help to ensure that there production can be maximized from all cows.

The present invention differs from current practice by using genetic test results to identify, select and group the animals. Rather than rely on a growth curve or visual inspection of animal traits, the present invention allows the farmer to milk and feed livestock according to the individual animal's genetic traits. According to the method of the present invention, it is possible to select a desired trait, such as milk production, identify the polypeptide which specifically encodes for a gene associate with that trait, and genotype animals possessing the associated gene.

Leptin, a 16-kDa adipocyte-specific polypeptide, is encoded by the *ob* (obese) gene and appears to be involved in the regulation of appetite, basal metabolism, fat deposition and milk production. The *ob* gene has been mapped to specific chromosomes in several different animals, allowing the gene to be sequenced in several different species. In the case of leptin, there is significant conservation among the sequences of *ob* DNAs and leptin polypeptides from the tested species. Mutations in the coding sequences of the *ob* gene causing alterations in the amino acid sequence of the leptin polypeptide have been associated with hyperphagia, hypometabolic activity, and excessive fat deposition, i.e., a phenotype characterized by larger body size (a fat phenotype). In the method of the present invention, it is possible to identify the absence or presence of a specific *ob* allele, thus predicting which animals will or will not possess certain carcass characteristics, e.g., increased fat deposition, increased mean fat deposition, increased percent rib fat, and decreased percent rib lean. For the *ob* gene, the presence of 138-bp allele was positively associated with these characteristics. Thus, bulls homogenous for the 138-bp allele exhibited greater average fat deposition than heterozygous animals.

The present invention provides methods wherein the genetic information obtained from individual animals is cross-matched against markers known in the art to predict specific characteristics. A cytosine (C) to thymine (T) transition within an exon (exon 2) of the *ob* gene correspond to an arginine (ARG) to cysteine (CYS) substitution in the leptin polypeptide. The exon 2 polymorphism is a C/T substitution located at position 305 of exon 2 of the bovine leptine gene (see, e.g., Buchanan et al. (2002) Genet Sel Evol. 34(1):105-16). Thus, once the genotype of the animal is determined, it is evaluated to determine whether each individual animal possesses the desired trait, i.e., possesses the specific gene. Animals having like genotypes for a specific gene/characteristic are then grouped together. These like-genotype groupings serve as the basis for breeding, feeding, milking and determining slaughter time. Accordingly, the like-genotype groupings provide a more objective method for determining mates for breeding, diets and lengths of feed cycles, milking and slaughter times.

The individual genotype data of each animal can be recorded and associated with various other data of the animal, e.g. health information, parentage, vaccination history, herd records, and the like. Such information can be forwarded to a government agency to provide traceability of a meat product, or it may serve as the basis for breeding, feeding and marketing information. Once the genotype data is established, and that data may or may not be associated with other data, the data is stored in an accessible database, such as a computer database or a microchip implanted in the animal.

Genetic tendencies can be predicted by the results of genotyping. A method and system of the invention comprises tissue sampling, extraction of genetic material from the sampled tissue, molecular genetic analysis of the genetic material, and where the tissue sample is taken from a meat product, comparison of the genotype with known animal genotypes stored on a database. It is contemplated by the methods and systems described herein that the continuity and integrity of each sample is maintained so that the data is accurate and reliable. Steps necessary for ensuring that the data is accurate and reliable are included in the methods and systems taught herein.

Additionally, the method of the present invention contemplates grouping animals according to their genotype in addition to using the phenotype criteria currently employed in feeding, breeding or growing stages practices. For example, in one embodiment of the present invention, feedlot operators who currently group livestock according to size and frame structures, among other phenotypic traits, would use the data obtained from animals' genotypes which correspond to an animal's propensity to exhibit a characteristic associated with the particular gene, and optionally any other associated data, in order to more efficiently manage production. Thus, the feeder is presented with opportunities for considerable efficiencies in livestock production.

Presently, the feeder feeds all his cattle the same, incurring the same costs for each animal, and typically, with excellent management practices, perhaps 40% will receive an optimal grade of Prime, and receive the premium price for the palatability grade. Of these, a significant number will have excess fat and will thus receive a reduced yield grade. The balance of the cattle, 60%, will grade less than Prime, and thus receive a reduced price, although the feed lot costs incurred by the feeder are substantially the same for these cattle receiving the lesser grade. Grouping and feeding the cattle by genotype allows the feeder to treat each group differently with a view to optimizing management strategies and increasing profits.

A tissue sample may be taken from an animal at any time in the lifetime of an animal but before the carcass identity is lost. The tissue sample can comprise hair, including roots, hide, bone, buccal swabs, blood, saliva, milk, semen, embryos, muscle or any internal organs.

The tissue sample is marked with an identifying number or other indicia that relates the sample to the individual animal from which the sample was taken. The identity of the sample advantageously remains constant throughout the methods and systems of the invention thereby guaranteeing the integrity and continuity of the sample during extraction and analysis. Alternatively, the indicia may be changed in a regular fashion that ensures that the data, and any other associated data, can be related back to the animal from which the data was obtained.

The amount/size of sample required is known to those skilled in the art and for example, can be determined by the subsequent steps used in the method and system of the invention and the specific methods of analysis used. Ideally, the size/volume of the tissue sample retrieved should be as consistent as possible within the type of sample and the species of animal. For example, for cattle, non-limiting examples of sample sizes/methods include non-fatty meat: 0.0002g-0.0010g; hide: 0.0004g--0.0010g; hair roots: greater than five and less than twenty; buccal swabs: 15 to 20 seconds of rubbing with modest pressure in the area between outer lip and gum using one Cytosoft® cytology brush; bone: 0.0020 g--0.0040 g; blood: 30 to 70µL.

Generally, the tissue sample is placed in a container that is labeled using a numbering system bearing a code corresponding to the animal, for example, to the animal's ear tag. Accordingly, the genotype of a particular animal is easily traceable at all times.

The tissue sample is then treated by the desired methods to retrieve the desired data, for example, such as fat content or genotype. Alternatively, the samples can be frozen for preservation and archived, for example, in the factory/slaughterhouse or a central storage location for future extraction/analysis as required.

In an advantageous embodiment of the invention, a sampling device and/or container is supplied to the farmer, a slaughterhouse or retailer. The sampling device advantageously takes a consistent and reproducible sample from individual animals while simultaneously avoiding any cross-contamination of tissue. Accordingly, the size and volume of sample tissues derived from individual animals would be consistent.

In the present invention, a sample of genomic DNA is obtained from a livestock. Generally, hair is used as the source of the DNA. A sufficient amount of cells are obtained to provide a sufficient amount of DNA for analysis. This amount will be known or readily determinable by those skilled in the art. The DNA is isolated from the blood cells by techniques known to those skilled in the art (see, e.g., U.S. Patent Nos. 6,548,256 and 5,989,431, Hirota et al., Jinrui Idengaku Zasshi. 1989 Sep;34(3):217-23 and John et al., Nucleic Acids Res. 1991 Jan 25;19(2):408; the disclosures of which are incorporated by reference in their entireties).

In the method of the present invention, the source of the test nucleic acid is not critical. For example, the test nucleic acid can be obtained from cells within a body fluid of the livestock or from cells constituting a body tissue of the subject. The particular body fluid from which the cells are obtained is also not critical to the present invention. For example, the body fluid may be selected from the group consisting of blood, ascites, pleural fluid and spinal fluid. Furthermore, the particular body tissue from which cells are obtained is also not critical to the present invention. For example, the body tissue may be selected from the group consisting of skin, endometrial, uterine and cervical tissue. Both normal and tumor tissues can be used. Further, the source of the target material may include RNA or mitochondrial DNA.

The invention further comprises methods of screening livestock to determine those having predictably increased milk production on based upon the presence or absence of certain polymorphisms in the *ob* gene. In an advantageous embodiment, the *ob* gene polymorphism is a C to T transition that results in an Arg25Cys in the leptin protein.

Any *ob* gene corresponding to the animal of interest can be used to identify the polymorphism(s) of interest in the *ob* gene. The *ob* gene that has been mapped to chromosome 6 in mice (Friedman & Leibel, 1992, Cell 69: 217-220), chromosome 7q31.3 in humans (Isse et al., 1995, J. Bio. Chem. 270: 27728-27733) chromosome 4 in cattle (Stone et al. 1996, Mamm. Genome 7: 399-400), and chromosome 18 in swine (Neuenschwander et al., 1996, Anim. Genet. 27: 275-278; Saskai et al., 1996, Mamm. Genome 7: 471-471). Sequences have been determined for the *ob* gene from mice (Zhang et al., 1994, Nature 372: 425-432), cattle (U.S. Patent No. 6,297,027 to Spurlock), pigs (U.S. Patent No. 6,277,592 and Neuenschwander et al., 1996, Anim. Genet. 27: 275-278), and humans (U.S. Patent No. 6,309,857) and there is significant conservation among the sequences of *ob* DNAs and leptin polypeptides from those species (Bidwell et al. 1997, Anim, Endocrinol. 8: 191-206; Ramsay et al. 1998, J. Anim. Sci. 76: 484-490).

In an advantageous embodiment, the *ob* sequence is a cattle *ob* sequence with the nucleotide sequence 
5'TCTGAAGACCTGGATGCGGGTGGTAACGGAGCACGTGGGTGTTCTCGGAGATC
GACGATGTGCCACGTGTGGTTTCTTCTGTTTTCAGGCCCCAGAAGCCCATCCCGG
GAAGGAAAATGCGCTGTGGACCCCTGTATCGATTCCTGTGGCTTTGGCCCTATCT
GTCTTACGTGGAGGCTGTGCCCATCTGCAAGGTCCAGGATGACACCAAAACCCTC
ATCAAGACAATTGTCACCAGGATCAATGACATCTCACACACGGTAGGGAGGGAC
TGGGAGACGAGGTAGAACCGTGGCCATCCCGTGGGGGACCCCAGAGGCTGGCGG
AGGAGGCTGTGCAGCCTTGCACAGGGCCCCAGTGGCCTGGACGCCCCCCTGGCA
TAAAGACAGCTCCTCTCCTCCTCCACTTCCCTTGCCTCCCGCCTTCTCACTCTCCT
CCCTCCCAGACCGGAATCCTAGTGCCCAGGCCCAGAAGGAGTCACAGAGGTCCT
GGGGTCCCCTTGGCAGGTGGCCAGAACCCCAGCAGCAGTCCCTCTGGGCCTCCAT
CTCATTTCTAGAATGTTTTAGTCGTTAGGCATTCTTCCTGCCTGGTAACTG 3' (SEQ
 ID NO:1), which contains the single nucleotide polymorphism at position 189. In another advantageous embodiment, the *ob* sequence is a cattle *ob* sequence with the nucleotide sequence 
5’TCTGAAGACCTGGATGCGGGTGGTAACGGAGCACGTGGGTGTTCTCGGAGATC
GACGATGTGCCACGTGTGGTTTCTTCTGTTTTCAGGCCCCAGAAGCCATCCCGG
GAAGGAAAATGCGCTGTGGACCCCTGTATCGATTCCTGTGGCTTTGGCCCTATCT
GTCTTACGTGGAGGCTGTGCCCATCCGCAAGGTCCAGGATGACACCAAAACCCTC
ATCAAGACAATTGTCACCAGGATCAATGACATCTCACACACGGTAGGGAGGGAC
TGGGAGACGAGGTAGAACCGTGGCCATCCCGTGGGGGACCCCAGAGGCTGGCGG
AGGAGGCTGTGCAGCCTTGCACAGGGCCCCAGTGGCCTGGACGCCCCCTGGCA
TAAAGACAGCTCCTCTCCTCCTCCACTTCCCTTGCCTCCCGCCTTCTCACTCTCCT
CCCTCCCAGACCGGAATCCTAGTGCCCAGGCCCAGAAGGAGTCACAGAGGTCCT
GGGGTCCCCTTGGCAGGTGGCCAGAACCCCAGCAGCAGTCCCTCTGGGCCTCCAT
CTCATTTCTAGAATGTTTTAGTCGTTAGGCATTCTTCCTGCCTGGTAACTG 3’ (SEQ
 ID NO:2), which does not contain the single nucleotide polymorphism at position 189. In another embodiment, the bovine *ob* nucleotide sequence can be selected from any one of the sequences corresponding to GenBank Accession Nos. AB003143, AB070368, AB070369, AE003406, AF120500, AF536174, AJ132764, AJ236854, AJ512638, AJ512639, AJ571671, AJ580799, AJ580800, AJ580801, AR171261, AR171262, AR171263, AR171264, AR171265, AY044438, AY138588, NM_000594, NM_000600, NM_000758, M_173926, NM_173928, NM_174140, NM_174216, NM_180996, U50365, U62385, U65793, U83512 and Y11369.

In an advantageous embodiment, the *ob* sequence is a cattle *ob* sequence with the amino acid sequence 
MRCGPLYRFLWLWPYLSYVEAVPIRKVQDDTKTLIKTIVTRINDISHTQSVSSKQRVT
GLDFIPGLHPLLSLSKMDQTLAIYQQILTSLPSRNVVQISNDLENLRDLLHLLAASKSC
PLPQVRALESLESLGVVLEASLYSTEVVALSRLQGSLQDMLRQLDLSPGC (SEQ ID
 NO:3). In another embodiment, the bovine *ob* amino acid sequence can be selected from any one of the sequences corresponding to Entrez Protein Accession Nos. AAE82807, AAK95823, AAN04050, AAN28921, BAA19750, BAB63371, CAA72197, CAB38018, CAB64255, CAD54745, CAE45337, CAE45338, CAE45339, NP_000585, NP_000591, NP_000749, NP_776351, NP_776353, NP_776565, NP_776641, NP_851339, P50595 and Q9BEG9, the disclosures of which are incorporated by reference in their entireties.

In an embodiment wherein the *ob* sequence is an ovine *ob* sequence, the ovine *ob* nucleotide sequence can be selected from any one of the sequences corresponding to GenBank Accession Nos. AF310264, AF118636 and U63719 and the ovine *ob* amino acid sequence can be selected from any one of the sequences corresponding to Entrez Protein Accession Nos; AAB51695, AAD17249, P79211, Q28602 and Q28603, the disclosures of which are incorporated by reference in their entireties.

In an embodiment wherein the *ob* sequence is an avian *ob* sequence, the avian *ob* nucleotide sequence can be selected from any one of the sequences corresponding to GenBank Accession Nos. NM_012614, NT_032977 and NW_047717 and the avian *ob* amino acid can be selected from the sequence corresponding to Entrez Protein Accession No. NP_036746, the disclosures of which are incorporated by reference in their entireties.

In an embodiment wherein the *ob* sequence is an swine *ob* sequence, the swine *ob* nucleotide sequence can be selected from any one of the sequences corresponding to GenBank Accession Nos. AF026976, AF036908, AF052691, AF092422, AF102856, AF167719, AF184172, AF184173, AF477386, AF477387, AH009271, AH011524, AJ223162, AJ223163, AY008846, AY079082, AY079083, U40812, U59894, U63540, U66254, U67739 and U72070 and the swine *ob* amino acid sequence can be selected from any one of the sequences corresponding to Entrez Protein Accession Nos. AAB06579, AAB40624, AAB61244, AAB62399, AAD23567, AAK95823, AAN04050, AAN28921, BAA19750, BAB63371, CAA72197, CAB38018, CAB64255, CAD54745, CAE45337, CAE45338, CAE45339, NP_776351, NP_776353, NP_776565, NP_776641, NP_851339, P50595 and Q9BEG9, the disclosures of which are incorporated by reference in their entireties.

Also disclosed herein are oligonucleotides that can be used as primers to amplify specific nucleic acid sequences of the *ob* gene. The present invention also provides oligonucleotides that can be used as probes in the detection of amplified specific nucleic acid sequences of the *ob* gene. In certain embodiments, these probes and primers consist of oligonucleotide fragments. Such fragments should be of sufficient length to provide specific hybridization to an RNA or DNA tissue sample. The sequences typically will be about 8 to about 44 nucleotides, but may be longer. Longer sequences, *e.g*., from about 14 to about 50, are advantageous for certain embodiments.

Nucleic acid molecules having contiguous stretches of about 8, 9, 10, 11, 12, 13, 14, 15, 16, 17, 18, 19, 20, 21, 22, 23, or 24 nucleotides from a sequence selected from SEQ ID NO:4, SEQ ID NO:5, SEQ ID NO:6, and SEQ ID NO:7 are contemplated. Molecules that are complementary to the above mentioned sequences and that bind to these sequences under high stringency conditions also are contemplated. These probes will be useful in a variety of hybridization embodiments, such as Southern and Northern blotting. In some cases, it is contemplated that probes may be used that hybridize to multiple target sequences without compromising their ability to effectively detect the *ob* gene.

Various probes and primers can be designed around the disclosed nucleotide sequences. Primers may be of any length but, typically, are about 10 to about 24 bases in length. A probe or primer can be any stretch of at least 8, advantageously at least 10, more advantageously at least 12, 13, 14, or 15, such as at least 20, e.g., at least 23 or 25, for instance at least 27 or 30 nucleotides. As to PCR or hybridization primers or probes and optimal lengths therefor, reference is also made to Kajimura et al., GATA 7(4):71-79 (1990), the disclosure of which is incorporated by reference in its entirety. In certain embodiments, it is contemplated that multiple probes may be used for hybridization to a single sample. Designing and testing the probes and primers around the *ob* nucleotide sequences described above and from any one of the sequences corresponding to the accession numbers listed can be accomplished by one of ordinary skill in the art.

The use of a hybridization probe of between 10 and 30 nucleotides in length allows the formation of a duplex molecule that is both stable and selective. Molecules having complementary sequences over stretches greater than 12 bases in length are generally advantageous, in order to increase stability and selectivity of the hybrid, and thereby improve the quality and degree of particular hybrid molecules obtained. One will generally prefer to design nucleic acid molecules having stretches of 16 to 24 nucleotides, or even longer where desired. Such fragments may be readily prepared by, for example, directly synthesizing the fragment by chemical means or by introducing selected sequences into recombinant vectors for recombinant production.

Methods for making a vector or recombinants or plasmid for amplification of the fragment either *in vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450 and 6; 312,683; U.S. patent application Serial No. 920,197, filed October 16,1986; WO 90/01543; W091/11525; WO 94/16716; WO 96/39491; WO 98/33510; EP 265785; EP 0 370 573; Andreansky et al., Proc. Natl. Acad. Sci. USA 1996;93:11313-11318; Ballay et al., EMBO J. 1993;4:3861-65; Felgner et al., J. Biol. Chem. 1994;269:2550-2561; Frolov et al., Proc. Natl. Acad. Sci. USA 1996;93:11371-11377; Graham, Tibtech 1990;8:85-87; Grunhaus et al., Sem. Virol. 1992;3:237-52; Ju et al., Diabetologia 1998;41:736-739; Kitson et al., J. Virol. 1991;65:3068-3075; McClements et al., Proc. Natl. Acad. Sci. USA 1996;93:11414-11420; Moss, Proc. Natl. Acad. Sci. USA 1996;93:11341-11348; Paoletti, Proc. Natl. Acad. Sci. USA 1996;93:11349-11353; Pennock et al., Mol. Cell. Biol. 1984;4:399-406; Richardson (Ed), Methods in Molecular Biology 1995;39, "Baculovirus Expression Protocols," Humana Press Inc.; Smith et al. (1983) Mol. Cell. Biol. 1983;3:2156-2165; Robertson et al., Proc. Natl. Acad. Sci. USA 1996;93 :11334-11340; Robinson et al., Sem. Immunol. 1997;9:271; and Roizman, Proc. Natl. Acad. Sci. USA 1996;93:11307-11312.

Accordingly, the nucleotide sequences of the invention may be used for their ability to selectively form duplex molecules with complementary stretches of genes or RNAs or to provide primers for amplification of DNA or RNA from tissues. Depending on the application envisioned, one will desire to employ varying conditions of hybridization to achieve varying degrees of selectivity of probe towards target sequence.

For applications requiring high selectivity, one will typically desire to employ relatively stringent conditions to form the hybrids, *e.g*., one will select relatively low salt and/or high temperature conditions, such as provided by about 0.02 M to about 0.10 M NaCl at temperatures of about 50° C to about 70° C. Such high stringency conditions tolerate little, if any, mismatch between the probe and the template or target strand, and would be particularly suitable for isolating specific genes or detecting specific mRNA transcripts. It is generally appreciated that conditions can be rendered more stringent by the addition of increasing amounts of formamide.

It will be understood that this invention is not limited to the particular probes disclosed herein and particularly is intended to encompass at least nucleic acid sequences that are hybridizable to the disclosed sequences or are functional sequence analogs of these sequences.

One embodiment of the present invention is directed to a nucleic acid sequences (oligonucleotides) useful as primers and/or probes in the detection of an *ob* gene polymorphism in specimens. Also, the present invention is directed to a method of detecting the presence of *ob* gene polymorphism in a specimen wherein the oligonucleotides of the present invention may be used to amplify target nucleic acid sequences of an *ob* gene polymorphism that may be contained within a livestock specimen, and/or to detect the presence or absence of amplified target nucleic acid sequences of the *ob* gene polymorphism. Respective oligonucleotides may be used to amplify and/or detect *ob* gene and *ob* gene nucleic acid sequences. By using the oligonucleotides of the present invention arid according to the methods of the present invention, as few as one to ten copies of the *ob* gene polymorphism may be detected in the presence of milligram quantities of extraneous DNA.

One embodiment of the present invention is directed to *ob* gene-specific oligonucleotides that can be used to amplify sequences of *ob* gene DNA, and to subsequently determine if amplification has occurred, from DNA extracted from a livestock specimen. A pair of *ob* gene-specific DNA oligonucleotide primers are used to hybridize to *ob* gene genomic DNA that may be present in DNA extracted from a livestock specimen, and to amplify the specific segment of genomic DNA between the two flanking primers using enzymatic synthesis and temperature cycling. Each pair of primers are designed to hybridize only to the *ob* gene DNA to which they have been synthesized to complement; one to each strand of the double-stranded DNA. Thus, the reaction is specific even in the presence of microgram quantities of heterologous DNA. For the purposes of this description, the primer derived from the sequence of the positive strand of DNA will be referred to as the "positive (+) primer", and the primer derived from the sequence of the negative strand will be referred to as the "negative (-) primer". Sequences that may be used include the primers AGGGATGCCTGGACACAAGA (sense, SEQ ID NO:4) and ATTGCCACCACCAGCAGCACCA (antisense, SEQ ID NO:5) and the probes CATCTGCTATGCGAATGCTTTG (SEQ ID NO:6) and GCTAATTATATTGTAAGACA (SEQ ID NO:7).

In one embodiment, the present invention relates to a composition for the detection of *ob* gene polymorphisms, consisting essentially of at least one purified and isolated oligonucleotide consisting of a nucleic acid sequence which complements and specifically hybridizes to an *ob* gene nucleic acid molecule, wherein said sequence is selected from the group consisting of SEQ ID NO:4, SEQ ID NO:5, SEQ ID NO:6, and SEQ ID NO:7, and a nucleotide sequence which differs from SEQ ID NO:4, SEQ ID NO:5, SEQ ID NO:6, or SEQ ID NO:7, by a one base change or substitution therein.

In another embodiment, the present invention relates to a method of detecting the presence of an *ob* gene polymorphism in a sample comprising (a) contacting the sample with the above-described nucleic acid probe, under conditions such that hybridization occurs, and (b) detecting the presence of the probe bound to the DNA segment. In an advantageous embodiment, the *ob* gene polymorphism is a C to T transition that results in an Arg25Cys in the leptin protein.

In another embodiment, the present invention relates to a method of detecting the presence of an *ob* gene polymorphism in a sample comprising (a) contacting the sample with the above-described nucleic acid probe, under conditions such that hybridization occurs, and (b) detecting the presence of the probe bound to the DNA segment. In an advantageous embodiment, the *ob* gene polymorphism is a C to T transition that results in an Arg25Cys in the leptin protein.

The actual hybridization reaction represents one of the most important and central steps in the whole process. The hybridization step involves placing the prepared DNA sample in contact with a specific sensor probe at set optimal conditions for hybridization to occur between the target DNA sequence and probe.

In their most basic form, hybridization assays function by discriminating oligonucleotide probe sensors against matched and mismatched targets. Currently, a variety of methods are available for detection and analysis of the hybridization events. Depending on the sensor group (fluorophore, enzyme, radioisotope, etc.) used to label the DNA probe, detection and analysis are carried out fluorimetrically, colorimetrically, or by autoradiography. By observing and measuring emitted radiation, such as fluorescent radiation or particle emission, information may be obtained about the hybridization events.

The secondary and tertiary structure of a single stranded target nucleic acid may be affected by binding "helper" oligonucleotides in addition to "probe" oligonucleotides causing a higher Tm to be exhibited between the probe and target nucleic acid.

Methods are provided for the analysis and determination of SNPs in a genetic target. In this embodiment, both wild type and mutant alleles are distinguished, if present in a sample, at a single capture site by detecting the presence of hybridized allele-specific probes labeled with fluorophores sensitive to excitation at various wave lengths.

In one embodiment, a target nucleic acid is first amplified, such as by PCR or SDA. The amplified dsDNA product is then denatured and hybridized with a probe. The hybridization complex formed is then subjected to destabilizing conditions to differentiate and determination of the *ob* SNP.

In another embodiment, the present invention relates to a method of detecting the presence of an *ob* gene polymorphism in a sample comprising a) contacting the sample with the above-described nucleic acid probe, under conditions such that hybridization occurs, b) enzymatically amplifying a specific region of the *ob* gene nucleic acid molecules, and c) detecting the presence of the probe bound to the DNA segment. In an advantageous embodiment, the *ob* gene polymorphism is a C to T transition that results in an Arg25Cys in the leptin protein.

In another embodiment, the present invention relates to a method of detecting the presence of an *ob* gene polymorphism in a sample comprising a) contacting the sample with the oligonucleotide primer pair of SEQ ID NO:4 and SEQ ID NO:5 that under suitable conditions permitting hybridization of the oligonucleotides to the nucleic acid molecules of the *ob* gene, b) enzymatically amplifying a specific region of the *ob* gene nucleic acid molecules using the oligonucleotide pair of SEQ ID NO:4 and SEQ ID NO:5 to form nucleic acid amplification products, c) contacting the amplified target sequences from step be, is present, with hybridization probes comprising the oligonucleotide pair of SEQ ID NO:6 and SEQ ID NO:7, labeled with a detectable moiety under suitable conditions permitting hybridization of the labeled oligonucleotide probe to amplified target sequences, and d) detecting the presence of amplified target sequences by detecting the detectable moiety of the labeled oligonucleotide probe hybridized to amplified target sequences. In an advantageous embodiment, prior to performing the above method, the sample is treated to release nucleic acid molecules from cells in the sample. In another advantageous embodiment, the presence of the amplified target sequences hybridized labeled oligonucleotide probe correlates to the presence of an *ob* gene polymorphism in the sample. In an advantageous embodiment, the *ob* gene polymorphism is a C to T transition that results in an Arg25Cys in the leptin protein.

Any one of the methods commercially available may accomplish amplification of DNA. For example, the polymerase chain reaction may be used to amplify the DNA. Once the primers have hybridized to opposite strands of the target DNA, the temperature is raised to permit replication of the specific segment of DNA across the region between the two primers by a thermostable DNA polymerase. Then the reaction is thermocycled so that at each cycle the amount of DNA representing the sequences between the two primers is doubled, and specific amplification of the *ob* gene DNA sequences, if present, results.

Further identification of the amplified DNA fragment, as being derived from *ob* gene DNA, may be accomplished by liquid hybridization. This method utilizes one or more oligonucleotides labeled with detectable moiety as probes to specifically hybridize to the amplified segment of *ob* gene DNA. Detection of the presence of sequence-specific amplified *ob* gene DNA may be accomplished by simultaneous detection of the complex comprising the labeled oligonucleotide hybridized to the sequence-specific amplified *ob* gene DNA ("amplified target sequences") with respect to the DNA amplification. Detection of the presence of sequence-specific amplified *ob* gene DNA may also be accomplished using a gel retardation assay with subsequent detection of the complex comprising the labeled oligonucleotide hybridized to the sequence-specific amplified *ob* gene DNA.

In such a enzymatic amplification reaction hybridization system of *ob* gene allele detection, a specimen of blood, CSF, amniotic fluid, urine, body secretions, or other body fluid is subjected to a DNA extraction procedure. High molecular weight DNA may be purified from blood cells, tissue cells, or virus particles (collectively referred to herein as "cells") contained in the livestock specimen using proteinase (proteinase K) extraction and ethanol precipitation. DNA may be extracted from a livestock specimen using other methods known in the art. Then, for example, the DNA extracted from the livestock specimen is enzymatically amplified in the polymerase chain reaction using *ob* gene-specific oligonucleotides (SEQ ID NO:4 and SEQ ID NO:5) as primer pairs. Following amplification, *ob* gene-specific oligonucleotides (SEQ ID NO:6 and SEQ ID NO:7) labeled with an appropriate detectable label are hybridized to the amplified target sequences, if present.

The contents of the hybridization reaction are then analyzed for detection of the sequence-specific amplified *ob* gene DNA, if present in the DNA extracted from the livestock specimen. Thus, the oligonucleotides of the present invention have commercial applications in diagnostic kits for the detection of *ob* gene DNA in livestock specimens.

The test samples suitable for nucleic acid probing methods of the present invention include, for example, cells or nucleic acid extracts of cells, or biological fluids. The sample used in the above-described methods will vary based on the assay format, the detection method and the nature of the tissues, cells or extracts to be assayed. Methods for preparing nucleic acid extracts of cells are well known in the art and can be readily adapted in order to obtain a sample that is compatible with the method utilized.

In a related embodiment of the present invention, the *ob* gene-specific oligonucleotides may be used to amplify and detect *ob* gene polymorphisms from DNA extracted from a livestock specimen. In this embodiment, the oligonucleotides used as primers may be labeled directly with detectable moiety, or synthesized to incorporate the label molecule. Depending on the label molecule used, the amplification products can then be detected, for example, after binding onto an affinity matrix, using isotopic or calorimetric detection. In an advantageous embodiment, the *ob* gene polymorphism is a C to T transition that results in an Arg25Cys in the leptin protein.

In an advantageous embodiment of this invention, cyclic polymerase-mediated reactions are performed. In certain embodiments of this invention, these processes are accomplished by changing the temperature of the solution containing the templates, primers, and polymerase. In such embodiments, the denaturation step is typically accomplished by shifting the temperature of the solution to a temperature sufficiently high to denature the template. In some embodiments, the hybridization step and the extension step are performed at different temperatures. In other embodiments, however, the hybridization and extension steps are performed concurrently, at a single temperature.

In some embodiments, the cyclic polymerase-mediated reaction is performed at a single temperature, and the different processes are accomplished by changing non-thermal properties of the reaction. For example, the denaturation step can be accomplished by incubating the template molecules with a basic solution or other denaturing solution.

In advantageous embodiments, the percentage of template molecules that are duplicated in the cycle steps is *e.g*. 90%, 70%, 50%, 30%, or less. Such cycles may be as short as 10, 8, 6, 5, 4.5, 4, 2, 1, 0.5 minutes or less. In certain embodiments, the reaction comprises 2, 5, 10, 15, 20, 30, 40, 50, or more cycles.

Typically, the reactions described herein are repeated until a detectable amount of product is generated. Often, such detectable amounts of product are between about 10 ng and about 100 ng, although larger quantities, *e.g*. 200 ng, 500 ng, 1 mg or more can also, of course, be detected. In terms of concentration, the amount of detectable product can be from about 0.01 pmol, 0.1 pmol, 1 pmol, 10 pmol, or more.

Any of a variety of polymerases can be used in the present invention. For thermocyclic reactions, the polymerases are thermostable polymerases such as Taq, KlenTaq, Stoffel Fragment, Deep Vent, Tth, Pfu, Vent, and UlTma, each of which are readily available from commercial sources. Similarly, guidance for the use of each of these enzymes can be readily found in any of a number of protocols found in guides, product literature, and other sources.

For non-thermocyclic reactions, and in certain thermocyclic reactions, the polymerase will often be one of many polymerases commonly used in the field, and commercially available, such as DNA pol 1, Klenow fragment, T7 DNA polymerase, and T4 DNA polymerase. Guidance for the use of such polymerases can readily be found in product literature and in general molecular biology guides.

Those of skill in the art are aware of the variety of nucleotides available for use in the present reaction. Typically, the nucleotides will consist at least in part of deoxynucleotide triphosphates (dNTPs), which are readily commercially available. Parameters for optimal use of dNTPs are also known to those of skill, and are described in the literature. In addition, a large number of nucleotide derivatives are known to those of skill and can be used in the present reaction. Such derivatives include fluorescently labeled nucleotides, allowing the detection of the product including such labeled nucleotides, as described below. Also included in this group are nucleotides that allow the sequencing of nucleic acids including such nucleotides, such as dideoxynucleotides and boronated nuclease-resistant nucleotides, as described below. Other nucleotide analogs include nucleotides with bromo-, iodo-, or other modifying groups, which groups affect numerous properties of resulting nucleic acids including their antigenicity, their replicatability, their melting temperatures, their binding properties, *etc.* In addition, certain nucleotides include reactive side groups, such as sulfhydryl groups, amino groups, N-hydroxysuccinimidyl groups, that allow the further modification of nucleic acids comprising them.

An oligonucleotide sequence used as a detection probe may be labeled with a detectable moiety. Various labeling moieties are known in the art. Said moiety may, for example, either be a radioactive compound, a detectable enzyme (*e.g*. horse radish peroxidase (HRP)) or any other moiety capable of generating a detectable signal such as a calorimetric, fluorescent, chemiluminescent or electrochemiluminescent signal. Advantageous analysis systems wherein said labels are used are electrochemiluminescence (ECL) based analysis or enzyme linked gel assay (ELGA) based analysis.

In one class of embodiments of this invention, a detectable label is incorporated into a nucleic acid during at least one cycle of the reaction. Spectroscopic, photochemical, biochemical, immunochemical, electrical, optical or chemical means can detect such labels. Useful labels in the present invention include fluorescent dyes (*e.g*., fluorescein isothiocyanate, Texas red, rhodamine, and the like), radiolabels (*e.g*., ³H, ¹²⁵I, ³⁵S, ¹⁴C, ³²P, *etc.*), enzymes (*e.g*. horseradish peroxidase, alkaline phosphatase *etc.*) colorimetric labels such as colloidal gold or colored glass or plastic (*e.g*. polystyrene, polypropylene, latex, *etc.*) beads. The label is coupled directly or indirectly to a component of the assay according to methods well known in the art. As indicated above, a wide variety of labels are used, with the choice of label depending on sensitivity required, ease of conjugation with the compound, stability requirements, available instrumentation, and disposal provisions. Non-radioactive labels are often attached by indirect means. Polymerases can also incorporate fluorescent nucleotides during synthesis of nucleic acids.

Reagents allowing the sequencing of reaction products can be utilized herein. For example, chain-terminating nucleotides will often be incorporated into a reaction product during one or more cycles of a reaction. Commercial kits containing the reagents most typically used for these methods of DNA sequencing are available and widely used. PCR exonuclease digestion methods for DNA sequencing can also be used.

Typically, the amplification sequence is serially diluted and then quantitatively amplified via the DNA Tag polymerase using a suitable PCR amplification technique. In PCR, annealing of the primers to the amplification sequence is generally carried out at about 37-50°C.; extension of the primer sequence by Taq polymerase in the presence of nucleoside triphosphates is carried out at about 70-75°C.; and the denaturing step to release the extended primer is carried out at about 90-95°C. In the two temperature PCR technique, the annealing and extension steps may both be carried at about 60-65°C., thus reducing the length of each amplification cycle and resulting in a shorter assay time.

Polymerase chain reactions (PCR) are generally carried out in about 25-50 µl samples containing 0.01 to 1.0 ng of template amplification sequence, 10 to 100 pmol of each generic primer, 1.5 units of Tag DNA polymerase (Promega Corp.), 0.2 mM DATP, 0.2 mM dCTP, 0.2 mM dGTP, 0.2 mM dTTP, 15 mM MgCl₂, 10 mM Tris-HCl (pH 9.0), 50 mM KCl, 1 µg/ml gelatin, and 10 µl/ml Triton X-100 (Saiki, 1988). Reactions are incubated at 94°C. for 1 minute, about 37 to 55°C. for 2 minutes (depending on the identity of the primers), and about 72°C. for about 3 minutes and repeated for about 5-40, cycles. A two temperature PCR technique differs from the above only in carrying out the annealing/extension steps at a single temperature, *e.g*., about 60-65°C. for about 5 minutes, rather than at two temperatures.

Another embodiment of the present invention is directed to *ob* gene-specific oligonucleotides that can be used to amplify sequences of *ob* gene DNA, and to subsequently determine if amplification has occurred, from DNA extracted from a livestock specimen. A pair of *ob* gene-specific DNA oligonucleotide primers are used to hybridize to *ob* gene genomic DNA that may be present in DNA extracted from a livestock specimen, and to amplify the specific segment of genomic DNA between the two flanking primers using enzymatic synthesis and temperature cycling. Each pair of primers are designed to hybridize only to the *ob* gene DNA to which they have been synthesized to complement; one to each strand of the double-stranded DNA. The region to which the primers have been synthesized to complement is conserved in *ob* gene. Thus, the reaction is specific even in the presence of microgram quantities of heterologous DNA.

A nucleic acid molecule of the present invention, *e.g*., a nucleic acid molecule having the nucleotide sequence of SEQ ID NO:4, SEQ ID NO:5, EQ ID NO:6 or SEQ ID NO:7, or a complement thereof, can be isolated using standard molecular biology techniques and the sequence information provided herein. Using all or a portion of the nucleic acid sequence of SEQ ID NO:4, SEQ ID NO:5, SEQ ID NO:6 or SEQ ID NO:7, as a hybridization probe, nucleic acid sequences can be isolated using standard hybridization and cloning techniques. Furthermore, oligonucleotides can be prepared by standard synthetic techniques, *e.g*., using an automated DNA synthesizer.

In another embodiment, an isolated nucleic acid molecule of the invention comprises a nucleic acid molecule that is a complement of the nucleotide sequence shown in SEQ ID NO:4, SEQ ID NO:5, SEQ ID NO:6 or SEQ ID NO:7 or a portion of this nucleotide sequence. A nucleic acid molecule that is complementary to the nucleotide sequence shown in SEQ ID NO:4, SEQ ID NO:5, SEQ ID NO:6 or SEQ ID NO:7, is one that is sufficiently complementary to the nucleotide sequence shown in SEQ ID NO:4, SEQ ID NO:5, SEQ ID NO:6 or SEQ ID NO:7 that it can bind with few or no mismatches to the nucleotide sequence shown in SEQ ID NO:4, SEQ ID NO:5, SEQ ID NO:6, or SEQ ID NO:7, thereby forming a stable duplex.

A nucleic acid molecule of the invention may include only a fragment of the nucleic acid sequence of SEQ ID NO:4, SEQ ID NO:5, SEQ ID NO:6 or SEQ ID NO:7. Fragments provided herein are defined as sequences of at least 6 (contiguous) nucleic acids, a length sufficient to allow for specific hybridization of nucleic acids, and are at most some portion less than a full-length sequence. Fragments may be derived from any contiguous portion of a nucleic acid sequence of choice. Derivatives are nucleic acid sequences formed from the native compounds either directly or by modification or partial substitution. Analogs are nucleic acid sequences that have a structure similar to, but not identical to, the native compound but differ from it in respect to certain components or side chains. Analogs may be synthetic or from a different evolutionary origin and may have a similar or opposite metabolic activity compared to wild type.

Derivatives and analogs may be full length or other than full length, if the derivative or analog contains a modified nucleic acid or amino acid, as described below. Derivatives or analogs of the nucleic acids of the invention include, but are not limited to, molecules comprising regions that are substantially homologous to the nucleic acids of the invention, in various embodiments, by at least about 70%, 80%, 85%, 90%, 95%, 96%, 97%, 98%, or even 99% identity (with an advantageous identity of 80-99%) over a nucleic acid sequence of identical size or when compared to an aligned sequence in which the alignment is done by a computer homology program known in the art. Derivatives or analogs of the nucleic acids of the invention also include, but are not limited to, molecules comprising regions that are substantially homologous to the nucleic acids of the invention, in various embodiments, by at least about 70%, 80%, 85%, 90%, 95%, 96%, 97%, 98%, or even 99% identity (with an advantageous identity of 80-99%) under stringent, moderately stringent, or low stringent conditions.

For the purposes of the present invention, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990;87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson & Lipman, Proc. Natl. Acad. Sci. USA 1988;85: 2444-2448.

Advantageous for use according to the present invention is the WU-BLAST (Washington University BLAST) version 2.0 software. WU-BLAST version 2.0 executable programs for several UNIX platforms can be downloaded from ftp ://blast. wustl. edu/blast/executables. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990;215: 403-410; Gish & States, 1993;Nature Genetics 3: 266-272; Karlin & Altschul, 1993;Proc. Natl. Acad. Sci. USA 90: 5873-5877; all of which are incorporated by reference herein).

In all search programs in the suite the gapped alignment routines are integral to the database search itself. Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology " or "identity", for instance, with respect to a nucleotide or amino acid sequence, can indicate a quantitative measure of homology between two sequences. The percent sequence homology can be calculated as (N*_{ref} -* N*_{dif})**100/N*_{ref},* wherein N*_{dif}* is the total number of non-identical residues in the two sequences when aligned and wherein *N_{ref}* is the number of residues in one of the sequences. Hence, the DNA sequence AGTCAGTC will have a sequence identity of 75% with the sequence AATCAATC (N*_{ref}* = 8; N*_{dif}*=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur & Lipman, Proc Natl Acad Sci USA 1983;80:726, incorporated herein by reference), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

And, without undue experimentation, the skilled artisan can consult with many other programs or references for determining percent homology.

The nucleotide sequence of probes and primers typically comprises a substantially purified oligonucleotide. The oligonucleotide typically comprises a region of nucleotide sequence that hybridizes under stringent conditions to at least about 6, 9, 12, 16, 24, or more consecutive sense strand nucleotide sequence of SEQ ID NO:4, SEQ ID NO:5, SEQ ID NO:6 or SEQ ID NO:7, or an anti-sense strand nucleotide sequence of SEQ ID NO:4, SEQ ID NO:5, SEQ ID NO:6 or SEQ ID NO:7, or of a naturally occurring mutant of SEQ ID NO:4, SEQ ID NO:5, SEQ ID NO:6 or SEQ ID NO:7.

In various embodiments, the probe further comprises a label group attached thereto. Such probes can be used as a part of a diagnostic test kit for assessing the presence of homozygous mutant alleles of the *ob* gene (*ob⁻*/*ob⁻* or TT animals), heterozygous mutant alleles of the *ob* gene (o*b⁻*/*ob⁺* or CT animals) and wild-type alleles of the *ob* gene *(ob⁺*/*ob⁺* or CC animals).

The invention further encompasses nucleic acid molecules that differ from the nucleotide sequences shown in SEQ ID NO:4, SEQ ID NO:5, SEQ ID NO:6 or SEQ ID NO:7, due to the degeneracy of the genetic code.

In addition to the nucleotide sequence shown in SEQ ID NO:4, SEQ ID NO:5, SEQ ID NO:6 or SEQ ID NO:7, it will be appreciated by those skilled in the art that DNA sequence polymorphisms in the *ob* gene DNA may exist within a population. Such natural allelic variations can typically result in about 1-5% variance in the nucleotide sequence of the gene. Any and all such nucleotide variations are intended to be within the scope of the invention.

Moreover, nucleic acid molecules that differ from the sequence of SEQ ID NO:4, SEQ ID NO:5, SEQ ID NO:6 or SEQ ID NO:7, are intended to be within the scope of the invention. Nucleic acid molecules corresponding to natural allelic variants and homologues of the DNAs of the invention can be isolated based on their homology to the nucleic acids disclosed herein using standard hybridization techniques under stringent hybridization conditions. Advantageously, such variations will differ from the sequence of SEQ ID NO:4., SEQ ID NO:5, SEQ ID NO:6 or SEQ ID NO:7, by only one nucleotide.

Accordingly, in another embodiment, an isolated nucleic acid molecule of the invention is at least 6 nucleotides in length and hybridizes under stringent conditions to the nucleic acid molecule comprising the nucleotide sequence of SEQ ID NO:4, SEQ ID NO:5, SEQ ID NO:6 or SEQ ID NO:7.

Homologs (*i.e*., nucleic acids derived from other species) or other related sequences (*e.g*., paralogs) can be obtained under conditions of standard or stringent hybridization conditions with all or a portion of the particular sequence as a probe using methods well known in the art for nucleic acid hybridization and cloning.

In another embodiment, a nucleic acid sequence that is hybridizable to the nucleic acid molecule comprising the nucleotide sequence of SEQ ID NO:4, SEQ ID NO:5, SEQ ID NO:6 or SEQ ID NO:7, or fragments, analogs or derivatives thereof, under conditions of standard or stringent hybridization conditions is provided.

In addition to naturally-occurring allelic variants of the nucleotide sequence, the skilled artisan will further appreciate that changes can be introduced by mutation into the nucleotide sequence of SEQ ID NO:4, SEQ ID NO:5, SEQ ID NO:6 or SEQ ID NO:7.

In one embodiment, the isolated nucleic acid molecule comprises a nucleotide sequence at least about 75% homologous to the nucleotide sequence of SEQ ID NO:4, SEQ ID NO:5, SEQ ID NO:6 or SEQ ID NO:7. Advantageously, the nucleic acid is at least about 80% homologous to the nucleotide sequence of SEQ ID NO:4, SEQ ID NO:5, SEQ ID NO:6 or SEQ ID NO:7, more advantageously at least about 90%, 95%, 96%, 97%, 98%, and most advantageously at least about 99% homologous to the nucleotide sequence of SEQ ID NO:4, SEQ ID NO:5, SEQ ID NO:6 or SEQ ID NO:7.

As already indicated above, and will be presented in the experimental part of the description, both the sensitivity and reliability of polymorphism detection is greatly improved using the oligonucleotides according to the present invention when compared to known methods used in this art.

It is understood that oligonucleotides consisting of the sequences of the present invention may contain minor deletions, additions and/or substitutions of nucleic acid bases, to the extent that such alterations do not negatively affect the yield or product obtained to a significant degree.

Test kits for assessing the presence of homozygous mutant alleles of the *ob* gene (*ob⁻* /*ob*⁻ or TT animals), heterozygous mutant alleles of the *ob* gene *(ob⁻*/*ob⁺* or CT animals) and wild-type alleles of the *ob* gene *(ob⁺*/*ob⁺* or CC animals) are also part of the present invention. A test kit according to the invention may comprise a pair of oligonucleotides according to the invention and a probe comprising an oligonucleotide according to the invention. Such a test kit may additionally comprise suitable amplification reagents such as DNA and or RNA polymerases and mononucleotides. Test kits that can be used with the method according to the invention may comprise the oligonucleotides according to the invention for the amplification and subsequent assessment of for the presence of homozygous mutant alleles of the *ob* gene *(ob⁻*/*ob⁻* or TT animals), heterozygous mutant alleles of the *ob* gene *(ob⁻*/*ob⁺* or CT animals) and wild-type alleles of the *ob* gene *(ob⁺*/*ob⁺* or CC animals). An advantageous embodiment for the test kit comprises the oligonucleotides: SEQ ID NO:4 and SEQ ID NO:5 as primer pairs for the amplification, and oligonucleotides SEQ ID NO:6 or SEQ ID NO:7, for use with SEQ ID NO:4 and SEQ ID NO:5, provided with a detectable label, as probes.

A diagnostic test kit for detection of *ob* gene according to the compositions and methods of the present invention may include, in separate packaging, a lysing buffer for lysing cells contained in the specimen; at least one oligonucleotide primer pair (SEQ ID NO:4 and SEQ ID NO:5); enzyme amplification reaction components such as dNTPs, reaction buffer, and/or amplifying enzyme; and at least one oligonucleotide probe labeled with a detectable moiety (SEQ ID NO:6 or SEQ ID NO:7), or various combinations thereof.

The present invention further provides nucleic acid detection kits, including arrays or microarrays of nucleic acid molecules that are based on one or more of the sequences provided in SEQ ID NO:4, SEQ ID NO:5, SEQ ID NO:6, and SEQ ID NO:7. As used herein "Arrays" or "Microarrays" refers to an array of distinct polynucleotides or oligonucleotides synthesized on a solid or flexible support, such as paper, nylon or other type of membrane, filter, chip, glass slide, or any other suitable solid support. In one embodiment, the microarray is prepared and used according to the methods and devices described in U.S. Pat. Nos. 5,446,603; 5,545,531; 5,807,522; 5,837,832; 5,874,219; 6,114,122; 6,238,910; 6,365,418; 6,410,229; 6,420,114; 6,432,696; 6,475,808 and 6,489,159 and PCT Publication No. WO 01/45843 A2, the disclosures of which are incorporated by reference in their entireties.

Although the above methods are described in terms of the use of a single probe and a single set of primers, the methods are not so limited. One or more additional probes and/or primers can be used, if desired. Additional enzymes, constructed probes and primers can be determined through routine experimentation.

The reagents suitable for applying the methods of the invention may be packaged into convenient kits. The kits provide the necessary materials, packaged into suitable containers. Advantageously, the containers are also supports useful in performing the assay. At a minimum, the kit contains a reagent that identifies a polymorphism in the livestock *ob* gene that is associated with an increased weight gain. Advantageously, the reagent is a probe and/or PCR set (a set of primers, DNA polymerase and 4 nucleoside triphosphates) that hybridize with the livestock *ob* gene or a fragment thereof.

Advantageously, both the probe (or PCR set) and a restriction enzyme that cleaves the livestock *ob* gene in at least one place are included in the kit. In a particularly advantageous embodiment of the invention, the probe comprises the human *ob* gene, the livestock *ob* gene, or a gene fragment that has been labeled with a detectable entity: Advantageously, the kit further comprises additional means, such as reagents, for detecting or measuring the detectable entity or providing a control. Other reagents used for hybridization, prehybridization, DNA extraction, etc. may also be included, if desired.

The methods and materials of the invention may also be used more generally to evaluate livestock DNA, genetically type individual livestock, and detect genetic differences in livestock. In particular, a sample of livestock genomic DNA may be evaluated by reference to one or more controls to determine if a polymorphism in the *ob* gene is present. Any method for determining genotype can be used for determining the ob genotype in the present invention. Such methods include, but are not limited to, amplimer sequencing, DNA sequencing, fluorescence spectroscopy, FRET-based hybridization analysis, high throughput screening, mass spectroscopy, microsatellite analysis, nucleic acid hybridization, polymerase chain reaction (PCR), RFLP analysis and size chromatography (e.g., capillary or gel chromatography), all of which are well known to one of skill in the art. In particular, methods for determining nucleotide polymorphisms, particularly single nucleotide polymorphisms, are described in U.S. Patent Nos. 6,514,700; 6,503,710; 6,468,742; 6,448,407; 6,410,231; 6,383,756; 6,358,679; 6,322,980; 6,316,230; and 6,287,766 and reviewed by Chen and Sullivan, Pharmacogenomics J 2003;3(2):77-96, the disclosures of which are incorporated by reference in their entireties.

Advantageously, FRET analysis is performed with respect to the livestock *ob* gene, and the results are compared with a control. The control is the result of a FRET analysis of the livestock *ob* gene of a different livestock where the polymorphism of the livestock *ob* gene is known. Similarly, the estrogen receptor genotype of a livestock may be determined by obtaining a sample of its genomic DNA, conducting FRET analysis of the *ob* gene in the DNA, and comparing the results with a control. Again, the control is the result of FRET analysis of the *ob* gene of a different livestock. The results genetically type the livestock by specifying the polymorphism in its *ob* genes. Finally, genetic differences among livestock can be detected by obtaining samples of the genomic DNA from at least two livestock, identifying the presence or absence of a polymorphism in the *ob* gene, and comparing the results.

These assays are useful for identifying the genetic markers relating to weight gain, as discussed above, for identifying other polymorphisms in the *ob* gene that may be correlated with other characteristics, and for the general scientific analysis of livestock genotypes and phenotypes.

The genetic markers, methods, and kits of the invention are also useful in a breeding program to improve feed conversion efficiency in a breed, line, or population of livestock. Continuous selection and breeding of livestock that are at least heterozygous and advantageously homozygous for a polymorphism associated with increased feed conversion efficiency would lead to a breed, line, or population having higher numbers of offspring in each litter of the females of this breed or line. Thus, the markers can be used as selection tools.

It is to be understood that the application of the teachings of the present invention to a specific problem or environment will be within the capabilities of one having ordinary skill in the art in light of the teachings contained herein. The examples of the products and processes of the present invention appear in the following examples.

Further, the invention provides a method of using oligonucleotide primers (SEQ ID No.2 & SEQ ID No 3) based on this DNA sequence in a polymerase chain reaction (PCR) assay to distinguish livestock animals homozygous for mutant alleles of the *ob* gene *(ob⁻*/*ob⁻* or TT animals), which alleles encode an altered leptin, from livestock animals heterozygous for mutant alleles of the *ob* gene *(ob⁻*/*ob⁺* or CT animals) and livestock animals homozygous for wild-type alleles of the *ob* gene *(ob⁺*/*ob⁺* or CC animals).

In another embodiment, the invention provides a method of using primers having SEQ ID No.2 & SEQ ID No 3 based on this DNA sequence in a polymerase chain reaction (PCR) assay to distinguish livestock animals homozygous for mutant alleles of the *ob* gene *(ob⁻*/*ob⁻* or TT animals), which alleles encode an altered leptin, from livestock animals heterozygous for mutant alleles of the *ob* gene (*ob⁻*/*ob⁺* or CT animals) and livestock animals homozygous for wild-type alleles of the *ob* gene (*ob⁺*/*ob⁺* or CC animals), wherein detection of the PCR amplified fragment is by detection of a radioactively labeled nucleotide that is incorporated into the PCR amplified product.

In yet another embodiment, a non-radioactively labeled nucleotide is incorporated into the PCR amplified product and detection is by colorimetry, chemiluminescence, or measurement of fluorescence.

In another embodiment, the method of detection is based on the use of flourescently labeled nucleotides in Fluorescence Resonance Energy Transfer (FRET) based detection systems including Taqman, Molecular Beacon, etc., which are familiar to those conversant with prior art.

The oligonucleotides in the present invention can be produced by a conventional production process for general oligonucleotides, It can be produced, for example, by a chemical synthesis process or by a microbial process which makes use of a plasmid vector, a phage vector or the like (Tetrahedron Letters, 22, 1859-1862, 1981; Nucleic Acids Research, 14, 6227-6245, 1986). Further, it is suitable to use a nucleic acid synthesizer currently available on the market.

To label an oligonucleotide with the fluorescent dye, one of conventionally-known labeling methods can be used (Nature Biotechnology, 14, 303-308, 1996; Applied and Environmental Microbiology, 63, 1143-1147, 1997; Nucleic Acids Research, 24,4532-4535, 1996). Reversed phase chromatography or the like used to provide a nucleic acid probe for use in the present invention can purify the synthesized oligonucleotide, which is labeled with the fluorescent dye.

The nucleic acid probe according to the present invention can be prepared as described above. An advantageous probe form is one labeled with a fluorescent dye at the 3' or 5'end and containing G or C as the base at the labeled end. If the 5'end is labeled and the 3'end is not labeled, the OH group on the C atom at the 3'-position of the 3'end ribose or deoxyribose may be modified with a phosphate group or the like although no limitation is imposed in this respect.

Inclusion of the nucleic acid probe according to the present invention in a kit for analyzing or determining polymorphism and/or mutation of a target nucleic acid or gene, therefore, makes it possible to suitably use the kit as a kit for the analysis or determination of the polymorphism and/or mutation of the target nucleic acid or gene.

The probe according to the present invention may be immobilized on a surface of a solid (support layer), for example, on a surface of a slide glass. In this case, the probe may advantageously be immobilized on the end not labeled with the fluorescent dye. The probe of this form is now called a "DNA chip". These DNA chips can be used for monitoring gene expressions, determining base sequences, analyzing mutations or analyzing polymorphisms such as single nucleotide polymorphism (SNP). They can also be used as devices (chips) for determining nucleic acids.

In one aspect, during the hybridization of the nucleic acid target with the probes, stringent conditions may be utilized, advantageously along with other stringency affecting conditions, to aid in the hybridization. Detection by differential disruption is particularly advantageous to reduce or eliminate slippage hybridization among probes and target, and to promote more effective hybridization. In yet another aspect, stringency conditions may be varied during the hybridization complex stability determination so as to more accurately or quickly determine whether a SNP is present in the target sequence.

Thus, the present invention provides for a method of determining a polymorphism comprising (a) obtaining a nucleic acid sample; (b) hybridizing the nucleic acid sample with a probe, and (c) disrupting the hybridization to determine the level of disruption energies required wherein the sensor probe has a different disruption energy if there is a mutation in the homology between the original nucleic acid sequence and sensor probe for hybridization. In one example, there is a lower disruption energy, e.g., melting temperature, for an allele that harbors the mutation site, and a higher required energy for an allele with no mutation since the homology is 100% and therefore requires more energy to cause the hybridized target to dissociate.

Optionally, in step (b) a second ("anchor") probe used. Generally, the anchor probe is not specific to either t or c allele, but hybridizes regardless whether there is a c or t allele. The anchor probe does not affect the disruption energy required to disassociate the hybridization complex but, instead, contains a complementary label for using with the first ("sensor") probe.

Hybridization stability may be influenced by numerous factors, including thermoregulation, chemical regulation, as well as electronic stringency control, either alone or in combination with the other listed factors. Through the use of stringency conditions, in either or both of the target hybridization step or the sensor oligonucleotide stringency step, rapid completion of the process may be achieved. This is desirable to achieve properly indexed hybridization of the target DNA to attain the maximum number of molecules at a test site with an accurate hybridization complex. By way of example, with the use of stringency, the initial hybridization step may be completed in ten minutes or less, more advantageously five minutes or less, and most advantageously two minutes or less. Overall, the analytical process may be completed in less than half an hour.

As to detection of the hybridization complex, it is advantageous that the complex is labeled. Typically, in the step of determining hybridization of probe to target, there is a detection of the amount of labeled hybridization complex at the test site or a portion thereof. Any mode or modality of detection consistent with the purpose and functionality of the invention may be utilized, such as optical imaging, electronic imaging, use of charge-coupled devices or other methods of quantification. Labeling may be of the target, capture, or sensor. Various labeling may be by fluorescent labeling, colormetric labeling or chemiluminescent labeling. In yet another implementation, detection may be via energy transfer between molecules in the hybridization complex. In yet another aspect, the detection may be via fluorescence perturbation analysis. In another aspect the detection may be via conductivity differences between concordant and discordant sites.

In yet another aspect, detection can be carried out using mass spectrometry. In such method, no fluorescent label is necessary. Rather detection is obtained by extremely high levels of mass resolution achieved by direct measurement, for example, by time of flight or by electron spray ionization (ESI). Where mass spectrometry is contemplated, sensor probes having a nucleic acid sequence of 50 bases or less are advantageous.

In one mode, the hybridization complex is labeled and the step of determining amount of hybridization includes detecting the amounts of labeled hybridization complex at the test sites. The detection device and method may include, but is not limited to, optical imaging, electronic imaging, imaging with a CCD camera, integrated optical imaging, and mass spectrometry. Further, the detection, either labeled or unlabeled, is quantified, which may include statistical analysis. The labeled portion of the complex may be the target, the stabilizer, the sensor or the hybridization complex in toto. Labeling may be by fluorescent labeling selected from the group of, but not limited to, Cy3, Cy5, Bodipy Texas Red, Bodipy Far Red, Lucifer Yellow, Bodipy 630/650-X, Bodipy R6G-X and 5-CR 6G. Labeling may further be accomplished by colormetric labeling, bioluminescent labeling and/or chemiluminescent labeling. Labeling further may include energy transfer between molecules in the hybridization complex by perturbation analysis, quenching, electron transport between donor and acceptor molecules, the latter of which may be facilitated by double stranded match hybridization complexes. Optionally, if the hybridization complex is unlabeled, detection may be accomplished by measurement of conductance differential between double stranded and non-double stranded DNA. Further, direct detection may be achieved by porous silicon-based optical interferometry or by mass spectrometry.

The label may be amplified, and may include for example branched or dendritic DNA. If the target DNA is purified, it may be unamplified or amplified. Further, if the purified target is amplified and the amplification is an exponential method, it may be, for example, PCR amplified DNA or strand displacement amplification (SDA) amplified DNA. Linear methods of DNA amplification such as rolling circle or transcriptional runoff may also be used.

By way of example, following incubation of the sensor probes, discrimination is achieved by subjecting the complex to destabilizing conditions, e.g., heating the complex to about 4°C below melting temperature of the perfectly matched sensor/amplicon in a low salt buffer (e.g., 50 mM NaPO4). For FRET, imaging is then performed using two different lasers, one corresponding to the fluorophore on the wild-type sensor and one to the fluorophore on the mutant sensor. From these signal intensities, backgrounds are subtracted and specific activities are taken into account. A determination of wild type and mutant signals is achieved from which the allelic compositions of the amplicon products are determined.

In one embodiment, the method comprises (a) contacting the target nucleic acid of interest with at least one sensor oligonucleotide, wherein the sensor oligonucleotide comprises a sequence complementary to at least a portion of the target nucleic acid of interest, wherein the sensor oligonucleotide hybridizes to the target nucleic acid at a position suspected of containing the *ob* gene polymorphism and (b) subjecting the captured target nucleic acid and hybridized sensor probe oligonucleotide to destabilizing conditions, wherein the destabilizing conditions are sufficient to cause the sensor oligonucleotide to dissociate under differing conditions depending upon the presence of the cc, ct or tt polymorphisms in the *ob* gene.

In another embodiment, the method further comprises (c) detecting the hybridization of the sensor oligonucleotide to the target nucleic acid under the varying destabilizing conditions, whereby the presence of the specific sequence in the target nucleic acid is determined.

In yet another embodiment, the method further comprises a preparatory step of amplifying one or more target nucleic acid sequences from the nucleic acids of a sample, wherein the amplicons become the target nucleic acids.

In one embodiment, the amplification step produces single stranded amplicons, which are then utilized as the single stranded target nucleic acids. In another embodiment, the amplification step produces double stranded amplicons, further comprising a step of subjecting the amplicons to denaturing conditions to form single stranded target nucleic acids.

In an alternate embodiment, the amplification step is by an amplification method selected from the group consisting of polymerase chain reaction (PCR), strand displacement amplification (SDA), nucleic acid sequence-based amplification (NASBA), rolling circle amplification, T7 mediated amplification, T3 mediated amplification, and SP6 mediated amplification.

In one embodiment, the method comprising a step of subjecting the target nucleic acids of the sample to denaturing conditions to form single stranded target nucleic acids.

In another embodiment, the detection of the hybridization of the sensor oligonucleotide is by the detection of a labeling moiety on the sensor oligonucleotide selected from the group consisting of fluorescent moieties, bioluminescent moieties, chemiluminescent moieties, and colorigenic moieties. Advantageously, the labeling moiety is a fluorescent moiety selected from the group consisting of fluorescein derivatives, BODIPYL dyes, rhodamine derivatives, Lucifer Yellow derivatives, and cyanine (Cy) dyes.

In an alternate embodiment, the destabilizing conditions are created by methods selected from the group consisting of making temperature adjustments, making ionic strength adjustments, making adjustments in pH, and combinations thereof.

In one embodiment, the method comprises (a) contacting a single stranded target nucleic acid of interest with (i) a first sensor oligonucleotide, wherein the first sensor oligonucleotide comprises a sequence complementary to at least a portion of the target nucleic acid of interest; (ii) further contacting the target nucleic acid with at least a second sensor oligonucleotide, wherein the second sensor oligonucleotide comprises a sequence complementary to at least a portion of the target nucleic acid of interest; (b) subjecting the target nucleic acid and hybridized sensor oligonucleotides to destabilizing conditions, wherein the destabilizing conditions are sufficient to cause the first and/or second sensor oligonucleotide to dissociate under different destabilizing conditions; and (c) detecting the hybridization of the first and second sensor oligonucleotide to the target nucleic acid, whereby the presence of the polymorphism in the target nucleic acid is determined. Advantageously, the first and second sensor oligonucleotides are differently labeled with first and second labeling moieties.

In detecting a polymorphisms by differential melting temperature, the region surrounding the SNP is amplified by PCR or other amplification method. In another embodiment, a detectable label is incorporated into the system, either by use of a labeled primer, a labeled nucleotide, a labeled ribonucleotide, a labeled, modified nucleotide or a labeled, modified ribonucleotide. Alternatively, a label may be incorporated after selective hybridization has occurred, *i.e*. after the temperature has been raised to a degree whereby at least one of the fragments dissociates from the oligonucleotide probe.

The cleavage products are hybridized to oligonucleotide probes designed to maximize the difference in hybridization signal obtained from the two different alleles. For optimal detection of single-base pair mismatches, an about a 1°C to about 10°C difference in melting temperature is advantageous. When the temperature is raised above the melting temperature of a fragment-oligonucleotide duplex corresponding to one of the alleles, that allele will disassociate. The remaining fragment-oligonucleotide duplexes can then be analyzed for the incorporated label that identified the polymorphism.

The present invention provides methods for identifying the presence of one or more SNP allele in a diploid DNA sample. The detection occurs when there is a loss of florescence emitted by the sensor probe. The sensor probe acquires energy from the anchor probe once conditions are adequate for hybridization between the target (genomic) DNA and the anchor and sensor probe. Once hybridization occurs, the anchor probe transfers its florescence energy to the sensor probe, which only will emit a specific wavelength after it has acquired the energy from the anchor probe. Detection of the SNP occurs as the temperature is raised at a predetermined rate, and a reading is acquired from the florescent light emitted. If there is a presence of the mutation (SNP) the sensor probe will dissociate sooner, or at a lower temperature, since the homology between the genomic DNA and the sensor probe will be less than that of genomic DNA that does not harbor the SNP. The melt occurs lower in the case of the DNA with the SNP since the stability is compromised slightly. This occurs, obviously, on both chromosomes at the same time, thus yielding either a reading of two identical melting temperatures, or a reading of two different melting temperatures, being the heterozygote. The individuals that harbor two copies of the SNP, dubbed "tt" melt at approximately 54 °C, and the individuals containing only wild type DNA (no SNP present), dubbed "cc", melt at approximately 63 °C.

In one embodiment, the leptin (*ob*) mutation is genotyped as "tt" if the sample melts only at a low temperature (generally, at about 54°C), as "ct" if the sample melts at both a high and a low temperature (generally, about 54°C and about 63 °C), and "cc" if it melts at only the high temperature (generally, about 63°C). The melting temperatures are generally within about 4°C, advantageously within about 2°C.

In one embodiment of the invention, the oligonucleotide probes used in the above assays can be immobilized on a solid support such as, without limitation, microchips, microbeads, glass slides or any other such matrix, all of which are within the scope of this invention.

Using an assay of this type, a fluorescent labeled probe anneals to the denatured single strand When the probe hybridizes to any specific target sequence produced as a result of the amplification reaction, the reactive molecule absorbs emission energy from labeled nucleotides or donates energy to the labeled nucleotides by means of FET or FRET, thus changing the signal from the fluorescent nucleotides. Advantageously, the receptor probe takes the energy emitted from the donor probe and emits energy at a different wavelength, which is then measured. This new wavelength emission may be detected and this indicates binding of the probe. Alternatively, the reactive molecule is able to absorb fluorescence from the labeled nucleotides and so the fluorescence from these is reduced. This reduction may be detected and this indicates binding of the probe.

Most advantageously, the reactive molecule is an acceptor molecule which it emits fluorescence at a characteristic wavelength. In this case, increase in fluorescence from the acceptor molecule, which is of a different wavelength to that of the labeled nucleotide, will also indicate binding of the probe.

The presence of the labeled amplification product can be detected by monitoring fluorescence from the acceptor molecule on the probe, which specifically binds only the target sequence. In this case, signal from the amplification product can be distinguished from background signal of the fluorescent label and also from any non-specific amplification product.

An assay of this nature can be carried out using inexpensive reagents. Single labeled probes are more economical to those that include both acceptor and donor molecules.

As used herein, the expression "set of nucleotides" refers to a group of nucleotides that are sufficient to form nucleic acids such as DNA and RNA. Thus these comprise adenosine, cytosine, guanine and thymine or uracil. One or more of these is fluorescently labeled.

Amplification is suitably effected using known amplification reactions such as the polymerase chain reaction (PCR) or the ligase chain reaction (LCR), strand displacement assay (SDA) or NASBA, but advantageously PCR.

In some embodiments, the fluorescence of both the nucleotide and the acceptor molecule are monitored and the relationship between the emissions calculated.

Suitable reactive molecules (such as acceptor molecules) are rhodamine dyes or other dyes such as Cy5. These may be attached to the probe in a conventional manner. The position of the reactive molecule along the probe is immaterial although it general, they will be positioned at an end region of the probe.

In order for FET, such as FRET, to occur between the reactive molecule and fluorescent emission of the nucleotides, the fluorescent emission of the element (reactive molecule or labeled nucleotide) which acts as the donor must be of a shorter wavelength than the element acceptor. Suitable combinations are SYBRGold and rhodamine; SYBRGreen I and rhodamine; SYBRGold and Cy5; SYBRGreen I and Cy5; and fluorescein and ethidium bromide

Advantageously, the molecules used as donor and/or acceptor produce sharp peaks, and there is little or no overlap in the wavelengths of the emission. Under these circumstances, it may not be necessary to resolve the "strand specific peak" from the signal produced by amplification product. A simple measurement of the strand specific signal alone (*i.e*. that provided by the reactive molecule) will provide information regarding the extent of the FET or FRET caused by the target reaction. The ethidium bromide/fluorescein combination may fulfill this requirement. In that case, the strand specific reaction will be quantifiable by the reduction in fluorescence at 640 nm, suitably expressed as 1/Fluorescence.

However, where there is a spectral overlap in the fluorescent signals from the donor and acceptor molecules, this can be accounted for in the results, for example by determining empirically the relationship between the spectra and using this relationship to normalize the signals from the two signals.

In one method of the invention, the sample may be subjected to conditions under which the probe hybridizes to the samples during or after the amplification reaction has been completed. The process allows the detection to be effected in a homogenous manner, in that the amplification and monitoring can be carried out in a single container with all reagents added initially. No subsequent reagent addition steps are required. Neither is there any need to effect the method in the presence of solid supports (although this is an option as discussed further hereinafter).

For example, where the probe is present throughout the amplification reaction, the fluorescent signal may allow the progress of the amplification reaction to be monitored. This may provide a means for quantitating the amount of target sequence present in the sample.

During each cycle of the amplification reaction, amplicon strands containing the target sequence bind to probe and thereby generate an acceptor signal. As the amount of amplicon in the sample increases, so the acceptor signal will increase. By plotting the rate of increase over cycles, the start point of the increase can be determined.

The probe may comprise a nucleic acid molecule such as DNA or RNA, which will hybridize to the target nucleic acid sequence when the latter is in single stranded form. In this instance, step (b) will involve the use of conditions which render the target nucleic acid single stranded. Alternatively, the probe may comprise a molecule such as a peptide nucleic acid that specifically binds the target sequence in double stranded form.

In particular, the amplification reaction used will involve a step of subjecting the sample to conditions under which any of the target nucleic acid sequence present in the sample becomes single stranded, such as PCR or LCR.

It is possible then for the probe to hybridize during the course of the amplification reaction provided appropriate hybridization conditions are encountered.

In an advantageous embodiment, the probe may be designed such that these conditions are met during each cycle of the amplification reaction. Thus at some point during each cycle of the amplification reaction, the probe will hybridize to the target sequence, and generate a signal as a result of the FET or FRET. As the amplification proceeds, the probe will be separated or melted from the target sequence and so the signal generated by the reactive molecule will either reduce or increase depending upon whether it comprises the donor or acceptor molecule. For instance, where it is an acceptor, in each cycle of the amplification, a fluorescence peak from the reactive molecule is generated. The intensity of the peak will increase as the amplification proceeds because more target sequence becomes available for binding to the probe.

By monitoring the fluorescence of the reactive molecule from the sample during each cycle, the progress of the amplification reaction can be monitored in various ways. For example, the data provided by melting peaks could be analyzed, for example by calculating (the area under the melting peaks and this data plotted against the number of cycles.

The probe may either be free in solution or immobilized on a solid support, for example to the surface of a bead such as a magnetic bead, useful in separating products, or the surface of a detector device, such as the waveguide of a surface plasma resonance detector. The selection will depend upon the nature of the particular assay being looked at and the particular detection means being employed.

An increase in fluorescence of the acceptor molecule in the course of or at the end of the amplification reaction is indicative of an increase in the amount of the target sequence present, suggestive of the fact that the amplification reaction has proceeded and therefore the target sequence was in fact present in the sample.

Thus, one embodiment of the invention comprises a method for detecting nucleic acid amplification comprising: performing nucleic acid amplification on a target polynucleotide in the presence of (a) a nucleic acid polymerase (b) at least one primer capable of hybridizing to the target polynucleotide, (c) a set of nucleotides, at least one of which is fluorescently labeled and (d) an oligonucleotide probe which is capable of binding to the target polynucleotide sequence and which contains a reactive molecule which is capable of absorbing fluorescence from or donating fluorescence to the labeled nucleotide; and monitoring changes in fluorescence during the amplification reaction. Suitably, the reactive molecule is an acceptor molecule that can absorb energy from the labeled nucleotide.

The amplification is suitably carried out using a pair of primers which are designed such that only the target nucleotide sequence within a DNA strand is amplified as is well understood in the art. The nucleic acid polymerase is suitably a thermostable polymerase such as Taq polymerase.

Suitable conditions under which the amplification reaction can be carried out are well known in the art. The optimum conditions may be variable in each case depending upon the particular amplicon involved, the nature of the primers used and the enzymes employed. The optimum conditions may be determined in each case by the skilled person. Typical denaturation temperatures are of the order of 95°C, typical annealing temperatures are of the order of 55°C and extension temperatures are of the order of 72°C

Alternatively or additionally, the method of the invention can be used in hybridization assays for determining characteristics of a sequence. Thus in a further aspect, the invention provides a method for determining a characteristic of a sequence, the method comprising (a) amplifying the sequence using a set of nucleotides, at least one of which is fluorescently labeled, (b) contacting amplification product with a probe under conditions in which the probe will hybridize to the target sequence, the probe comprising a reactive molecule which is able to absorb fluorescence from or donate fluorescent energy to the fluorescent labeled nucleotide and (c) monitoring fluorescence of the sample and determining a particular reaction condition, characteristic of the sequence, at which fluorescence changes as a result of the hybridization of the probe to the sample or destabilization of the duplex formed between the probe and the target nucleic acid sequence.

Suitable reaction conditions include temperature, electrochemical, or the response to the presence of particular enzymes or chemicals. By monitoring changes in fluorescence as these properties are varied, information characteristic of the precise nature of the sequence can be achieved. For example, in the case of temperature, the temperature at which the probe separates from the sequences in the sample as a result of heating can be determined.

Another way to produce a FRET signal that discriminates between the two variant alleles is to incorporate a nucleotide with a dye that interacts with the dye on the primer. The key to achieving differential FRET is that the dye modified nucleotide must first occur (after the 3' end of the primer) beyond the polymorphic site so that, after cleavage, the nucleotide dye of one allele (cleaved) will no longer be in within the requisite resonance producing distance of the primer dye while, in the other (uncleaved) allele, the proper distance will be maintained and FRET will occur.

In the present invention, the above-described nucleic acid probe is added to a measurement system and is caused to hybridize to a target nucleic acid. This hybridization can be by conventionally known methods. As conditions for hybridization, the salt concentration may range from 0 to 2 molar concentration, advantageously from 0.1 to 1.0 molar concentration, and the pH may range from 6 to 8, advantageously from 6.5 to 7.5.

The reaction temperature may advantageously be in a range of the Tm value of the hybrid complex, which is to be formed by hybridization of the nucleic acid probe to the specific site of the target nucleic acid, +/- 10°C This temperature range can prevent non-specific hybridization. Reaction temperature lowers than Tm-10°C allows non-specific hybridization, while a reaction temperature higher than Tm+10°C allows no hybridization. Incidentally, a Tm value can be determined in a similar manner as in an experiment that is needed to design the nucleic acid probe for use in the present invention. Described specifically, an oligonucleotide which is to be hybridized with the nucleic acid probe and has a complementary base sequence to the nucleic acid probe is chemically synthesized by the above-described nucleic acid synthesizer or the like, and the Tm value of a hybrid complex between the oligonucleotide and the nucleic acid probe is then measured by a conventional method.

The reaction time may range from 1 second to 180 minutes, advantageously from 5 seconds to 90 minutes. If the reaction time is shorter than 1 second, a substantial portion of the nucleic acid probe according to the present invention remains unreacted in the hybridization. On the other hand, no particular advantage can be brought about even if the reaction time is set excessively long. The reaction time varies considerably depending on the kind of the nucleic acid, namely, the length or base sequence of the nucleic acid.

In the present invention, the nucleic acid probe is hybridized to the target nucleic acid as described above. The intensity of fluorescence emitted from the fluorescent dye is measured both before and after the hybridization, and a decrease in fluorescence intensity after the hybridization is then calculated. As the decrease is proportional to the concentration of the target nucleic acid, the concentration of the target nucleic acid can be determined.

In certain embodiments of the present invention, the detection of polymorphic sites in a target polynucleotide may be facilitated through the use of nucleic acid amplification methods. Such methods may be used to specifically increase the concentration of the target polynucleotide (*i.e*., sequences that span the polymorphic site, or include that site and sequences located either distal or proximal to it). Such amplified molecules can be readily detected by gel electrophoresis, or other means.

The most advantageous method of achieving such amplification employs PCR (see *e.g*., U.S. Pat. Nos. 4,965,188; 5,066,584; 5,338,671; 5,348,853; 5,364,790; 5,374,553; 5,403,707; 5,405,774; 5,418,149; 5,451,512; 5,470,724; 5,487,993; 5,523,225; 5,527,510; 5,567,583; 5,567,809; 5;587,287; 5,597,910; 5,602,011; 5,622,820; 5,658,764; 5,674,679; 5,674,738; 5,681,741; 5,702,901; 5,710,381; 5,733,751; 5,741,640; 5,741,676; 5,753,467; 5,756,285; 5,776,686; 5,811,295; 5,817,797; 5,827,657; 5,869,249; 5,935,522; 6,001,645; 6,015,534; 6,015,666; 6,033,854; 6,043,028; 6,077,664; 6,090,553; 6,168,918; 6,174,668; 6,174,670; 6,200,747; 6,225,093; 6,232,079; 6,261,431; 6,287,769; 6,306,593; 6,440,668; 6,468,743; 6,485,909; 6,511,805; 6,544,782; 6,566,067; 6,569,627; 6,613,560; 6,613,560 and 6,632,645; the disclosures of which are incorporated by reference in their entireties), using primer pairs that are capable of hybridizing to the proximal sequences that define or flank a polymorphic site in its double-stranded form.

In some embodiments of the present invention, the amplification method is itself a method for determining the identity of a polymorphic site, as for example, in allele-specific PCR. In allele-specific PCR, primer pairs are chosen such that amplification is dependent upon the input template nucleic acid containing the polymorphism of interest. In such embodiments, primer pairs are chosen such that at least one primer is an allele-specific oligonucleotide primer. In some sub-embodiments of the present invention, allele-specific primers are chosen so that amplification creates a restriction site, facilitating identification of a polymorphic site. In other embodiments of the present invention, amplification of the target polynucleotide is by multiplex PCR. Through the use of multiplex PCR, a multiplicity of regions of a target polynucleotide may be amplified simultaneously. This is particularly advantageous in those embodiments wherein greater than a single polymorphism is detected.

In lieu of PCR, alternative methods, such as the "Ligase Chain Reaction" ("LCR") may be used (Barany, F., Proc. Natl. Acad. Sci. (U.S.A.) 88:189-193 (1991)). The "Oligonucleotide Ligation Assay" ("OLA") (Landegren, U. et al., Science 241:1077-1080 (1988)) shares certain similarities with LCR and is also a suitable method for analysis of polymorphisms. Nickerson, D. A. *et al.* have described a nucleic acid detection assay that combines attributes of PCR and OLA (Nickerson, D. A. et al., Proc. Natl. Acad. Sci. (U.S.A.) 87:8923-8927 (1990)). Other known nucleic acid amplification procedures, such as transcription-based amplification systems (Malek, L. T. et al., U.S. Pat. No. 5,130,238; Davey, C. *et al.,* European Patent Application 329,822; Schuster et al., U.S. Pat. No. 5,169,766; Miller, H. I. et al., PCT Application WO89/06700; Kwoh, D. et al., Proc. Natl. Acad. Sci. (U.S.A.) 86:1173 (1989); Gingeras, T. R. et al., PCT Application WO88/10315)), or isothermal amplification methods (Walker, G. T. et al., Proc. Natl. Acad. Sci. (U.S.A.) 89:392-396 (1992)) may also be used.

An advantageous gene, particularly its alleles, the *ob* gene. Other genetic sequences include, but are not limited to, microsatellite loci for use in bovine parentage verification, including those designated ISAG markers, URB markers as developed by H. Lewin at the Bovine Blood Typing Lab, Saskatchewan Research Council, Saskatoon, Saskatchewan, Canada, and other species specific and genotype specific nucleotide sequences.

The invention also provides for the sequencing of the genetic sequences described above. Advantageously, the nucleic acid sequencing is by automated methods (reviewed by Meldrum, Genome Res. 2000 Sep;10(9):1288-303, the disclosure of which is incorporated by reference in its entirety). Methods for sequencing nucleic acids include, but are not limited to, automated fluorescent DNA sequencing (see, e.g., Watts & MacBeath, Methods Mol Biol. 2001;167:153-70 and MacBeath et al., Methods Mol Biol. 2001;167:119-52), capillary electrophoresis (see, e.g., Bosserhoff et al., Comb Chem High Throughput Screen. 2000 Dec;3(6):455-66), DNA sequencing chips (see, e.g., Jain, Pharmacogenomics. 2000 Aug;1(3):289-307), mass spectrometry (see, e.g., Yates, Trends Genet. 2000 Jan;16(1):5-8), pyrosequencing (see, e.g., Ronaghi, Genome Res. 2001 Jan;11(1):3-11), and ultrathin-layer gel electrophoresis (see, e.g., Guttman & Ronai, Electrophoresis. 2000 Dec;21(18):3952-64), the disclosures of which are hereby incorporated by reference in their entireties. The sequencing can also be done by any commercial company. Examples of such companies include, but are not limited to, the University of Georgia Molecular Genetics Instrumentation Facility (Athens, Georgia) or SeqWright DNA Technologies Services (Houston, Texas).

Advantageously, the amino acid sequencing is by automated methods. Methods for sequencing amino acids include, but are not limited to, alkylated-thiohydantoin method (see, e.g., Dupont et al., EXS. 2000;88:119-31), chemical protein sequencing (see, e.g., Stolowitz, Curr Opin Biotechnol. 1993 Feb;4(1):9-13), Edman degradation (see, e.g., Prabhakaran et al., J Pept Res. 2000 Jul;56(1):12-23), and mass spectrometry (see, e.g., McDonald et al., Dis Markers. 2002;18(2):99-105), the disclosures of which are incorporated by reference in their entireties. Alternatively, amino acid sequences can be deduced from nucleic acid sequences. Such methods are well known in the art, e.g., EditSeq from DNASTAR, Inc.

The results of the analysis provide the genotype data that is associated with the individual animal from which the sample was taken. The genotype data is then kept in an accessible database, and may or may not be associated with other data from that particular individual or from other animals.

The data obtained from genotyping individual animals is stored in a database which can be integrated or associated with and/or cross-matched to other databases. The database along with the associated data allows information about the individual animal to be known through every stage of the animal's life, i.e., from conception to consumption of the animal product.

Unless otherwise defined, all technical and scientific terms used herein have the same meaning as commonly understood by one of ordinary skill in the art of molecular biology. Although methods and materials similar or equivalent to those described herein can be used in the practice or testing of the present invention, suitable methods and materials are described herein. All publications, patent applications, patents, and other references mentioned herein are incorporated by reference in their entirety. In addition, the materials, methods, and examples are illustrative only and are not intended to be limiting.

### EXAMPLES

### EXAMPLE 1: Improving Protein and Milk Production of Dairy Herds

A C to T transition in exon 2 of the leptin gene that results in an Arg25Cys substitution has been associated with increased fat deposition in beef cattle (see, e.g., Buchanan et al., Genet Sel Evol. 2002 Jan-Feb;34(1):105-16). The T allele is associated with increased fat deposition in beef cattle and higher leptin mRNA levels in adipose tissue. As described in this Example, this same genetic variant is also present in dairy breeds.

Leptin is a hormone secreted predominantly from white adipose tissue and performs important roles in the control of body weight, feed intake, immune function and reproduction (see, e.g., Block et al., J Endocrinol. 2001 Nov;171(2):339-48; Santos-Alvarez et al., Cell Immunol. 1999 May 25;194(1):6-11 and Kadokawa et al., Reprod Fertil Dev. 2000;12(7-8):405-11). The primary factors affecting plasma leptin levels include body fat mass and energy balance (see, e.g., Block et al., J Endocrinol. 2001 Nov;171(2):339-48). Leptin has been shown to regulate the immune response (see, e.g., Santos-Alvarez et al., Cell Immunol. 1999 May 25;194(1):6-11) and a delay in the recovery of leptin secretion post-partum increases the delay to first ovulation in Holstein dairy cows (see, e.g., Kadokawa et al., Reprod Fertil Dev. 2000;12(7-8):405-11).

Body fat stores and energy balance change dramatically through the lactation cycle of a dairy cow. Body fat reserves play an important role in sustaining high milk production in early lactation, when energy intake is limited. In early lactation, dairy cattle are in a negative energy balance, such that energy must be drawn from the body fat of the cow to support milk production. Hence, body fat condition is increased prior to calving to provide energy stores.

To test for an association between leptin single nucleotide polymorphism (SNP) and milk productivity, 416 Holstein cows were genotyped and compared as to lactation performance data using a mixed model. Animals homozygous for the T allele-produced more milk (1.5 kg/day versus CC animals) and somatic cell count linear scores, over the entire lactation. The increase in milk yield is most prominent in the first 100 days of lactation (2.44 kg/d), declining to 1.74 k/d between 101 and 200 days in lactation. Protein yield is also increased in such TT cows. The milk and protein yield advantages, observed in cows homozygous for the T allele, represent an economic advantage to dairy producers.

Using the PCR-RFLP to distinguish the alleles (see, e.g., Buchanan et al., Genet Sel Evol. 2002 Jan-Feb;34(1):105-16), individuals from six dairy breeds were genotyped. The SNP was present in all breeds examined (Table 1). Using Dairy Herd Improvement records for 416 Holstein cows and a total of 9584 observations (from 11 Saskatchewan herds; Table 2), associations between milk production, milk fat percentage, milk protein percentage, SCC linear score and leptin genotype were analyzed. Data were analyzed using a mixed model (SAS v. 8.0 for Windows, PROC MIXED); SAS Institute, Cary, North Carolina, USA) to account for the repeated observations within cow and the clustering of observations at the herd level. Model specifications included a random statement with subject equal to cow within herd and a compound symmetry covariance structure. Initial bivariate analyses were examined looking at the association between genotype and milk production outcomes. Potentially important covariates were then introduced using a manual step-wise process to produce the final model. Additional covariates included milk fat percent, milk protein percent, days in milk (DIM), lactation number, month the lactation started (for potential seasonal effects) and linear score. The main effects model was assessed for first-order interactions where genotype and one or more covariates remained in the model with P < 0.05. Model diagnostics included visual examination of the raw and standardized residuals (SAS User's guide: Statistics, Version 8 Edition, 2000, SAS Institute, Cary, North Carolina, USA).

The analysis demonstrated a significant impact of leptin genotype on milk yield, particularly in early lactation. Table 3 provides an estimate of the increase in milk yield of TT and TC genotypes relative to the CC genotype. Over the entire lactation, the TT genotype was associated with an increase in test-day milk yield of 1.5 kg per day versus CC. This effect was most prominent in early lactation (2.44 kg/d) declining to 1.74 kg/d between 101 and 200 DIM.

Analysis of milk composition indicated a negative impact of the T allele on milk fat percent, but an insignificant impact on milk fat yield. As a result, yield of 3.5% fat corrected milk was not significantly affected by genotype. The tests show that milk fat yield was substantially constant, with the milk fat distributed through a larger milk yield, thus reducing the milk fat percent. Table 4 illustrates the effect of a T allele at the leptin SNP on test-day milk fat percent.

Analysis of test-day milk protein indicated a significant protein yield increase in TT and TC cows. Table 5 illustrates the effect of a T allele at the leptin SNP on test-day milk protein yield. Increased milk yield in cows with a T allele is thus associated with a decline in milk fat percent without changing milk protein product percent such that protein yield, but not fat yield, is significantly increased.

The analysis also showed a significant impact of genotype at the leptin SNP on milk somatic cell count linear score. Cows homozygous for the T allele demonstrated a significant increase in somatic linear score over the entire lactation (P = 0.002) and within each of the early (P = 0.018), mid (P = 0.04) and late (P = 0.033) lactation periods (Table 6).

Liefers et al. using a different intronic SNP in the leptin gene also found an increase in milk and protein yield in Holsteins (see, e.g., Liefers et al., J Dairy Sci. 2002 Jun;85(6):1633-8). However, in their population the favored allele was very rare (1/613 homozygous) compared to the high frequency of the SNP reported herein.

The results in this Example indicate that the leptin TT genotype is associated with increased milk and protein yield, without changing the yield of milk fat. Selecting TT cows for milking herds will increase the milk and protein production of the herd while maintaining milk fat yield compared to a similar sized herd of CC cows.

Table 1 depicts the frequency of the C and T alleles at the leptin SNP.

Table 2 provides a description of 11 Saskatchewan dairy herds used in the study.

Table 3 illustrates the effect of a T allele at the leptin SNP on test-day milk yield (kg/d).

Table 4 depicts the effect of a T allele at the leptin SNP on test-day milk fat percent.

Table 5 relates to the effect of a T allele at the leptin SNP on test-day milk protein yield (kg/d).

Table 6 illustrates the effect of a T allele at the leptin SNP on test-day somatic cell count linear score.

**Table 1**

| Breed | # of Animals | T allele | C allele |
|---|---|---|---|
| Holstein | 416 | 0.46 | 0.54 |
| Ayrshire | 17 | 0.62 | 0.38 |
| Brown Swiss | 21 | 0.45 | 0.55 |
| Canadienne | 9 | 0.11 | 0.89 |
| Guernsey | 16 | 0.06 | 0.94 |
| Jersey | 20 | 0.53 | 0.47 |

**Table 2**

| Item | Mean | Minimum | Maximum |
|---|---|---|---|
| Number of milking cows | 71 | 36 | 129 |
| Herd average milk yield (kg/d) | 30.5 | 19.0 | 36.8 |
| Herd average fat percent (%) | 3.68 | 2.94 | 4.51 |
| Herd average protein percent (%) | 3.22 | 3.01 | 3.44 |
| Herd average somatic cell count (cells/mL) | 300,000 | 81,000 | 518,000 |

**Table 3**

| Genotype¹ | Estimate (kg/d) | Degrees of Freedom | Probability P | Lower confidence limit (kg/d) | Upper confidence limit (kg/d) |
|---|---|---|---|---|---|
| Entire lactation | | | | | |
| TT | 1.50 | 9149 | 0.04 | 0.05 | 2.95 |
| TC | 0.91 | 9149 | 0.12 | -0.24 | 2.07 |
| CC | -- | -- | -- | -- | -- |

| 0-100 DIM² | | | | | |
|---|---|---|---|---|---|
| TT | 2.44 | 2499 | 0.004 | 0.78 | 4.11 |
| TC | 1.74 | 2499 | 0.01 | 0.41 | 3.07 |
| cc | -- | -- | -- | -- | -- |

| 101-200 DIM | | | | | |
|---|---|---|---|---|---|
| TT | 1.74 | 2507 | 0.04 | 0.11 | 3.37 |
| TC | 1.38 | 2507 | 0.04 | 0.08 | 2.68 |
| CC | -- | -- | -- | -- | -- |

| > 200 DIM | | | | | |
|---|---|---|---|---|---|
| TT | 0.24 | 3299 | 0.729 | -1.14 | 1.63 |
| TC | 0.22 | 3299 | 0.693 | -0.88 | 1.32 |
| CC | -- | -- | -- | -- | -- |

| | | | | | |
|---|---|---|---|---|---|
| ¹Covariates included milk fat percent, milk protein percent, days in milk, lactation number, month that the lactation started (for potential seasonal effects), and somatic cell linear score. ²Days in milk | | | | | |

**Table 4**

| Genotype¹ | Estimate (%) | Degrees of Freedom | Probability *P* | Lower confidence limit (%) | Upper confidence limit (%) |
|---|---|---|---|---|---|
| 0-300 DIM² | | | | | |
| TT | -0.10 | 9150 | 0.140 | -0.24 | 0.03 |
| TC | -0.07 | 9150 | 0.179 | -0.18 | 0.03 |
| CC | -- | -- | -- | -- | -- |

| 101-200 DIM | | | | | |
|---|---|---|---|---|---|
| TT | -0.15 | 2508 | 0.057 | -0.31 | 0.00 |
| TC | -0.12 | 2508 | 0.056 | -0.25 | 0.00 |
| CC | -- | -- | -- | -- | -- |

| | | | | | |
|---|---|---|---|---|---|
| ¹Covariates included milk fat percent, milk protein percent, days in milk, lactation number, month that the lactation started (for potential seasonal effects), and somatic cell linear score. ²Days in milk | | | | | |

**Table 5**

| Genotype¹ | Estimate (kg/d) | Degrees of Freedom | Probability *P* | Lower confidence limit (kg/d) . | Upper confidence limit (kg/d) |
|---|---|---|---|---|---|
| 0-300 DIM² | | | | | |
| TT | 0.043 | 9161 | 0.063 | -0.002 | 0.090 |
| TC | 0.025 | 9161 | 0.182 | -0.012 | 0.062 |
| cc | -- | -- | -- | -- | -- |

| 0-100 DIM | | | | | |
|---|---|---|---|---|---|
| TT | 0.072 | 2500 | 0.006 | 0.021 | 0.123 |
| TC | 0.050 | 2500 | 0.017 | 0.009 | 0.091 |
| CC | -- | -- | -- | -- | -- |

| 101-200 DIM | | | | | |
|---|---|---|---|---|---|
| TT | 0.047 | 2508 | 0.073 | -0.004 | 0.099 |
| TC | 0.037 | 2508 | 0.074 | -0.004 | 0.079 |
| CC | -- | -- | -- | -- | -- |

| | | | | | |
|---|---|---|---|---|---|
| ¹Covariates included milk fat percent, milk protein percent, days in milk, lactation number, month that the lactation started (for potential seasonal effects), and somatic cell linear score. ²Days in milk | | | | | |

**Table 6**

| Genotype¹ | Estimate | Degrees of Freedom | Probability *P* | Lower confidence limit | Upper confidence limit |
|---|---|---|---|---|---|
| 0-300 DIM² | | | | | |
| TT | 0.540 | 9152 | 0.002 | 0.203 | 0.876 |
| TC | 0.230 | 9152 | 0.092 | -0.037 | 0.498 |
| CC | -- | -- | -- | -- | -- |

| 0-100 DIM | | | | | |
|---|---|---|---|---|---|
| TT | 0.482 | 2513 | 0.018 | 0.083 | 0.881 |
| TC | 0.227 | 2513 | 0.164 | -0.092 | 0.546 |
| CC | -- | -- | -- | -- | -- |

| 101-200 DIM | | | | | |
|---|---|---|---|---|---|
| TT | 0.517 | 2507 | 0.040 | 0.120 | 0.913 |

| Genotype¹ | Estimate | Degrees of Freedom | Probability *P* | Lower confidence limit | Upper confidence limit |
|---|---|---|---|---|---|
| TC | 0.099 | 2507 | 0.040 | -0.218 | 0.415 |
| cc | -- | -- | -- | -- | -- |

| Plus 200 DIM | | | | | |
|---|---|---|---|---|---|
| TT | 0.386 | 3313 | 0.033 | 0.032 | 0.740 |
| TC | 0.140 | 3113 | 0.330 | -0.142 | 0.422 |
| CC | -- | -- | -- | -- | -- |

| | | | | | |
|---|---|---|---|---|---|
| ¹covariates included milk fat percent, milk protein percent, days in milk, lactation number, month that the lactation started (for potential seasonal effects), and somatic cell linear score. ²Days in milk | | | | | |

### EXAMPLE 2: Distribution of Leptin Genotype in Guelph Cows

The objective of this Example is to examine the relationship between leptin genotype and milk production in approximately 1000 mixed age dairy cows from farms in Canada.

A hair sample is collected from each animal and the specific leptin genotype is ascertained. The result of each cow will be matched against its individual performance data for milk yield, somatic cell count, milk fat and milk protein. Any associations between animal leptin genotype and milk production or composition will be evaluated and reported.

All genotype results have been reported back to the investigators and descriptive statistics of the frequency distribution of genotypes were prepared (see Table 7).

Table 7 depicts the percent distribution of leptin genotypes in Guelph cows.

**Table 7**

| Genotype | Number | Percent (%) |
|---|---|---|
| CC | 171 | 33.7 |
| CT | 262 | 51.7 |
| TT | 74 | 14.6 |
| Total | 507 | 100.0 |

### EXAMPLE 3: Relationship Between Leptin Genotype, Milk Production And Energy Balance

The objective of this Example is to examine the relationship between leptin genotype, milk production and energy balance during the peri-parturient period.

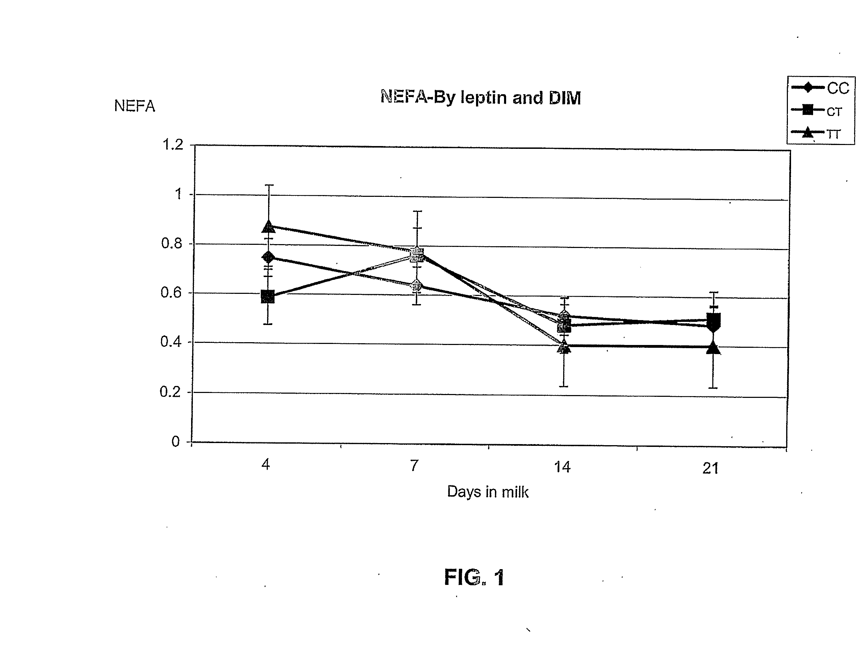

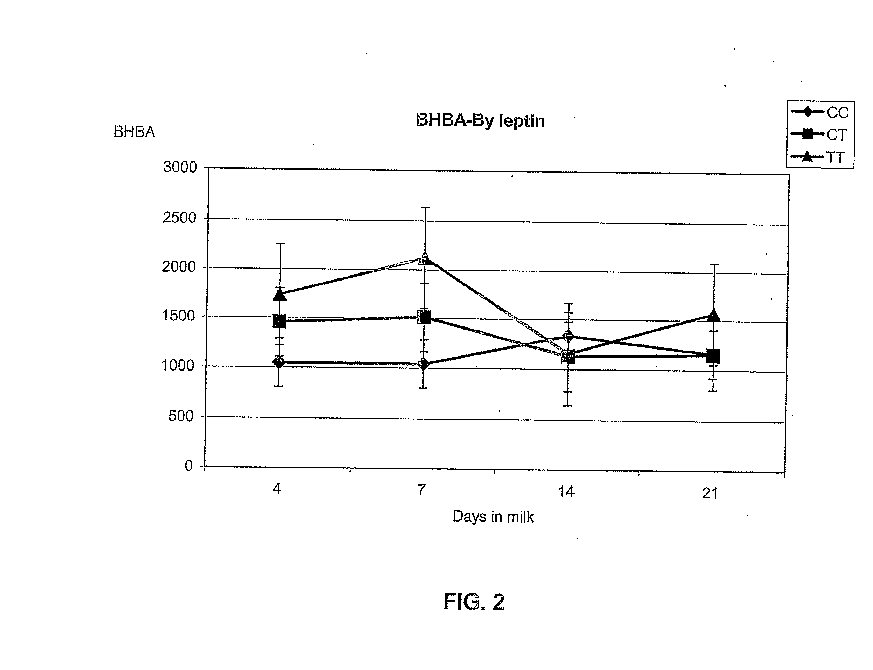

Milk yield, dry matter intake and various metabolic hormones were measured to assess the effect of leptin genotype on energy balance in dairy cows. There is evidence in some cows for increased milk yield in TT cows as well as non-esterified fatty acid (NEFA) and beta-hydroxy butyrate (BHBA) .levels indicating differences between cows in energy balance level associated with genotype (see, e.g., FIGS. 1 and 2). Cows that produce more milk should eat more, in which case they will not have effects on NEFA and BHBA. Conversely, if those cows milk more but do not eat any more, they will have elevated NEFA and BHBA levels.

The energy balance in the dairy cow is related to dry matter intake (DMI). The DMI drops 32% in the last three weeks prepartum and 90% of that drop is in the last week. Heifers eat less than cows in an absolute sense (lbs/day) and in a relative sense (% body weight). Diet composition, BCS (Body Condition Score or the amount of fat on the animal), parity and time from calving together explain only 18% of cow-to-cow variation in prepartum DMI.

A dairy cow faces metabolic challenges during the transition from prepartum to postpartum. Decreased DMI and increased output of energy, vitamins and minerals lead to negative energy balance (NEB) and hypocalcemia which results in immunosuppression. Burning NEFA in liver may inhibit DMI and probable roles of insulin (especially postpartum) and leptin decreases DMI. Decreased DMI is part of the fatty liver syndrome, in which decreased DMI leads to increased NEB, which results in increased fat mobilization, leading to increased NEFA, which results in increased ketosis and fat accumulation in liver, which leads to decreased DMI. If DMI is maintained throughout transition (see, e.g., Bertics et al., J Dairy Sci. 1992 Jul;75(7):1914-22) or if energy intake is supplemented (see, e.g., Studer et al., J Dairy Sci. 1993 Oct;76(10):2931-9), then there is a decrease in liver fat accumulation.

High-producing dairy cows inevitably experience a period of negative-energy balance during early lactation. The extent and duration of postpartum negative energy balance has been related to an increased incidence of metabolic disorders, and decreased reproductive efficiency. Cows that undergo severe and/or prolonged energy deficits are at increased risk of subclinical ketosis and several other health problems (see, e.g., Duffield, Vet Clin North Am Food Anim Pract. 2000 Jul;16(2):231-53). In addition, the role of negative energy balance in the etiology of displaced abomasums in postparturient dairy cows has recently been reviewed (see, e.g., Geishauser et al., Vet Clin North Am Food Anim Pract. 2000 Jul;16(2):255-65).

A great deal of research and extension has been expended to develop and implement strategies for improvement of negative energy balance in periparturient dairy cows. Most dairy producers have gone to considerable effort to organize transition management and nutritional programs for their cows immediately before and after calving. In addition, supplementation programs are widely used to improve energy sources or utilization during this period. A monensin controlled release capsule is available for prevention of subclinical kestosis and displaced abomasums (see, e.g., Duffield et al., J Dairy Sci. 1998 Sep;81(9):2354-61 and Duffield et al., J Dairy Sci. 1998 Nov;81(11-):2866-73). Rumen-protected choline is commonly used in transition cow diets to improve liver metabolism and energy utilization (see, e.g., Erdman & Sharma, J Dairy Sci. 1991 May;74(5):1641-7). Propylene glycol is used for both therapy and prevention of subclinical ketosis (see, e.g., Studer et al., J Dairy Sci. 1993 Oct;76(10):2931-9). All of these strategies are quite successful in assisting dairy producers to manage their cows through this stressful period of the lactation cycle. It is interesting to note that there is wide variability between cows in the success of these strategies (see, e.g., Duffield, Vet Clin North Am Food Anim Pract. 2000 Jul; 16(2):231-53). The reasons for this variability are far from fully elucidated.

Leptin is a naturally occurring hormone secreted by adipocytes and is involved in the control of energy balance (see, e.g., Liefers et al., Mamm Genome. 2003 Sep;14(9):657-63). The production of leptin is controlled by a single gene location. Leptin concentrations in the blood affect the regulation of food consumption and energy expenditure. As leptin concentrations rise, the body responds by reducing appetite and increasing metabolism (see, e.g., Liefers et al., Mamm Genome. 2003 Sep;14(9):657-63). However, there is a lack of information on the control of these mechanisms. In a recent report, it was concluded that plasma leptin was regulated by nutrition in early postnatal life, but that a sudden increase in plasma leptin is not required for the onset of puberty in dairy cattle (see, e.g., Block et al., J Dairy Sci. 2003 Oct;86(10):3206-14).

Body fat reserves play an important role in sustaining milk production in early lactation, when energy intake is limiting. Allelic variation (C to T transition) in the leptin gene has been associated with increased fat deposition in beef cattle (see, e.g., Buchanan et al., Genet Sel Evol. 2002 Jan-Feb;34(1):105-16). The T allele was associated with increased fat deposition and higher leptin mRNA levels in adipose tissue. In a recent study, animals that were homozygous for the T allele produced more milk, had higher somatic cell count linear scores, without significantly affecting milk fat or protein percent over the entire lactation (see, e.g., Buchanan et al., J Dairy Sci. 2003 Oct;86(10):3164-6). Thus, leptin genotype testing could provide considerable insight into the metabolic status and milk production potential of dairy cattle, as well as to guide dairy producers with selection, breeding and herd management decisions (see, e.g., Buchanan et al., J Dairy Sci. 2003 Oct;86(10):3164-6).

Recently, the Igenity-L genotype test has become commercially available. This test determines the status of an individual animal for the C and T leptin alleles. The test is performed on DNA extracted from hair follicles.

The association between the leptin genotype and various aspects of periparturient metabolism and performance in dairy cattle are to be determined. It is hypothesized that leptin allele type will be associated with dry matter intake, metabolic indicators of energy balance in blood, liver metabolism, general health and production in periparturient dairy cattle.

The objectives are to (1) determine the associations between leptin genotype, dry matter intake, health and production; (2) to study the relationships of leptin genotype with metabolic function and liver concentrations of glycogen and triglyceride in the periparturient period and (3) to evaluate interactions between the effect of supplementation with monensin, choline or propylene glycol and leptin genotype.

The experimental animals will be from two distinct populations of dairy cows that have been intensively studied through their involvement in separate transition cow research efforts. Both populations were used to study the effect of specific intervention protocols on the metabolism and productivity of the cows involved. For each of the populations, very similar (but not exactly the same) outcome variables, have been collected. For example, one of the populations had serum profiles taken only in the postpartum period. Also, a subset of animals in one of the populations had liver biopsies taken at calving and a Day 28 postpartum. The details of the outcome variables will be described later. The two distinct populations were obtained following their participation in the specific experiments as follows.

In Experimental Group A, approximately 180 primaparous and multiparous Holstein animals at the University of Guelph Elora and Posenby dairy research centers were used to conduct this study. Cows were housed in a tie-stall barn from three weeks prior to expected calving date. The animals were moved to maternity pens for calving, and then transferred back to the tie-stall barn for the duration of the lactation. Cows were fed a total mixed ration (TMR) twice daily and were milked twice daily. Disease treatment protocols and complete diet information were provided. Cows were in good general health at the time of enrollment.

At enrollment, between day 24 and day 21 prior to expected calving date, each cow or heifer was randomly assigned to one of four treatment groups: rumen-protected choline top-dress or nothing, and/or a monensin controlled release capsule. For the choline-supplemented animals, feed was top-dressed every day thereafter until 28 days postpartum. Staff at Elora Daily Research Station were responsible for administration of the monensin controlled-release capsule and delivery of the rumen protected choline top-dress; however, were not involved in any of the assessment or data collection.

In Experimental Group B, approximately 45 Holstein cows at the Pennsylvania State University dairy herd were used to conduct this study. After calving, cows went into the tiestall barn and were fed the herd TMR for fresh cows. Each cow was randomly assigned to one of three treatment groups. One group received the dry propylene glycol product (Propylene 65 at a rate of 250 g/day) in the TMR. The second group received the same rate of dried propylene glycol supplementation, but as a top-dress on their TMR. Cows in the third group were assigned to be non-supplemented control cows. Milk production was measured for three weeks during which time weekly milk composition (AM and PM samples) were measured (to start no earlier than three days after calving. Milk Ketone analyses were conducted at the same time milk was sampled. Cows were allocated to treatment such that previous 305 day ME (Mature Equivalent lactation record), calving date, lactation number, days dry and body condition score is balanced across treatments. Body condition was evaluated at dry off, and weekly after calving. Blood was collected for serum metabolic profile analysis at 4 days after calving, and weekly until three weeks after calving. All health records of cows were recorded. Milk production was measured daily up to 56 days in milk.

The samples and measurements collected for each of the major outcome variables is as follows.

For serum metabolic profiles, blood samples were taken from the coccygeal vein and tested for a serum metabolic profile, which includes beta-hydroxybutyrate (BHB), glucose, non-esterified fatty acids (NEFA), urea, aspartate transaminase (AST) and cholesterol. Blood samples were taken at enrollment, one week prior to expected calving, and at one and two weeks post calving. Blood serum were stored by freezing at -20 C. Batches of serum were submitted to the University of Guelph Animal Health Laboratory for analysis on a periodic basis.

For milk ketones, milk samples were collected during week one and week two following calving. Ketone levels will be determined immediately following sample collection using the KetoTest cowside ketone test.

Body condition scores (BCS) were determined at the time of enrollment and two weeks after calving (day +8 to +14) for each animal using a five-point scale with quarter point intervals.

Weekly milk production was measured using automated milk recording equipment in the milking parlour. Milk weights will be collected on a daily basis from calving until Day 60 postpartum. A weekly average was taken.

For milk components and somatic cell counts, both yield and percentage composition of milk butterfat and protein were determined from samples collected for Ontario DHI records. This is done on an approximately 30 days test date interval. In addition, milk somatic cell counts (SCC) were determined on the source samples. These data will be collected for the first two test dates postpartum.

Liver biopsies were performed on approximately 60 cows in Experiment A (15 randomly selected animals from each treatment group) to determine glycogen and triglyceride content. These biopsies were taken by a trained technical associate during the first and fourth weeks after calving.

Disease occurrences and treatments were recorded in the animal's lifetime record. Standard disease protocols were followed.

Statistical analyses will be conducted using the Statistical Analysis Software (SAS). Descriptive statistics will be determined by comparing the differences between the three leptin allele groups. Multivariable regression analysis in SAS will be used to determine the associations between individual cow leptin allele status and the biological performance and metabolic outcome variables, while controlling for the effects of the energy status enhancement strategies that were evaluated in Experiments A and B, respectively.

### EXAMPLE 4: Relationship Between Leptin Genotype, Milk Production, Milk Components And Dry Matter Intake

The objective of this Example is to examine the relationship between leptin genotype, milk production, milk components and dry matter intake in cows during the peri-parturient period.

Production data from 80 cows was retrospectively assessed for any associations with leptin genotype.

Preliminary data suggests an effect of leptin genotype on dry matter intake up to 4 weeks post-calving, and an effect on milk production, value per lactation, milk fat content and milk protein content. This data will be evaluated for its effect on energy balance and changes recommended to ration formulation in order that diet can most appropriately be linked to individual production.

Table 8 provides dry matter intake (DMI) in pounds (weeks before freshening and after freshening).

Table 9 depicts ME (305-day Mature Equivalent) lactation production by genotype and lactation number.

Table 10 relates to average all lactation ETA (Estimated Transmitting Ability).

Table 11 illustrates first lactation ETA (Estimated Transmitting Ability).

Table 12 provides for less than 200 days in milk (test day model (TDM) = residuals from the test day model, milk is just the raw average).

Table 13 relates to less than 100 days in milk (TDM = residuals from the test day model, milk is just the raw average).

**Table 8**

| Week | CC-DMI | CC-n | CT-DMI | CT-n | TT-DMI | TT-n |
|---|---|---|---|---|---|---|
| -4 | 30.98 | 22 | 29.71 | 22 | 30.57 | 13 |
| -3 | 31.89 | 30 | 31.03 | 30 | 31.51 | 20 |
| -2 | 31.05 | 36 | 29.88 | 36 | 31.42 | 26 |
| -1 | 26.04 | 36 | 25.27 | 36 | 27.87 | 27 |
| 1 | 29.32 | 30 | 31.86 | 30 | 35.04 | 20 |
| 2 | 36.92 | 30 | 37.35 | 30 | 38.79 | 20 |
| 3 | 40.73 | 30 | 41.43 | 30 | 42.60 | 20 |
| 4 | 43.07 | 30 | 43.55 | 30 | 44.84 | 20 |
| 5 | 44.71 | 23 | 42.75 | 23 | 42.15 | 13 |
| 6 | 46.31 | 23 | 43.52 | 23 | 45.55 | 13 |
| 7 | 48.47 | 23 | 45.35 | 23 | 43.47 | 13 |
| 8 | 48.76 | 23 | 46.19 | 23 | 45.36 | 13 |
| 9 | 48.74 | 23 | 48.58 | 23 | 45.74 | 13 |

**Table 9**

| Test | Lact | MEM | MEF | MEP | Count |
|---|---|---|---|---|---|
| CC | 1 | 34665 | 1176 | 960 | 22 |
| cc | 2 | 30045 | 915 | 783 | 19 |
| CC | 3 | 29620 | 950 | 785 | 12 |
| CC | 4 | 29722 | 947 | 758 | 7 |
| CT | 1 | 33726 | 1222 | 965 | 32 |
| CT | 2 | 30308 | 1093 | 851 | 29 |
| CT | 3 | 29592 | 1053 | 804 | 21 |
| CT | 4 | 27544 | 954 | 692 | 15 |
| TT | 1 | 33569 | 1234 | 963 | 13 |
| TT | 2 | 31820 | 1052 | 801 | 12 |
| TT | 3 | 29918 | 1061 | 825 | 9 |
| TT | 4 | 25819 | 974 | 740 | 4 |

| | | | | | |
|---|---|---|---|---|---|
| MEM: Mature Equivalent Milk production MEF: Mature Equivalent Fat production in kilograms MEP: Mature Equivalent Protein production in kilograms Count: Somatic Cell Count (1000 cells/mL of milk) | | | | | |

It should be noted that MEM, MEF, MEP and Count are all measures of the production of milk or milk component for a hypothetical 305-day lactation period, or the average cell count on a test day for somatic cell count.

**Table 10***

| Test | $$$¹ | Milk² | Fat³ | Protein⁴ | Count⁵ |
|---|---|---|---|---|---|
| CC | 182 | 1522 | 25 | 39 | 22 |
| CT | 196 | 1454 | 32 | 40 | 33 |
| TT | 218 | 1634 | 36 | 45 | 13 |

| | | | | | |
|---|---|---|---|---|---|
| ¹estimated value of the production of the animal in comparison to breed averages ²estimated additional milk production per lactation for test cows in comparison to breed average ³estimated additional fat production for test cows in comparison to breed average ⁴estimated additional protein production for test cows in comparison to breed average ⁵average somatic cell count for test cows (1000 cells/mL) *the figures shown in this table are quoted for all lactations | | | | | |

**Table 11***

| Test | $$$¹ | Milk² | Fat³ | Protein⁴ | Count⁵ |
|---|---|---|---|---|---|
| CC | 195 | 1635 | 29 | 41 | 21 |
| CT | 188 | 1393 | 32 | 38 | 30 |
| TT | 222 | 1608 | 35 | 46 | 12 |

| | | | | | |
|---|---|---|---|---|---|
| ¹estimated value of the production of the animal in comparison to breed averages ²estimated additional milk production per lactation for test cows in comparison to breed average ³estimated additional fat production for test cows in comparison to breed average ⁴estimated additional protein production for test cows in comparison to breed average ⁵average somatic cell count for test cows (1000 cells/mL) *the figures shown in this table are quoted for 1^{st} lactation cows. | | | | | |

**Table 12**

| Test | Lact | TDM¹ Milk | TDM Fat | TDM Pro | TDM RSCC² | Milk | Fat % | Pro % | SCC³ | Count |
|---|---|---|---|---|---|---|---|---|---|---|
| CC | 1 | 0.59 | -0.194 | -0.48 | -0.219 | 82 | 3.33 | 2.41 | 1.63 | 115 |
| CC | 2 | 4.76 | -0.026 | 0.052 | -0.072 | 99 | 3.20 | 2.43 | 2.21 | 78 |
| CC | 3 | 11.25 | 0.377 | 0.101 | 0.262 | 112 | 3.26 | 2.58 | 3.12 | 44 |
| CC | 4 | 12.16 | 0.004 | 0.162 | 0.948 | 109 | 3.41 | 2.68 | 4.64 | 23 |
| CT | 1 | -1.11 | -0.153 | -0.0028 | -0.051 | 81 | 3.56 | 2.44 | 1.91 | 170 |
| CT | 2 | -0.72 | 0.061 | 0.022 | -0.025 | 91 | 3.62 | 2.25 | 2.35 | 157 |
| CT | 3 | 1.69 | -0.581 | -0.043 | -0.373 | 102 | 3.61 | 2.64 | 2.06 | 98 |
| CT | 4 | 2.78 | 0.152 | 0.045 | -0.496 | 104 | 3.83 | 2.60 | 2.35 | 46 |
| TT | 1 | -0.51 | -0.017 | 0.053 | -0.084 | 80 | 3.62 | 2.48 | 1.86 | 74 |
| TT | 2 | 3.62 | -0.062 | 0.019 | -0.322 | 98 | 3.60 | 2.60 | 1.80 | 53 |
| TT | 3 | -2.21 | 0.544 | -0.038 | -0.738 | 96 | 3.82 | 2.75 | 1.67 | 38 |
| TT | 4 | -14.21 | 0.081 | -0.237 | 0.400 | 87 | 3.92 | 2.11 | 3.50 | 14 |

| | | | | | | | | | | |
|---|---|---|---|---|---|---|---|---|---|---|
| ¹test day model ² residual of somatic cell count ³somatic cell counts | | | | | | | | | | |

**Table 13**

| Test | Lact | TDM¹ Milk | TDM Fat | TDM Pro | TDM SCC | Milk | Fat % | Pro % | SCC² | Count |
|---|---|---|---|---|---|---|---|---|---|---|
| CC | 1 | -1.50 | -0.079 | -0.098 | -0.113 | 79 | 3.38 | 2.10 | 1.81 | 54 |
| CC | 2 | -0.46 | -0.244 | -0.098 | -0.209 | 97 | 3.31 | 2.19 | 1.83 | 41 |
| cc | 3 | 9.54 | 0.210 | -0.044 | 0.212 | 113 | 3.45 | 2.49 | 2.70 | 23 |
| CC | 4 | 15.46 | -0.275 | 0.210 | 0.974 | 115 | 3.49 | 2.66 | 4.62 | 15 |
| CT | 1 | -3.32 | -0.028 | -0.090 | -0.149 | 773 | 3.65 | 2.03 | 1.81 | 81 |
| CT | 2 | -0.89 | -0.125 | 0.006 | 0.038 | 943 | 3.70 | 1.78 | 2.23 | 81 |
| CT | 3 | -1.32 | 0.391 | -0.208 | -0.411 | 102 | 3.76 | 2.53 | 1.92 | 49 |
| CT | 4 | 0.82 | -0.047 | -0.136 | -0.450 | 103 | 4.08 | 2.41 | 2.59 | 27 |
| TT | 1 | -2.16 | 0.027 | 0.046 | 0.102 | 76 | 3.81 | 2.14 | 2.14 | 35 |
| TT | 2 | 3.33 | -0.169 | -0.050 | -0.082 | 100 | 3.96 | 2.47 | 2.03 | 27 |
| TT | 3 | -1.90 | 0.321 | -0.106 | -0.858 | 99 | 3.92 | 2.59 | 1.32 | 21 |
| TT | 4 | -1.69 | -0.125 | -0.087 | -0.217 | 94 | 3.70 | 1.38 | 2.40. | 8 |

| | | | | | | | | | | |
|---|---|---|---|---|---|---|---|---|---|---|
| ¹TDM = test day model ²SCC = somatic cell counts | | | | | | | | | | |

The invention is further described by the following numbered paragraphs:
1. A composition for the detection of *ob* gene polymorphisms, comprising at least one oligonucleotide consisting essentially of a nucleic acid sequence which complements and specifically hybridizes to an *ob* gene nucleic acid molecule, wherein the sequence is at least 80% homologous to a sequence selected from the group consisting of SEQ ID NO:4, SEQ ID NO:5, SEQ ID NO:6, and SEQ ID NO:7.
2. A composition for the detection of *ob* gene polymorphisms, comprising at least one oligonucleotide consisting essentially of a nucleic acid sequence which complements and specifically hybridizes to an *ob* gene nucleic acid molecule, wherein the sequence is selected from the group consisting of SEQ ID NO:4, SEQ ID NO:5, SEQ ID NO:6, and SEQ ID NO:7, and a nucleotide sequence which differs from SEQ ID NO:4, SEQ ID NO:5, SEQ ID NO:6, and SEQ ID NO:7 by a one base change or substitution therein.
3. An isolated and purified oligonucleotide primer pair for enzymatic amplification of *ob* gene DNA, comprising a pair of nucleic acid sequences which complement and specifically hybridize to an *ob* gene nucleic acid molecule, wherein the pair of nucleic acid sequences is at least 95% homologous to sequences selected from the group consisting of (a) the oligonucleotide pair of SEQ ID NO:4 and SEQ ID NO:5 and (b) the oligonucleotide pair of SEQ ID NO:6 and SEQ ID NO:7.
4. An isolated and purified oligonucleotide primer pair for enzymatic amplification of *ob* gene DNA, comprising a pair of nucleic acid sequences which complement and specifically hybridize to an *ob* gene nucleic acid molecule, wherein the pair of nucleic acid sequences is selected from the group consisting of (a) the oligonucleotide pair of SEQ ID NO:4 and SEQ ID NO:5, and (b) a nucleotide pair which differs from SEQ ID NO:4 and SEQ ID NO:5 by a one base change or substitution therein.
5. An oligonucleotide primer for identifying bovine having an *ob* gene polymorphism, the primer comprising at least 10 nucleotides in length and which includes at least nine contiguous nucleotides of a sequence selected from the group consisting of SEQ ID NO:4 and SEQ ID NO:5.
6. An oligonucleotide probe for identifying bovine having an *ob* gene polymorphism, the probe comprising at least 10 nucleotides in length and which includes at least nine contiguous nucleotides of a sequence selected from the group consisting of SEQ ID NO:6 and SEQ ID NO:7.
7. The composition of paragraph 1, 2, 3, 4, 5 or 6 wherein the oligonucleotide is labeled with a detectable moiety.
8. The composition of paragraph 7 wherein the detectable moiety is selected from the group consisting of a digoxigenin-dUTP, biotin, calorimetric, fluorescent, chemiluminescent, electrochemiluminescent signal and a radioactive component.
9. The composition of paragraph 7, wherein the detectable moiety is a fluorescent component generating a fluorescent signal.
10. The composition of paragraph 5 wherein the primer is from about 10 to about 24 bases in length.
11. The composition of paragraph 6 wherein the primer is from about 14 to about 30 bases in length.
12. The composition of paragraph 5 wherein the primer is immobilized on a solid support.
13. An oligonucleotide microarray having immobilized thereon a plurality of probes, wherein at least one of said probes is specific for the variant form of the single nucleotide polymorphism at position 189 of SEQ ID NO:1.
14. An oligonucleotide microarray having immobilized thereon a plurality of probes, wherein at least one of said probes is specific for the reference form of the single nucleotide polymorphism at position 189 of SEQ ID NO: 1.
15. The microarray of paragraph 13 wherein the probe is a nucleic acid sequence which complements and specifically hybridizes to an *ob* gene nucleic acid molecule, wherein the sequence is selected from the group consisting of SEQ ID NO:4, SEQ ID NO:5, SEQ ID NO:6, and SEQ ID NO:7, and a nucleotide sequence which differs from SEQ ID NO:4, SEQ ID NO:5, SEQ ID NO:6, and SEQ ID NO:7 by a one base change or substitution therein.
16. A method for analyzing or determining polymorphism or mutation of a target nucleic acid or gene, which comprises hybridizing a nucleic acid probe according to paragraph 7 to the target nucleic acid or gene, and measuring a change in detectable moiety.
17. A method for analyzing or determining polymorphism or mutation of a target nucleic acid or gene, which comprises hybridizing a nucleic acid probe according to paragraph 9 to the target nucleic acid or gene, and measuring a change in fluorescence.
18. A method of detecting the presence of *ob* gene polymorphisms in a nucleic acid sample comprising: comprising (a) contacting the target nucleic acid of interest with at least one sensor oligonucleotide, wherein the sensor oligonucleotide comprises a sequence complementary to at least a portion of the target nucleic acid of interest, wherein the sensor oligonucleotide hybridizes to the target nucleic acid at a position suspected of containing the *ob* gene polymorphism and (b) subjecting the captured target nucleic acid and hybridized sensor probe oligonucleotide to destabilizing conditions, wherein the destabilizing conditions are sufficient to cause the sensor oligonucleotide to dissociate under differing conditions depending upon the presence of the cc, ct or tt polymorphisms in the ob gene.
19. The method of paragraph 18 wherein the method further comprises (c) detecting the hybridization of the sensor oligonucleotide to the target nucleic acid under the varying destabilizing conditions, whereby the presence of the specific sequence in the target nucleic acid is determined.
20. The method of paragraph 18 wherein the method further comprises a preparatory step of amplifying one or more target nucleic acid sequences from the nucleic acids of a sample, wherein the amplicons become the target nucleic acids,
21. The method of paragraph 20 wherein the amplification step produces single stranded amplicons, which are then utilized as the single stranded target nucleic acids.
22. The method of paragraph 20 wherein the amplification step produces double stranded amplicons, further comprising a step of subjecting the amplicons to denaturing conditions to form single stranded target nucleic acids.
23. The method of paragraph 20 wherein the amplification step is by an amplification method selected from the group consisting of polymerase chain reaction (PCR), strand displacement amplification (SDA), nucleic acid sequence-based amplification (NASBA), rolling circle amplification, T7 mediated amplification, T3 mediated amplification, and SP6 mediated amplification.
24. The method of paragraph 18 wherein the detection of the hybridization of the sensor oligonucleotide is by the detection of a labeling moiety on the sensor oligonucleotide selected from the group consisting of fluorescent moieties, bioluminescent moieties, chemiluminescent moieties, and colorigenic moieties. Advantageously, the labeling moiety is a fluorescent moiety selected from the group consisting of fluorescein derivatives, BODIPYL dyes, rhodamine derivatives, Lucifer Yellow derivatives, and cyanine (Cy) dyes.
25. The method of paragraph 18 wherein the destabilizing conditions are created by methods selected from the group consisting of making temperature adjustments, making ionic strength adjustments, making adjustments in pH, and combinations thereof.
26. A method of detecting the presence of *ob* gene polymorphisms in a nucleic acid sample comprising: (a) contacting a single stranded target nucleic acid of interest with (i) a first sensor oligonucleotide, wherein the first sensor oligonucleotide comprises a sequence complementary to at least a portion of the target nucleic acid of interest; (ii) further contacting the target nucleic acid with at least a second sensor oligonucleotide, wherein the second sensor oligonucleotide comprises a sequence complementary to at least a portion of the target nucleic acid of interest; (b) subjecting the target nucleic acid and hybridized sensor oligonucleotides to destabilizing conditions, wherein the destabilizing conditions are sufficient to cause the first and/or second sensor oligonucleotide to dissociate under different destabilizing conditions; and (c) detecting the hybridization of the first and second sensor oligonucleotide to the target nucleic acid, whereby the presence of the polymorphism in the target nucleic acid is determined.
27. The method of paragraph 1 wherein the first and second sensor oligonucleotides are differently labeled with first and second labeling moieties.
28. A method of detecting the presence of *ob* gene polymorphisms in a nucleic acid sample comprising: a) contacting the sample with a hybridization probe comprising one or more oligonucleotides of at least 10 nucleotides in length comprising at least nine contiguous bases of the sequences selected from the group consisting of SEQ ID NO:4, SEQ ID NO:5, SEQ ID NO:6, and SEQ ID NO:7, labeled with a detectable moiety, under suitable conditions permitting hybridization of the labeled oligonucleotide probe to the *ob* gene nucleic acid to form a hybridization complex, and b) detecting the presence of the probe bound to the nucleic acid sequences by detecting the detectable moiety of the labeled oligonucleotide probe hybridized to the *ob* gene polymorphism sequences.
29. A method of detecting the presence of *ob* gene polymorphism in a nucleic acid sample comprising: a) obtaining a nucleic acid molecule sample containing an *ob* gene polymorphism from a subject; b) amplifying a region of the *ob* gene polymorphism using the oligonucleotide pair of SEQ ID NO:4 and SEQ ID NO:5 to form nucleic acid amplification products; c) contacting the amplified *ob* gene polymorphism sequences from step (b), if present, with hybridization probes comprising the oligonucleotide pair of SEQ ID NO:6 and SEQ ID NO:7, labeled with a detectable moiety under suitable conditions permitting hybridization of the labeled oligonucleotide probe to amplified *ob* gene polymorphism sequences to form a hybridization complex, and d) detecting the presence of amplified *ob* gene polymorphism sequences by detecting the detectable moiety of the labeled oligonucleotide probe hybridized to the amplified *ob* gene polymorphism sequences.
30. The methods of paragraphs 29, wherein the method further comprises, after contacting the *ob* gene polymorphism sequences with hybridization probes, subjecting the hybridized complex structures to destabilizing conditions sufficient to cause the probes to dissociate from the complex structures if there is at least one base-pair mismatch between the probes and the target nucleic acids or amplification products, and detecting a loss or a retention of the probes from the hybridization complex.
31. The method of paragraph 29 wherein the amplification step is by an amplification method selected from the group consisting of polymerase chain reaction (PCR), strand displacement amplification (SDA), nucleic acid sequence-based amplification (NASBA), rolling circle amplification, T7 mediated amplification, T3 mediated amplification, and SP6 mediated amplification.
32. The method of paragraph 29 wherein the method comprises a step of subjecting the target nucleic acids of the sample to denaturing conditions to form single stranded target nucleic acids.
33. The method of paragraph 29 wherein the detection of the hybridization of the sensor oligonucleotide is by the detection of a labeling moiety on the sensor oligonucleotide selected from the group consisting of fluorescent moieties, bioluminescent moieties, chemiluminescent moieties, and colorigenic moieties. Advantageous, the labeling moiety is a fluorescent moiety selected from the group consisting of fluorescein derivatives, BODIPYL dyes, rhodamine derivatives, Lucifer Yellow derivatives, and cyanine (Cy) dyes.
34. The method of paragraph 29 wherein the destabilizing conditions are created by methods selected from the group consisting of making temperature adjustments, making ionic strength adjustments, making adjustments in pH, and combinations thereof.
35. The method of paragraph 29, wherein the presence of the amplified *ob* gene polymorphism sequences hybridized to labeled oligonucleotide probe correlates to the subject's propensity to deposit fat.
36. The method of paragraph 29, wherein the amplified DNA sequences are from the *ob* region of the Bos taurus genome.
37. The method of paragraph 29, additionally comprising adding an internal standard for accessing relative amounts of DNA after amplification.
38. The method of paragraph 29, wherein presence of the amplified *ob* gene polymorphism sequences hybridized to labeled oligonucleotide probe is correlated to the presence of an *ob* gene polymorphism in the sample by comparing the amount of amplification product to the quantity of amplification products formed from known internal standards.
39. The method of paragraph 29, wherein the primers comprise the oligonucleotide pair of SEQ ID NO:4 and SEQ ID NO:5.
40. The method of paragraph 29, wherein the detectable moiety is selected from the group consisting of a digoxigenin-dUTP, biotin, calorimetric, fluorescent, chemiluminescent, electrochemiluminescent signal and a radioactive component.
41. The method of paragraph 29, wherein the detectable moiety is a fluorescent component generating a fluorescent signal.
42. A method of selecting livestock comprising the steps of: a) obtaining a nucleic acid molecule sample containing an *ob* gene polymorphism from livestock; b) amplifying a region of the *ob* gene polymorphism using the oligonucleotide pair of SEQ ID NO:4 and SEQ ID NO:5 to form nucleic acid amplification products; c) contacting the amplified *ob* gene polymorphism sequences from step (b), if present, with hybridization probes comprising the oligonucleotide pair of SEQ ID NO:6 and SEQ ID NO:7, labeled with a detectable moiety under suitable conditions permitting hybridization of the labeled oligonucleotide probe to amplified *ob* gene polymorphism sequences to form duplex structures, d) detecting the presence of amplified *ob* gene polymorphism sequences by detecting the detectable moiety of the labeled oligonucleotide probe hybridized to the amplified *ob* gene polymorphism sequences; and e) identifying those livestock animals having a greater or lesser milk productivity based on the detection.
43. A diagnostic test kit for detection of an *ob* gene polymorphism comprising: (a) at least one oligonucleotide primer pair selected from the group consisting of the oligonucleotide pair of SEQ ID NO:4 and SEQ ID NO:5, and (b) at least one oligonucleotide probe labeled with a detectable moiety selected from the group consisting SEQ ID NO:6 and SEQ ID NO:7.
44. The diagnostic test kit of paragraph 43, further comprising at least one additional reagent selected from the group consisting of a lysing buffer for lysing cells contained in the specimen; enzyme amplification reaction components dNTPs, reaction buffer, and amplifying enzyme; and a combination thereof.
45. The diagnostic kit of paragraph 43, wherein the primers comprise the oligonucleotide pair of SEQ ID NO:4 and SEQ ID NO:5.
46. The diagnostic kit of paragraph 43, wherein the hybridization probes comprise SEQ ID NO:6 and SEQ ID NO:7.
47. The diagnostic kit of paragraph 43, wherein the hybridization probe further comprises a detectable moiety selected from the group consisting of a chemiluminescent component, a fluorescent component, and a radioactive component.
48. A method of increasing milk production in a selected group of livestock animals of the same species comprising:
   (i) determining a genetic predisposition of each animal to produce milk by determining their *ob* genotype; and
   (ii) selecting animals that possess the T-containing allele of the *ob* gene for inclusion in the group.
49. The method of paragraph 48 wherein increasing milk production in a selected group of livestock animals of the same species occurs during the first one hundred days of lactation.
50. The method of paragraph 49 wherein determining comprises determining whether the animal is a TT animal homozygous with respect to the T-allele of the *ob* gene, a CC animal homozygous with respect to the C-allele of the *ob* gene, or a CT animal heterozygous with respect to the T-allele and the C-allele of the *ob* gene.
51. A method of paragraph 50 wherein selecting is selecting from the group consisting of TT animals homozygous with respect to the T-allele of the *ob* gene and CT animals heterozygous with respect to the T-allele and the C-allele of the *ob* gene.
52. A method of identifying those animals having increased feed conversion efficiency compared to general population of animals of the same species by determining their *ob* genotype wherein animals that possess the T-containing allele of the *ob* gene have an increased feed conversion efficiency compared to animals that possess only the C-containing allele of the *ob* gene.
53. A method of paragraph 52 wherein TT animals homozygous with respect to the T-allele of the *ob* gene have a greater milk productivity than CT animals heterozygous with respect to the T-allele.
54. A method of breeding livestock animals to increase milk production in the offspring comprising selecting breeding pairs of livestock animals of the same species to increase occurrence of the *ob* T-allele in the offspring.
55. The method of paragraph 54 wherein the milk production is increased in the first one hundred days of lactation in the offspring.
56. A method of increasing milk production in a selected group of livestock animals of the same species comprising:
   (a) determining a genetic predisposition of each animal to produce milk by determining their *ob* genotype;
   (a) selecting animals that possess the T-containing allele of the *ob* gene for inclusion in the group; and
   (b) increasing the amount of feed for in the selected group.
57. The method of paragraph 56 wherein increasing milk production in a selected group of livestock animals of the same species occurs during the first one hundred days of lactation.
58. The method of paragraph 57 wherein determining comprises determining whether the animal is a TT animal homozygous with respect to the T-allele of the *ob* gene, a CC animal homozygous with respect to the C-allele of the *ob* gene, or a CT animal heterozygous with respect to the T-allele and the C-allele of the *ob* gene.
59. A method of paragraph 58 wherein selecting is selecting from the group consisting of TT animals homozygous with respect to the T-allele of the *ob* gene and CT animals heterozygous with respect to the T-allele and the C-allele of the ob gene.
60. The method of any one of the paragraphs 48 to 59 wherein the livestock animal is a bovine, an ovine, an avian or a swine.
61. The method of paragraph 60 wherein the livestock animal is a bovine.
62. The method of paragraph 61 wherein the bovine is a dairy cattle.

Having thus described in detail advantageous embodiments of the present invention, it is to be understood that the invention defined by the above paragraphs is not to be limited to particular details set forth in the above description as many apparent variations thereof are possible without departing from the spirit or scope of the present invention.

## Claims

1. A method of identifying those animals having increased milk productivity compared to general population of animals of the same species by determining their *ob* genotype wherein animals that possess the T-containing allele of the *ob* gene have increased milk productivity compared to animals that possess only the C-containing allele of the *ob* gene.

2. A method of claim 1 wherein TT animals homozygous with respect to the T-allele of the *ob* gene have a greater milk productivity than CT animals heterozygous with respect to the T-allele.

3. The method of claim 1 wherein the identifying comprises determining whether the livestock animal is a TT animals homozygous with respect to the T-allele of the *ob* gene, a CC animal homozygous with respect to the C-allele of the *ob* gene, or a CT animal heterozygous with respect to the T-allele and the C-allele of the *ob* gene.

4. A method of claim 1 wherein the identifying is identifying from the group consisting of TT animals homozygous with respect to the T-allele of the *ob* gene and CT animals heterozygous with respect to the T-allele and the C-allele of the *ob* gene to identify those animals having a greater feed conversion efficiency.

5. The method of claim 1 wherein the livestock animal is a bovine, an ovine, an avian or a swine.

6. The method of claim 1 wherein the livestock animal is a bovine.

7. The method of claim 6 wherein the bovine is a dairy cattle.

8. The method of claim 7 wherein increasing milk production in a selected group of livestock animals of the same species occurs during the first one hundred days of lactation.

9. A method of breeding livestock animals to increase milk production in the offspring comprising selecting breeding pairs of livestock animals of the same species to increase occurrence of the *ob* T-allele in the offspring using the method of identification of claim 1.


SEQUENCE LISTING

<110>  QUANTUM GENETICS IRELAND LTD.

<120>  SYSTEMS AND METHODS FOR IMPROVING PROTEIN AND MILK PRODUCTION OF
       DAIRY HERDS

<130>  P43490.EP.02

<150>  US10/770,307
<151>  2004-02-02

<150>  US60/466,523
<151>  2003-04-29

<150>  US60/509,775
<151>  2003-10-08

<150>  CA2,422,437
<151>  2003-03-18

<150>  US60/456,489
<151>  2003-03-21

<160>  7

<170>  PatentIn version 3.2

<210>  1
<211>  596
<212>  DNA
<213>  Bos sp.

<400>  1
tctgaagacc tggatgcggg tggtaacgga gcacgtgggt gttctcggag atcgacgatg      60

tgccacgtgt ggtttcttct gttttcaggc cccagaagcc catcccggga aggaaaatgc     120

gctgtggacc cctgtatcga ttcctgtggc tttggcccta tctgtcttac gtggaggctg     180

tgcccatctg caaggtccag gatgacacca aaaccctcat caagacaatt gtcaccagga     240

tcaatgacat ctcacacacg gtagggaggg actgggagac gaggtagaac cgtggccatc     300

ccgtggggga ccccagaggc tggcggagga ggctgtgcag ccttgcacag ggccccagtg     360

gcctggacgc ccccctggca taaagacagc tcctctcctc ctccacttcc cttgcctccc     420

gccttctcac tctcctccct cccagaccgg aatcctagtg cccaggccca gaaggagtca     480

cagaggtcct ggggtcccct tggcaggtgg ccagaacccc agcagcagtc cctctgggcc     540

tccatctcat ttctagaatg ttttagtcgt taggcattct tcctgcctgg taactg         596


<210>  2
<211>  596
<212>  DNA
<213>  Bos sp.

<400>  2
tctgaagacc tggatgcggg tggtaacgga gcacgtgggt gttctcggag atcgacgatg      60

tgccacgtgt ggtttcttct gttttcaggc cccagaagcc catcccggga aggaaaatgc     120

gctgtggacc cctgtatcga ttcctgtggc tttggcccta tctgtcttac gtggaggctg     180

tgcccatccg caaggtccag gatgacacca aaaccctcat caagacaatt gtcaccagga     240

tcaatgacat ctcacacacg gtagggaggg actgggagac gaggtagaac cgtggccatc 300

ccgtggggga ccccagaggc tggcggagga ggctgtgcag ccttgcacag ggccccagtg 360

gcctggacgc ccccctggca taaagacagc tcctctcctc ctccacttcc cttgcctccc 420

gccttctcac tctcctccct cccagaccgg aatcctagtg cccaggccca gaaggagtca 480

cagaggtcct ggggtcccct tggcaggtgg ccagaacccc agcagcagtc cctctgggcc 540

tccatctcat ttctagaatg ttttagtcgt taggcattct tcctgcctgg taactg 596

<210> 3
<211> 167
<212> PRT
<213> Bos sp.

<400> 3

Met Arg Cys
1

Ser Tyr Val
20 25 30

Thr Leu Ile Lys Thr Ile Val Thr Arg Ile Asn Asp Ile Ser His Thr
35 40 45

Gln Ser Val Ser Ser Lys Gln Arg Val Thr Gly Leu Asp Phe Ile Pro
50 55 60

Gly Leu His Pro Leu Leu Ser Leu Ser Lys Met Asp Gln Thr Leu Ala
65 70 75 80

Ile Tyr Gln Gln Ile Leu Thr Ser Leu Pro Ser Arg Asn Val Val Gln
85 90 95

Ile Ser Asn Asp Leu Glu Asn Leu Arg Asp Leu Leu His Leu Leu Ala
100 105 110

Ala Ser Lys Ser Cys Pro Leu Pro Gln Val Arg Ala Leu Glu Ser Leu
115 120 125

Glu Ser Leu Gly Val Val Leu Glu Ala Ser Leu Tyr Ser Thr Glu Val
130 135 140

Val Ala Leu Ser Arg Leu Gln Gly Ser Leu Gln Asp Met Leu Arg Gln
145 150 155 160

Leu Asp Leu Ser Pro Gly Cys
165

<210> 4
<211> 20
<212> DNA

<213>  Artificial

<220>
<223>  Synthetic primer

<400>  4
agggatgcct ggacacaaga                                                    20


<210>  5
<211>  22
<212>  DNA
<213>  Artificial

<220>
<223>  Synthetic primer

<400>  5
attgccacca ccagcagcac ca                                                 22


<210>  6
<211>  22
<212>  DNA
<213>  Artificial

<220>
<223>  Synthetic probe

<400>  6
catctgctat gcgaatgctt tg                                                 22


<210>  7
<211>  20
<212>  DNA
<213>  Artificial

<220>
<223>  Synthetic probe

<400>  7
gctaattata ttgtaagaca                                                    20